# EUROPEAN PATENT SPECIFICATION

(11) **EP 3 292 131 B1**
(45) Date of publication and mention of the grant of the patent: **01.05.2019**
(21) Application number: 17716941.4
(22) Date of filing: 18.04.2017
(51) Int. Cl.: C07H 1/00, C07H 3/06

(54) **IMPROVED PREPARATION OF VACCINES AGAINST STREPTOCOCCUS PNEUMONIAE TYPE 3**
VERBESSERTE HERSTELLUNG VON IMPFSTOFFEN GEGEN STREPTOCOCCUS PNEUMONIAE DES TYPS 3
PRÉPARATION AMÉLIORÉE DE VACCINS CONTRE STREPTOCOCCUS PNEUMONIAE TYPE3

(30) Priority: 14.04.2016 EP 16165453; 15.04.2016 EP 16165694
(43) Date of publication of application: 14.03.2018
(73) Proprietor: Max-Planck-Gesellschaft zur Förderung der Wissenschaften e.V., 80539 München (DE); Vaxxilon AG, 4153 Reinach (CH)
(72) Inventor: SEEBERGER, Peter, 14532 Kleinmachnow (DE); PEREIRA, Claney Lebev, 12203 Berlin (DE); PARAMESWARAPPA, Sharavathi Guddehalli, 12203 Berlin (DE); CALOW, Adam Daniel James, Bristol, BS4 2HB (GB); MENOVA, Petra, 16628 Praha 6 (CZ); BAEK, Ju Yuel, Daejon 34049 (KR)
(74) Representative: Arth, Hans-Lothar
(86) International application number: PCT/EP2017/059215
(87) International publication number: WO 2017/178664

(56) References cited:
- EP-A1- 2 851 092
- LEFEBER DIRK J ET AL: "Synthesis of Streptococcus pneumoniae type 3 neoglycoproteins varying in oligosaccharide chain length, loading and carrier protein", CHEMISTRY - A EUROPEAN JOU, , vol. 7, no. 20 1 January 2001 (2001-01-01), pages 4411-4421, XP008173688, ISSN: 0947-6539, DOI: 10.1002/1521-3765(20011015) Retrieved from the Internet: URL:http://onlinelibrary.wiley.com/doi/10. 1002/1521-3765%2820011015%297:20%3C4411::A ID-CHEM4411%3E3.0.CO [retrieved on 2001-10-05] cited in the application
- LEFEBER D J ET AL: "Isolation of oligosaccharides from a partial-acid hydrolysate of pneumococcal type 3 polysaccharide for use in conjugate vaccines", CARBOHYDRATE RESEARCH, PERGAMON, GB, vol. 337, no. 9, 30 April 2002 (2002-04-30), pages 819-825, XP004355355, ISSN: 0008-6215, DOI: 10.1016/S0008-6215(02)00059-9 cited in the application
- B. BENAISSA-TROUW ET AL: "Synthetic Polysaccharide Type 3-Related Di-, Tri-, and Tetrasaccharide-CRM197 Conjugates Induce Protection against Streptococcus pneumoniae Type 3 in Mice", INFECTION AND IMMUNITY, vol. 69, no. 7, 1 July 2001 (2001-07-01), pages 4698-4701, XP055155921, ISSN: 0019-9567, DOI: 10.1128/IAI.69.7.4698-4701.2001 cited in the application
- SZABO Z B ET AL: "Synthesis of fully protected alpha-l-fucopyranosyl-(1->2)-beta-d-galact opyranosides with a single free hydroxy group at position 2', 3' or 4' using O-(2-naphthyl)methyl (NAP) ether as a temporary protecting group", TETRAHEDRON ASYMMETRY, PERGAMON PRESS LTD, OXFORD, GB, vol. 16, no. 1, 10 January 2005 (2005-01-10), pages 83-95, XP027676386, ISSN: 0957-4166 [retrieved on 2005-01-10] cited in the application

## Description

The present invention relates to the preparation of a synthetic tetrasaccharide, hexasaccharide and octasaccharide representing part of the repeating unit of the *Streptococcus pneumoniae* type 3 capsular polysaccharide as well as conjugates thereof. Said conjugates are particularly useful for prevention and/or treatment of diseases associated with *Streptococcus pneumoniae,* and more specifically of diseases associated with *Streptococcus pneumoniae* type 3.

### Background of the invention

The Gram-positive bacterium *Streptococcus pneumoniae* is one of the main pathogens and causes severe invasive diseases like meningitis, pneumonia or bacteremia. Particularly infants, the elderly and immunocompromised patients are at high risk towards pneumococcal infections. About more than 90 serotypes of *Streptococcus pneumoniae* have been identified throughout the world, with a small number of these serotypes accounting for most diseases. The serotypes are identified by their different core capsular polysaccharide (CPS) structure. The CPS consists of polymers of repeating oligosaccharide units, which represent the epitope of the bacteria. These CPS or parts of them are often immunogenic and constitute potential candidates for pneumococcal vaccines.

The resistance of *Streptococcus pneumoniae* to antibiotics is a rapidly increasing problem. Thus, pneumococcal vaccines providing protection against pneumococcal infections are becoming increasingly important. Two classes of pneumococcal vaccines are currently available, one based on polysaccharides and the other based on polysaccharides conjugated to a carrier protein. The polysaccharides are thymus-independent type 2 antigens and thus induce low-affinity antibodies. In addition, they evoke no B-cell memory. Pneumococcal conjugate vaccines can circumvent these disadvantages with an increased serotype-specific antibody response to capsular polysaccharides. The conjugate vaccine PCV-13 contains immunogenic conjugates comprising the purified polysaccharides of 13 different *S*. *pneumoniae* serotypes covalently linked to a protein, such as CRM₁₉₇.

*Streptococcus pneumoniae* type 3 (ST3) is part of the current pneumococcal vaccines. The capsular polysaccharide of ST3 consists of [→3)-β-D-Glc*pA-*(1→4)-β-D-Glc*p*-(1→] repeating units. Immunization experiments with ST3 oligosaccharides conjugated to CRM₁₉₇ showed that increasing IgG antibody titers were found with increasing chain length (from monosaccharide to tetrasaccharide) and no influence on the immunogenicity by the oligosaccharide/CRM₁₉₇ ratio (see Benaissa-Trouw et al., Infection and Immunity, 2001, 69, 4698 - 4701).

Oligosaccharides derived from the repeating unit of *S*. *pneumoniae* type 3 can be obtained either by hydrolysis of the capsular polysaccharide of *S*. *pneumoniae* type 3 or synthetically. Hydrolysis of the capsular polysaccharide of *S*. *pneumoniae* type 3 results in a mixture of fragments of increasing degree of oligomerisation. Separation of the different fragments is difficult and due to practical considerations the conjugation chemistry available for polysaccharide-protein conjugation is basically restricted to reductive amination reactions (see for example: Snippe et al., Infection and Immunity, 1983, 42, 842 - 844). On the other hand, synthetic methods provide oligosaccharides with defined structures and the ability to use a much broader range of conjugation methods.

Lefeber et al. (Carbohydrate Research, 2002, 337, 819 - 825) teach the isolation of ST3 oligosaccharides with the repeating unit [→3)-β-D-Glc*p*A-(1→4)-β-D-Glc*p-*(1→]ₙ, wherein n = 1 - 7. The ST3 oligosaccharides are isolated from a partial-acid hydrolysate of the capsular polysaccharide of *S*. *pneumoniae* type 3 by Sepharose Q ion-exchange chromatography. The isolated fragments were contaminated by side-products originating from cleavage of β-D-Glc*p*A-(1→4)-β-D-Glcp linkage.

Lefeber et al. (Chemistry - Eur. J. 2001, 7, 4411 - 4421) also disclose the synthesis of a tetrasaccharide derived from the repeating unit of *S*. *pneumoniae* type 3 linked to 3-aminopropanol and the synthesis of a hexasaccharide derived from the repeating unit of *S*. *pneumoniae* type 3 (Canadian Journal of Chemistry 2002, 80, 76 - 81). The approach consists of sequentially coupling 4,6-benzylidene protected glucose molecules with 6-benzoyl protected glucose to di-, tri- or tetrasaccharide. Subsequent selective removal of benzylidene groups and selective oxidation of primary 6-OH group introduces the carboxylic groups. Glycosylation is realized by trichloroacetimidate activated glucosyl donors and O-allyl protected glucosyl acceptors. The 3-aminopropanol spacer is introduced as 3-azido-1-propanol and the azide group reduced to amino group during the deprotection of the oligosaccharide. The 3-aminopropyl glycoside is conjugated to a carrier protein (i.e. CRM₁₉₇, KLH or TT) using the squarate coupling method. The approach makes this synthesis elaborate for longer oligosaccharides, because only one glucose unit can be introduced in each step.

Rathwell et al. (EP 2 851 092) disclose protein- and peptide-free synthetic vaccines against *S*. *pneumoniae* type 3. The oligosaccharides derived from the repeating unit of *S*. *pneumoniae* type 3 are synthesized in a sequential manner, i.e. repeatedly coupling of suitably protected glucose disaccharide fragments to spacer-containing fragment and subsequent introduction of carboxylic acid groups by selective oxidation of the 4,6-deprotected glucose parts. The amine functionalized oligosaccharide is conjugated to a glycosphingolipid (GSL) derived from galactosyl ceramide. The Synthesis of the oligosaccharide proceeded only in moderate yields due to the TBS group (tert-butyldimethylsilyl) at position 3 of the glucuronic acid moiety. Partial cleavage of the TBS group was observed under these reaction conditions. While elongation of the carbohydrate is achieved with a disaccharide fragment, the synthesis of longer oligosaccharides is still elaborate.

Szabó et al. (Tetrahedron: Asymmetry 2005, 16, 83) report the synthesis of perbenzylated α-L-Fuc*p*-(1→2)-β-D-Gal*p*-(1→OMe) having a single free OH group either at position C-2', C-3' or C-4' utilizing the O-(2-naphthyl)methyl group (Nap) as temporary protecting group. The Nap group was introduced as acetal and acted as a non-participating group. The yields of the glycosylation reaction were above 80%. The Nap group was removed by oxidative cleavage with DDQ.

Thus, the state of the art discloses the synthesis of oligosaccharides derived from the repeating unit of *S*. *pneumoniae* type 3 as well as their conjugation to proteins and glycosphingolipids
However these syntheses are elaborate, contain many tedious reaction steps with low or moderate yields and are therefore not practical for the synthesis of oligosaccharides derived from the repeating unit of *S*. *pneumoniae* type 3 with more than 4 repeating units. Thus a convenient synthesis is so far not known in the state of the art.

It is the objective of the present invention to provide a method for the synthesis of a tetrasaccharide, a hexasaccharide and an octasaccharide derived from the repeating unit of *S*. *pneumoniae* type 3 suitable for covalently binding to carrier proteins like CRM₁₉₇ or TT or to a glycosphingolipid. Pharmaceutical compositions of said conjugates are useful for prevention and/or treatment of diseases associated with *Streptococcus pneumoniae,* and more specifically of diseases associated with *Streptococcus pneumoniae* type 3. Immunization with said conjugates results in the production of high titers of antibodies against pneumococcal capsular polysaccharide of ST3. The antibodies present opsonophagocytosis activity and bactericidal activity.

The objective of the present invention is solved by the teaching of the independent claims. Further advantageous features, aspects and details of the invention are evident from the dependent claims, the description, the figures, and the examples of the present application.

### Brief description of the invention

The present invention relates to a synthetic method for preparation of tetrasaccharide, hexasaccharide and octasaccharide derived from the repeating unit of S. *pneumoniae* type 3 of formula **1**, 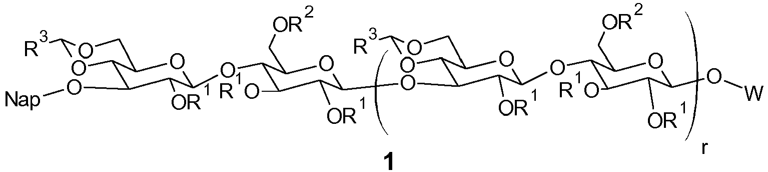 wherein R¹ and R² represent orthogonal protecting groups, R³ is an aryl or alkyl group, r is either 1 (**1-A**) or 2 (**1-B**) or 3 (**1-C**) and W is either a benzyl group or protected linker -L-P for conjugation to a carrier protein or glycosphingolipid.

Formation of the saccharide of formula **1** is achieved by coupling the two saccharides of formula **2** and **3** in the presence of a catalyst, 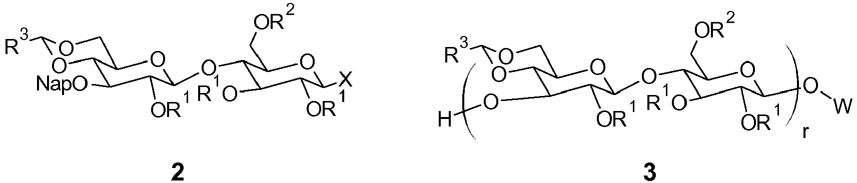 wherein X is a leaving group, preferably a acyl or thioether group like a thioalkyl group, r is 1, 2 or 3 and W is either a benzyl group or protected linker -L-P for conjugation to a carrier protein or glycosphingolipid. The coupling of the saccharides of formula **2** and **3** is a key step of the synthesis of longer saccharides such as tetrasaccharide **1-A** or hexasaccharide **1-B** or octasaccharide **1-C**.

One aspect of the innovation of this improved synthesis relies on the use of the 2-naphthylmethyl protecting group (Nap). The Nap group can be easily installed and selectively removed under mild conditions, i.e. in the presence of DDQ (2,3-dichloro-5,6-dicyano-1,4-benzoquinone) or ceric ammonium nitrate (CAN) or by hydrogenation. Despite the facile removal under mild conditions, Nap-containing intermediates are stable under coupling reaction conditions. No side reactions occurred during synthesis due to undesired removal of Nap group. Other protecting groups like TBS-ether showed partial cleavage under coupling reaction conditions. Therefore, increased yields are achieved by using the Nap group, especially in the formation of the saccharides of formula **1**.

Surprisingly, it was found that all Nap-protected products and intermediates were crystalline. This facilitated the purification procedures during synthesis. All Nap-protected products and intermediates could be purified by recrystallization or by trituration. The use of time- and material-consuming column chromatography for purification was avoided in many steps. Thus, the use of the Nap group facilitates performing the synthesis and further allows scaling up the synthesis without significant increase of required time and material.

Another aspect of the present invention is that the tetrasaccharide, hexasaccharide and octasaccharide of formula **1** containing a protected linker enable conjugation to a protein or glycosphingolipid, at the reducing end following deprotection.

Another aspect of the present invention is that the tetrasaccharide, hexasaccharide or octasaccharide of formula **1** having a benzyl group at the reducing end can be converted to a tetrasaccharide, hexasaccharide or octasaccharide of *S*. *pneumoniae* serotype 3 of formula **8'**, wherein r is 1 or 2 or 3.

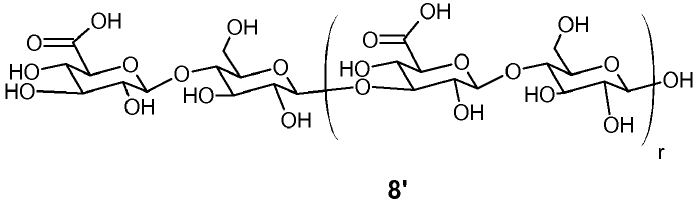

A further aspect of the disclosed invention relates to the deprotection of the saccharide of formula **1** bearing a protected linker. Removal of the acetal groups under addition of an acid is followed by selective oxidation of the primary 6-OH-group. Subsequent removal of the remaining protective groups and simultaneous deprotection of the linker lead to the deprotected saccharide of formula **8**, 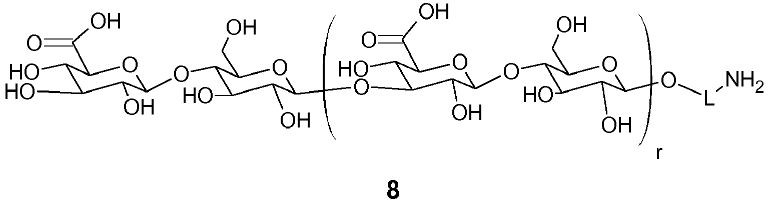 wherein L may be an aliphatic or aromatic residue, e.g. an alkyl(en) group or phenyl(en) group and r is 1 or 2 or 3.

In a further aspect, the unprotected saccharide of formula **8** bearing a reactive linker is conjugated to a carrier protein or a glycosphingolipid. The carrier protein can be any carrier protein known in the art, in particular in the field of vaccine development. More specifically, the carrier protein is selected from the group comprising diphtheria toxoid CRM₁₉₇, tetanus toxoid (TT), outer membrane protein (OMP), bovine serum albumin, (BSA), keyhole limpet hemocyanine (KLH), diphtheria toxoid (DT), cholera toxoid (CT), recombinant *Pseudomonas aeruginosa* exotoxin A (rEPA), Clostridium difficile toxin A (TcdA), Clostridium difficile toxin B (TcdB).

Thus the synthetic method disclosed herein has huge unexpected advantages over the state of the art synthetic methods that intermediate products are crystalline, coupling reaction yields are higher, less reaction steps are required and purification of intermediate products is easier. Therefore, the improved method is less time and material consuming, less costly and easier to scale up because column chromatography is in many reaction steps no longer necessary.

### Detailed Description of the invention

### Definitions

The linker or spacer group herein disclosed may be any moiety that enables to couple the oligosaccharide to a carrier molecule, i.e. a carrier protein or a glycosphingolipid. A large variety of such linker groups are known in the art and a suitable linker group can be selected in dependence from the respective carrier molecule. For example, the linker may be an aliphatic or aromatic residue, e.g. an alkyl(en) group or phenyl(en) group, comprising a reactive functional group, such as an amino group, preferably a primary amino group, (activated) carboxy group, aldehyde, azide, alkenyl or alkynyl group. In specific embodiments the linker may comprise a polyether or polyester chain. In particular, the linker is selected from the group comprising primary alkylamines, alkyl or aralkyl residues with a terminal aldehyde, azide, alkyne or alkene group or (activated) carboxy group, and alkylaryl and aryl residues, e.g. phenyl residues, comprising a reactive amine, an aldehyde or an azide group, or (activated) carboxy group.

In a specific embodiment of the invention, the linker is -(CH₂)ₙ-NH₂, with n being an integer from 2 to 10, preferably 2 to 8, more preferably 2 to 5. In another embodiment of the invention, the linker is -C₂H₄-O-C₂H₄.

The invention concerns a synthetic method for preparation of a ST3 tetrasaccharide as well as a ST3 tetrasaccharide bearing a linker.

The invention further concerns a synthetic method for preparation of a ST3 hexasaccharide as well as a ST3 hexasaccharide bearing a linker.

The invention also concerns a synthetic method for preparation of a ST3 octasaccharide as well as a ST3 octasaccharide bearing a linker.

The present invention provides very favorable and efficient methods for synthesizing the ST3 tetrasaccharide, ST3 hexasaccharide, ST3 octasaccharide as well as ST3 tetrasaccharide conjugates, ST3 hexasaccharide conjugates and ST3 octasaccharide conjugates selectively and in high yields.

### Retrosynthetic analysis

The inventive method for preparation of the tetrasaccharide of formula **8-A** is outlined in Scheme 1 below. The synthetic method comprises the following steps:
- construction of monosaccharide building block of formula **14** from thioglucoside of formula **18**
- coupling monosaccharide building blocks of formula **14** and **15** or **15** and **18** to disaccharide of formula **2** with special consideration being given to assure that only the β-anomer is formed,
- converting disaccharide of formula **2** to disaccharide of formula **3-A**, which comprises coupling disaccharide of formula **2** with a benzyl alcohol or protected linker and subsequent selective removal of the Nap-group,
- coupling disaccharides of formula **2** and **3-A** to fully protected tetrasaccharide of formula **1** bearing a benzyl group or a protected linker at the reducing end with special consideration being given to assure that only the β-anomer is formed,
- selective removal of the acetal groups of fully protected tetrasaccharide of formula **1-A**,
- introduction of carboxylic acid groups by selective oxidation of the primary 6-OH-groups of protected tetrasaccharide of formula **4-A,**
- removal of the remaining protecting groups, i.e. Nap, R¹, R² and protecting groups of the linker to obtain deprotected tetrasaccharide bearing a linker of formula **8-A** or removal of the remaining protecting groups and the benzyl group at the reducing end to obtain deprotected tetrasaccharide of formula **8'-A**.

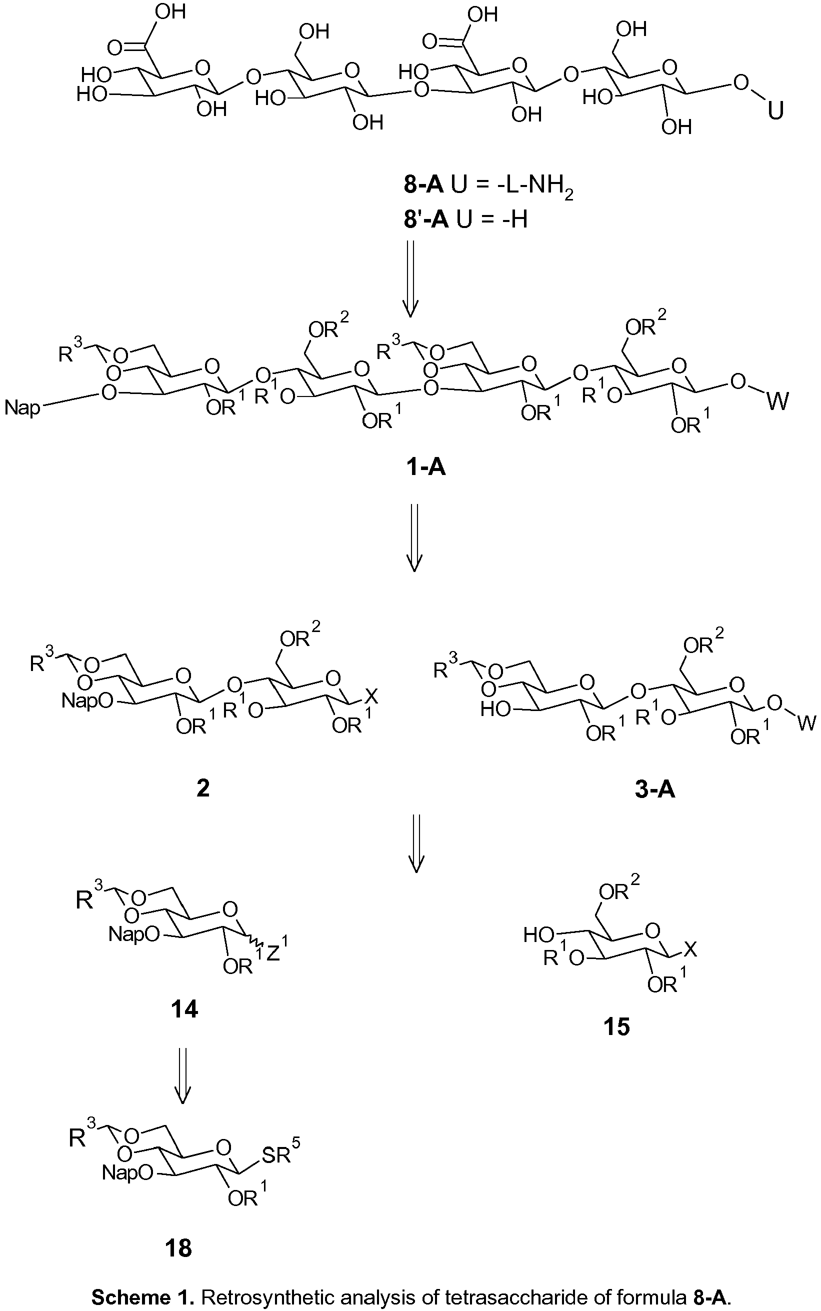

The inventive method for preparation of the hexasaccharide of formula **8-B** and octasaccharide of formula **8-C** is outlined in Scheme 2 below. The synthetic method comprises the following steps:
- converting the saccharide of formula **1-B** / **1-C** to the saccharide of formula **3-B** / **3-C** by selective removal of the Nap-group
- coupling saccharides of formula **2** and **3-B** / **3-C** to fully protected saccharide of formula **1-B** / **1-C** bearing a benzyl group or a protected linker at the reducing end with special consideration being given to assure that only the β-anomer is formed,
- selective removal of the acetal groups of fully protected saccharide of formula **1-B/ 1-C**,
- introduction of carboxylic acid groups by selective oxidation of the primary 6-OH-groups of protected saccharide of formula **4-B** / **4-C**,
- removal of the remaining protecting groups, i.e. Nap, R¹, R² and protecting groups of the linker to obtain deprotected saccharide bearing a linker of formula **8-B** / **8-C** or removal of the remaining protecting groups and the benzyl group at the reducing end to obtain deprotected hexasaccharide of formula **8'-B** or deprotected octasaccharide of formula **8'-C**.

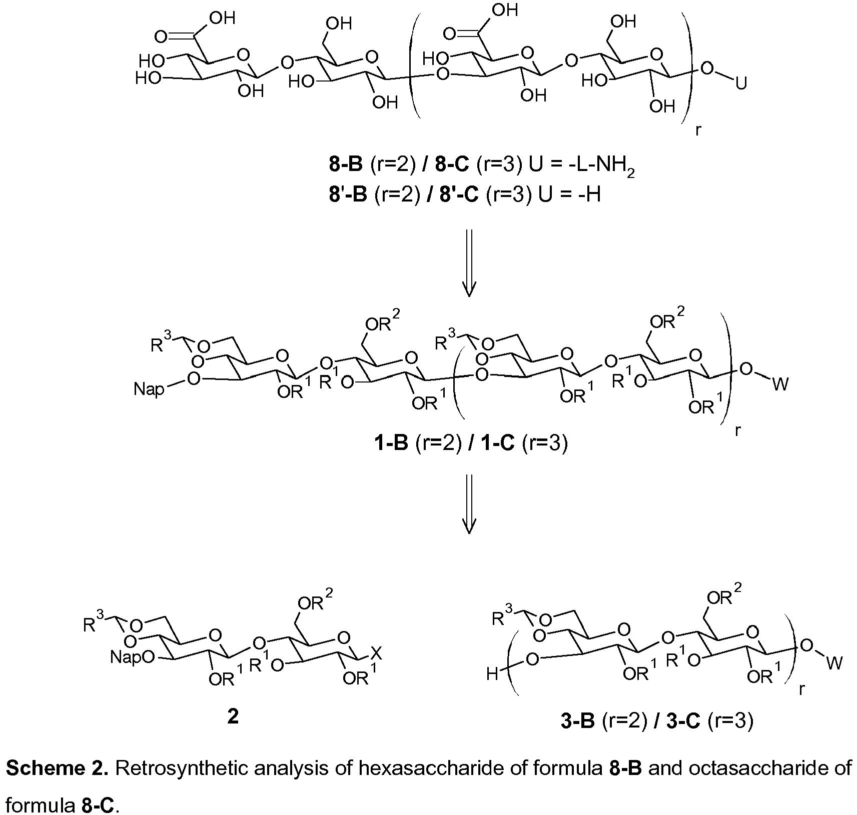

The method follows the approach of first constructing the oligosaccharide containing only glucose fragments and subsequent oxidizing every second glucose fragment to glucuronic acid fragment.

### Protecting group strategy

It is necessary for the tetrasaccharide synthesis to provide a set of orthogonal protecting groups. In order to construct the saccharide of formula **8** from disaccharides of formula **2**, the ability to selectively and independently remove the Nap-group and the protecting group R³ in the presence of all other protecting groups is essential. The term "protecting group" or "protective group" as used herein refers to commonly used groups in organic synthesis, preferably used for protection of amines, hydroxyl groups, thiols, imines, carbonyls, carboxyls or other common functional groups, and particularly preferred for amines and hydroxyl groups. The protecting groups are characterized in that they are stable under reaction conditions applied during the synthesis, i.e. they are not cleaved off or undergo undesired side reactions and prevent any reaction of the protected functional group they are bonded to. Additionally, the protecting groups are selected to not hinder or to not affect the performed reaction steps in terms of yield or stereoselectivity.

The orthogonal protecting groups R¹ and R² are present during the synthesis and removed in the last deprotection step(s). R¹ and R² are therefore regarded as permanent orthogonal protecting groups. On the other hand, the Nap-group and R³ are removed during the synthesis and are therefore temporary protecting groups.

In order to selectively and independently remove the Nap-group in the presence of the permanent orthogonal protecting groups R¹ and R², the permanent protecting groups have to be stable towards an oxidizing agent, i.e. DDQ and CAN. As used herein the term "oxidation-stable protecting group" refers to a protecting group that is stable during oxidation reaction performed with DDQ or CAN as reagents. Thus, the permanent orthogonal protecting groups R¹ and R² have to be oxidation-stable. In addition the permanent orthogonal protecting groups R¹ and R² have to be stable under acidic conditions, which are applied to remove the acetal protecting group installed at the 4^{th} and 6^{th} position of the glucose moiety of the saccharide **1** and which are also applied in the glycosylation reactions. The acetal protecting group installed at the 4^{th} and 6^{th} position of the glucose moiety of the saccharide **1** can be removed by treatment with a nucleophile in presence of a catalytic amount of an acid. Thus, orthogonal protecting groups R¹ and R² have to be stable upon treatment of the saccharide with a nucleophile and a catalytic amount of acid. Examples of nucleophiles that can be used in the removal of the acetal protecting group installed at the 4^{th} and 6^{th} position of the glucose moiety of the saccharide **1** include, but are not restricted to: water, alcohol and thiol. Preferably, the nucleophile is a thiol, such as ethanethiol (EtSH), tolylthiol, phenylthiol, ethylenethiol and more preferably the nucleophile is ethanethiol (EtSH). Examples of acids that can be used in catalytic amount for the removal of the acetal protecting group installed at the 4^{th} and 6^{th} position of the glucose moiety of the saccharide **1**, include but are not restricted to the following Brønstedt acids: methanesulfonic acid, p-toluenesulfonic acid, camphorsulfonic acid (CSA) and trifluoromethanesulfonic acid and the Lewis acid Er(OTf)₃.

Glycosylation reactions take place upon treatment of a donor and an acceptor with an activator or an activating agent: Activators or activating agents known to the skilled person include, but are not restricted to: AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST). Preferably the activator or activating agent is NIS/TfOH.
Hence, permanent orthogonal protecting groups R¹ and R² have to be stable under treatment of the saccharides with any of the above-mentioned activating agents.

As used herein the term "acid-stable protecting group" refers to a protecting group which is stable in the presence of a nucleophile and catalytic amounts of an acid as well as in the presence of an activating agent.

Preferably, acid-stable orthogonal protecting groups R¹ and R² are protecting groups which are stable in the presence of ethanethiol and p-toluenesulfonic acid as well as in the presence of NIS/TfOH.

Consequently, the permanent orthogonal protecting groups R¹ and R² have to be oxidation-stable i.e. have to be stable upon treatment with the oxidizing agent required for removal of the Nap protecting group (e.g. DDQ or CAN) and acid-stabile.

The orthogonal protecting group R¹, which also blocks the 2-OH-group, is chosen to facilitate the formation of β-anomers in glycosylation reactions, because the ST3 repeating unit consists only of β-anomers of glucose and glucuronic acid. The formation of β-anomers can be significantly promoted when the protecting group employed at the 2-OH-group of the glucosyl donor participates in the glycosylation reaction so that it blocks the lower side of the anomeric center. Therefore the β-anomer is predominantly formed. Such participating groups are known from the state of the art. Thus, the orthogonal protecting group R¹ is preferably an ester group, more preferably a benzoyl, chloroacetyl (ClAc), acetyl or pivaloyl (Piv) group and even more preferably a benzoyl group.

For the orthogonal protecting group R² the same limitations hold true, i.e. oxidation-stable and acid-stabile. However, R² does not need to participate in the coupling reaction. R² may be an ester group, a benzyl group or a TBS-group. More preferably, R² is a benzyl group.

The acetal protecting group R³ may be any alkyl or aryl group. Preferred are phenyl and substituted phenyl groups like 4-methoxyphenyl, 4-nitrophenyl and 2-nitrophenyl. Even more preferred is when R³ represents a phenyl group.

Accordingly, the functional group of the linker has to be protected with oxidation-stable and acid-stable protecting group(s) during the ST3 tetrasaccharide, ST3 hexasaccharide or ST3 octasaccharide synthesis. Particularly, when the functional group is a primary amine, it can be protected preferably but not exclusively as: -N(Bn)Cbz, -NHCbz, -N₃, -NBn₂, -NAllyl₂ or -NPhth. When the protected functional group can be written as -NR'R", -NPhth can be understood as R' and R" form together with the nitrogen atom they are attached to a phthalimide or R' and R" form together phthaloyl. Methods for deprotection or conversion of such groups into primary amines are known to the skilled person.

The term "coupling reaction" herein used refers to reactions between two saccharides wherein one reducing end reacts or the term refers to reactions between one saccharide and an alcohol. Consequently, O-glycosylation methods are employed in the coupling reaction steps of the synthetic method according to the invention. These O-glycosylation methods are known from the state of the art. Generally, they require a leaving group at the reducing end of the donor, which is activated in the presence of a catalyst. Leaving groups can be selected in dependence on the used catalyst and may be for example: a halogen, -O-C(=NH)-CCl₃, -O-C(=NPh)-CF₃, -OAc, -SR⁵, -SO-Ph, -O-(CH₂)₃-CH=CH₂, -O-P(OR⁵)₂, -O-PO(OR⁵)₂, -O-CO-OR⁵, -O-CO-SR⁵, -O-CS-SR⁵, -O-CS-OR⁵, 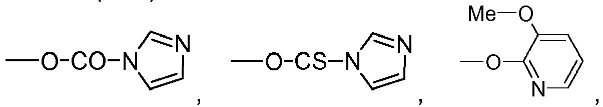 wherein R⁵ may be any alkyl or aryl group.

In a coupling or glycosylation reaction any suitable catalyst or promoter or activator or combinations thereof for O-glycosylation reactions are known in the art, i.e. Brønstedt acids or Lewis acids, may be employed. Specifically, these acids can be selected from the non-exhaustive list comprising AgOTf, BF₃•OEt₂, trimethylsilyl trifluoromethanesulfonate (TMSOTf), trifluoromethanesulfonic acid (TfOH), trifluoromethanesulfonic anhydride (Tf₂O, triflic anhydride), lanthanoid(III) triflates, NIS/AgOTf, NIS/TfOH or dimethyl(methylthio)sulfonium trifluoromethanesulfonate (DMTST).

### Construction of monosaccharide building blocks 14 and 18

The monosaccharide building block **14** is prepared from thioglucoside **18**. The preparation of thioglucoside **18** is known to a skilled person, especially when R¹ = -Bz, R³ = -Ph and R⁵ = -Et. Thioglucoside **18** can be formed starting from glucose diacetonide **21** (see Scheme 3) or is also commercially available, when R¹ = -Bz, R³ = -Ph and R⁵ = -Et. The residue R⁵ of the thioglucosides **18**, **25**, **26** and **27** may contain any alkyl or aryl group. Preferably, R⁵ is either an ethyl group or a tolyl group.

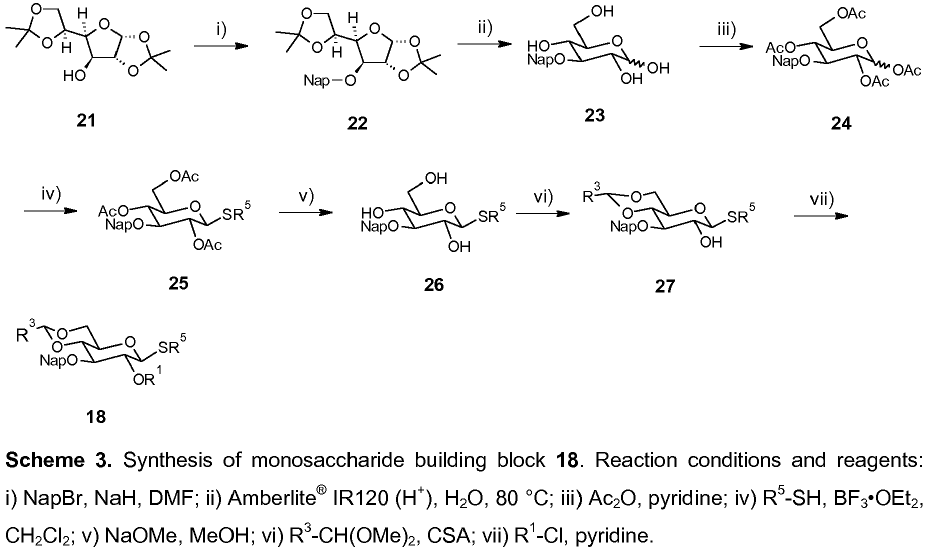

Starting from glucose diacetonide **21** the 3-OH group is blocked by 2-naphthylmethyl bromide in the presence of sodium hydride. Subsequent removal of acetonide groups of glucose derivative **22** leads to 3-Nap-protected glucose **23** which is then completely acetylated. Peracetylated glucose **24** is then converted to thioglucoside **25**. Deacetylation is achieved in the presence of a base and by acetalization with an aldehyde or preferably the corresponding dialkyl acetal and a strong acid the 4-OH and 6-OH groups are selectively blocked. Finally, the remaining 2-OH-group of **26** is protected as an ester.

### Preparation of monosaccharide of formula 14

The synthesis of the monosaccharide of formula **14** from monosaccharide building block of formula **18** according to the present invention is outlined in Scheme 4 below.

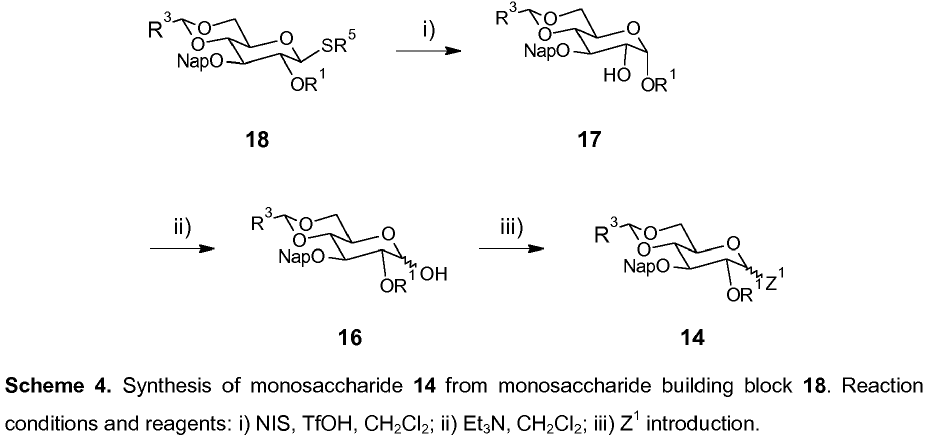

The inventive method comprises the following steps:
a3) Providing a monosaccharide of formula **18** 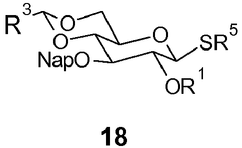 wherein
   R¹ is an orthogonal oxidation-stable and acid-stable protecting group,
   R³ is an aryl or alkyl group,
   R⁵ is selected from -Me, -Et, -Pr, -Bu, -Ph, and -Tol;
b3) Converting the monosaccharide of formula **18** to a monosaccharide of formula **17** 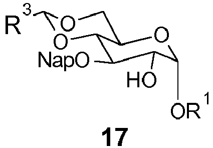 wherein
   R¹ is an orthogonal oxidation-stable and acid-stable protecting group,
   R³ is an aryl or alkyl group; and
c3) Treatment of the monosaccharide of formula **17** with a base to obtain a monosaccharide of formula **16** 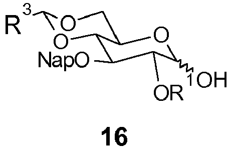 wherein
   R¹ is an orthogonal oxidation-stable and acid-stable protecting group,
   R³ is an aryl or alkyl group;
   and
d3) converting the monosaccharide of formula **16** to the monosaccharide of formula **14** 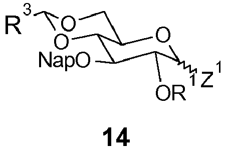 wherein
   R¹ represents an orthogonal oxidation-stable and acid-stable protecting group,
   R³ is an aryl or alkyl group
   and Z¹ is selected from -O-C(=NH)-CCl₃, -O-C(=NPh)-CF₃, -OAc and -SR⁵, with R⁵ being selected from -Me, -Et, -Pr, -Bu, -Ph and -Tol.

It is preferred that the method comprises the following steps:
a3) Providing a monosaccharide of formula **18** wherein
   R¹ is selected from -Bz, -ClAc, -Ac and -Piv, R³ is selected from -Me, -Et, -Ph, -tBu and -PMP, and R⁵ is selected from -Me, -Et, -Pr, -Bu, -Ph and -Tol;
b3) Converting the monosaccharide of formula **18** to a monosaccharide of formula **17**; and c3) Treatment of the monosaccharide of formula **17** with a base to obtain a monosaccharide of formula **16**; and d3) converting the monosaccharide of formula **16** to the monosaccharide of formula **14**, wherein Z¹ is selected from -O-C(=NH)-CCl₃, -O-C(=NPh)-CF₃, -OAc and -SR⁵.

More preferably, the method according to the invention comprises the following steps: a3) Providing a monosaccharide of formula **18** wherein R¹ represents -Bz, R³ is selected from -Me, -Et, -Ph, -tBu and -PMP, and R⁵ is selected from -Me, -Et, -Pr, -Bu, -Ph, and -Tol;
b3) Converting the monosaccharide of formula **18** to a monosaccharide of formula **17**; and c3) treatment of the monosaccharide of formula **17** with a base to obtain a monosaccharide of formula **16**; and; d3) converting the monosaccharide of formula **14** to the monosaccharide of formula **14**, wherein Z¹ is selected from -O-C(=NH)-CCl₃, -O-C(=NPh)-CF₃, -OAc and -SR⁵.

More preferably, the method according to the invention comprises the following steps: a3) Providing a monosaccharide of formula **18** wherein R¹ is selected from -Bz, -ClAc, -Ac and -Piv, R³ represents, -Ph, R⁵ is selected from -Me, -Et, -Pr, -Bu, -Ph,and -Tol;
b3) Converting the monosaccharide of formula **18** to a monosaccharide of formula **17**; and c3) treatment of the monosaccharide of formula **17** with a base to obtain a monosaccharide of formula **16**; and d3) converting the monosaccharide of formula **16** to the monosaccharide of formula **14**, wherein Z¹ is selected from -O-C(=NH)-CCl₃, -O-C(=NPh)-CF₃, -OAc or -SR⁵.

Even more preferably, the method according to the invention comprises the following steps: a3) Providing a monosaccharide of formula **18** wherein R¹ is selected from -Bz, -ClAc, -Ac and -Piv, R³ is selected from -Me, -Et, -Ph, -tBu and -PMP, R⁵ represents -Et;
b3) Converting the monosaccharide of formula **18** to monosaccharide of formula **17**; and c3) treatment of the monosaccharide of formula **17** with a base to obtain a monosaccharide of formula **16**; and d3) converting the monosaccharide of formula **16** to the monosaccharide of formula **14**, wherein Z¹ is selected from -O-C(=NH)-CCl₃, -O-C(=NPh)-CF₃, -OAc and -SR⁵, wherein R⁵ is selected from -Me, -Et, -Pr, -Bu, -Ph, and -Tol.

Even more preferably, the method according to the invention comprises the following steps: a3) Providing a monosaccharide of formula **18** wherein R¹ is selected from -Bz, -ClAc, -Ac and -Piv, R³ is selected from -Me, -Et, -Ph, -tBu and -PMP, R⁵ is selected from -Me, -Et, -Pr, -Bu, -Ph, and -Tol;
b3) Converting the monosaccharide of formula **18** to a monosaccharide of formula **17**; and c3) treatment of the monosaccharide of formula **17** with a base to obtain a monosaccharide of formula **16**; and d3) converting the monosaccharide of formula **16** to the monosaccharide of formula **14**, wherein Z¹ represents -O-C(=NPh)-CF₃.

Even more preferably, the method according to the invention comprises the following steps: a3) Providing a monosaccharide of formula **18** wherein R¹ is selected from -Bz, and -Ac, R³ represents -Ph, R⁵ represents -Et;
b3) Converting the monosaccharide of formula **18** to a monosaccharide of formula **17**; and c3) treatment of the monosaccharide of formula **17** with a base to obtain a monosaccharide of formula **16**; and d3) converting the monosaccharide of formula **16** to the monosaccharide of formula **14**, wherein Z¹ represents -O-C(=NPh)-CF₃.

It is preferred that the conversion of monosaccharide of formula **18** to monosaccharide of formula **17** is performed in the presence of NIS and catalytic amounts of a strong acid in a mixture of water and an aprotic apolar solvent at low temperatures between -20°C and 10°C. Even more preferred is that the conversion of monosaccharide of formula **18** to monosaccharide of formula **17** is performed in the presence of NIS and catalytic amounts of trifluoromethanesulfonic acid in a mixture of water and CH₂Cl₂ at 0°C.

It is also preferred that the reaction of monosaccharide of formula **17** to monosaccharide of formula **16** is performed in the presence of a base in an aprotic apolar solvent. Even more preferred is that the reaction of monosaccharide of formula **17** to monosaccharide of formula **16** is performed in the presence of triethylamine in CH₂Cl₂.

It is preferred that the conversion of monosaccharide of formula **16** to monosaccharide of formula **14** is achieved by an imidoyl chloride, or trihalogenated acetonitrile in the presence of a base in an aprotic apolar solvent. More preferred is that the conversion of monosaccharide of formula **16** to monosaccharide of formula **14** is achieved by 2,2,2-trifluoro-N-phenylacetimidoyl chloride in the presence of Cs₂CO₃ and/or K₂CO₃ in methylene chloride. According to the present invention the monosaccharide of formula **14** is preferably obtained in form of crystals by recrystallization or trituration.

Thus, it is especially preferred that the method for the preparation of monosaccharide of formula **14** comprises the following steps: a3) Providing a monosaccharide of formula **18** with R¹ representing benzoyl, R³ is a phenyl group and R⁵ representing ethyl; b3) Converting the monosaccharide of formula **18** to a monosaccharide of formula **17**; and c3) treatment of the monosaccharide of formula **17** with triethylamine to obtain a monosaccharide of formula **16**; and d3) converting the monosaccharide of formula **16** to the monosaccharide of formula **14** with Z¹ representing -O-C(=NPh)-CF₃.

### Preparation of disaccharide of formula 2

The preparation of disaccharide of formula **2** according to the present invention comprises the following steps:
a2) Providing a monosaccharide building block of formula **14** 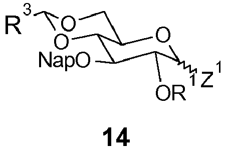 wherein
   R¹ represents an orthogonal oxidation-stable and acid-stable protecting group,
   R³ is an aryl or alkyl group
   and Z¹ is selected from -O-C(=NH)-CCl₃, -O-C(=NPh)-CF₃, -OAc and -SR⁵, with R⁵ being selected from -Me, -Et, -Pr, -Bu, -Ph and -Tol,
   and a monosaccharide building block **15** 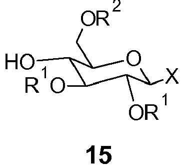 wherein
   R¹ represents an orthogonal oxidation-stable and acid-stable protecting group,
   R² represents an orthogonal oxidation-stable and acid-stable protecting group, and
   X represents -OAc or -SR⁵, with R⁵ being selected from -Me, -Et, -Pr, -Bu, -Ph and -Tol;
   and
b2) Coupling of the monosaccharide building block of formula **14** with the monosaccharide building block of formula **15** in presence of a catalyst to yield disaccharide of formula **2** 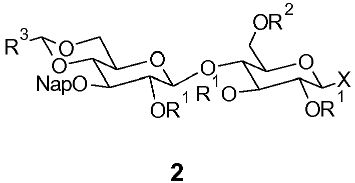 wherein
   R¹ represents an orthogonal oxidation-stable and acid-stable protecting group,
   R² represents an orthogonal oxidation-stable and acid-stable protecting group,
   R³ is an aryl or alkyl group,
   X represents -OAc or -SR⁵, with R⁵ being selected from -Me, -Et, -Pr, -Bu, -Ph and -Tol.

It is preferred that the method for the preparation of disaccharide of formula **2** comprises the following steps:
a2) Providing a monosaccharide building block of formula **14** wherein R¹ is selected from -Bz, -ClAc, -Ac and -Piv, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block **15** wherein R² is selected from -Bz, -Ac, -Piv, -Bn and -TBS, X represents -OAc or -SR⁵ and R⁵ is selected from -Me, -Et, -Pr, -Bu, -Ph, and -Tol;
b2) Coupling of the monosaccharide building block of formula **14** with the monosaccharide building block of formula **15** in presence of a catalyst to yield disaccharide of formula **2**.

It is also preferred that the method for the preparation of disaccharide of formula **2** comprises the following steps:
a2) Providing a monosaccharide building block of formula **14** wherein R¹ represents -Bz, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block **15** wherein R² is selected from -Bz, -Ac, -Piv, -Bn and -TBS, X represents -OAc or -SR⁵ and R⁵ is selected from -Me, -Et, -Pr, -Bu, -Ph, and -Tol;
b2) Coupling of the monosaccharide building block of formula **14** with the monosaccharide building block of formula **15** in presence of a catalyst to yield disaccharide of formula **2**.

It is even more preferred that the method for the preparation of disaccharide of formula **2** comprises the following steps:
a2) Providing a monosaccharide building block of formula **14** wherein R¹ is selected from -Bz, -ClAc, -Ac and -Piv, R³ represents -Ph and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block **15** wherein R² is selected from -Bz, -Ac, -Piv, -Bn and -TBS, X represents -OAc or -SR⁵ and R⁵ is selected from -Me, -Et, -Pr, -Bu, -Ph, and -Tol;
b2) Coupling of the monosaccharide building block of formula **14** with the monosaccharide building block of formula **15** in presence of a catalyst to yield disaccharide of formula **2**.

It is even more preferred that the method for the preparation of disaccharide of formula **2** comprises the following steps:
a2) Providing a monosaccharide building block of formula **14** wherein R¹ is selected from -Bz, -ClAc, -Ac and -Piv, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block **15** wherein R² represents -Bn, X represents -OAc or -SR⁵ and R⁵ is selected from -Me, -Et, -Pr, -Bu, -Ph, and -Tol;
b2) Coupling of the monosaccharide building block of formula **14** with the monosaccharide building block of formula **15** in presence of a catalyst to yield disaccharide of formula **2**.

It is even more preferred that the method for the preparation of disaccharide of formula **2** comprises the following steps:
a2) Providing a monosaccharide building block of formula **14** wherein R¹ is selected from -Bz, -ClAc, -Ac and -Piv, R³ is selected from -Me, -Et, -Ph, -tBu and -PMP and Z¹ is selected from -O-C(=NH)-CCl₃, -O-C(=NPh)-CF₃, -OAc and -SR⁵ with R⁵ is selected from -Me, -Et, -Pr, -Bu, -Ph, and -Tol and a monosaccharide building block **15** wherein R² is selected from -Bz, -Ac, -Piv, -Bn and -TBS, X represents -OAc or -SEt;
b2) Coupling of the monosaccharide building block of formula **14** with the monosaccharide building block of formula **15** in presence of a catalyst to yield disaccharide of formula **2**.

It is even more preferred that the method for the preparation of disaccharide of formula **2** comprises the following steps:
a2) Providing a monosaccharide building block of formula **14** wherein R¹ is selected from -Bz, -ClAc, -Ac and -Piv, R³ is selected from -Me, -Et, -Ph, -tBu and -PMP and Z¹ represents -O-C(=NPh)-CF₃ and a monosaccharide building block **15** wherein R² is selected from -Bz, -Ac, -Piv, -Bn and -TBS, X represents -OAc or -SR⁵ and R⁵ is selected from -Me, -Et, -Pr, -Bu, -Ph, and -Tol;
b2) Coupling of the monosaccharide building block of formula **14** with the monosaccharide building block of formula **15** in presence of a catalyst to yield disaccharide of formula **2**

It is even more preferred that the method for the preparation of disaccharide of formula **2** comprises the following steps:
a2) Providing a monosaccharide building block of formula **14** wherein R¹ is selected from -Bz, and -Ac, R³ represents -Ph, and Z¹ represents -O-C(=NPh)-CF₃ and a monosaccharide building block **15** wherein R² represents -Bn and X is -OAc or -SEt; b2) Coupling of the monosaccharide building block of formula **14** with the monosaccharide building block of formula **15** in presence of a catalyst to yield disaccharide of formula **2**.

Preferably, the coupling of the monosaccharide building block of formula **14** with the monosaccharide building block of formula **15** is performed in the presence of catalytic amounts of a Lewis acid in an aprotic apolar solvent at temperatures between -78 °C and -20 °C. More preferably, the coupling of monosaccharide building block of formula **14** with monosaccharide building block of formula **15** is performed in the presence of catalytic amounts of TMSOTf or TfOH in an aprotic apolar solvent at temperatures between -78 °C and -20 °C. More preferably, the coupling of monosaccharide building block of formula **14** with monosaccharide building block of formula **15** is performed in the presence of catalytic amounts of TMSOTf or TfOH in methylene chloride or toluene at temperatures between -78 °C and -20 °C. More preferably, the coupling of monosaccharide building block of formula **14** with monosaccharide building block of formula **15** is performed in the presence of catalytic amounts of TMSOTf or TfOH in an aprotic apolar solvent at temperatures between -78 °C and -20 °C. Even more preferably, the coupling of monosaccharide building block of formula **14** with monosaccharide building block of formula **15** is performed in the presence of catalytic amounts of a Lewis acid in an aprotic apolar solvent at temperatures between -60 °C and -20 °C. Even more preferably, the coupling of monosaccharide building block of formula **14** with monosaccharide building block of formula **15** is performed in the presence of catalytic amounts of a Lewis acid in an aprotic apolar solvent at temperatures between -60 °C and 0 °C. Even more preferably, the coupling of monosaccharide building block of formula **14** with monosaccharide building block of formula **15** is performed in the presence of catalytic amounts of TMSOTf or TfOH in methylene chloride or toluene at -40 °C. According to the present invention the disaccharide of formula **2** is preferably obtained in form of crystals by recrystallization.

Thus, It is especially preferred that the method for the preparation of disaccharide of formula **2** comprises the following steps:
a2) Providing a monosaccharide building block of formula **14** wherein R¹ represents -Bz, R³ is a phenyl group and Z¹ is -O-C(=NPh)-CF₃; and a monosaccharide building block **15** with R² is -Bn and X represents -OAc or -SEt b2) Coupling of the monosaccharide building block of formula **14** with the monosaccharide building block of formula **15** in presence of catalytic amounts of TMSOTf or TfOH to yield disaccharide of formula **2**.

### Preparation of disaccharide of formula 3-A

The preparation of a disaccharide of formula **3-A** according to the present invention comprises the following steps:
a6) Reacting the disaccharide of formula **2** with an alcohol HO-W in presence of a catalyst to yield a disaccharide of formula **9** 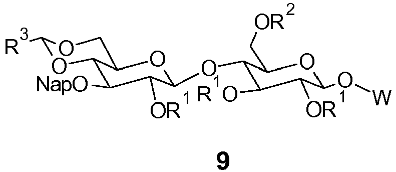 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl
      or R' and R" form together phthaloyl,
   L represents -(CH₂)ₙ- or -C₂H₄-O-C₂H₄-,
   n is an integer selected from 2, 3, 4, 5, 6, 7, 8, 9 and 10;
b6) Converting the disaccharide of formula **9** to the disaccharide of formula **3-A** by treatment with an oxidizing agent; 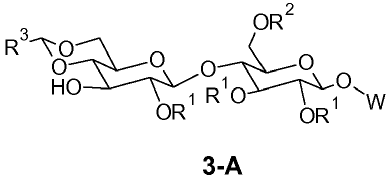 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn
   P is -NR'R" or -N₃,
   R' and R" represent independently of each other -Bn, -Cbz, -Allyl
      or R' and R" form together phthaloyl,
   L represents -(CH₂)ₙ- or -C₂H₄-O-C₂H₄-,
   n is an integer selected from 2, 3, 4, 5, 6, 7, 8, 9 and 10.

Preferably, the inventive method for preparation of a disaccharide of formula **3-A** comprises the following steps: a6) Reacting the disaccharide of formula **2** with an alcohol HO-W in presence of a catalyst to yield a disaccharide of formula **9** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10; b6) Converting the disaccharide of formula **9** to the disaccharide of formula **3-A** by treatment with an oxidizing agent.

More preferably, the inventive method for preparation of a disaccharide of formula **3-A** comprises the following steps a6) Reacting the disaccharide of formula **2** with an alcohol HO-W in presence of a catalyst to yield a disaccharide of formula **9** wherein R¹ represents -Bz, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10; b6) Converting the disaccharide of formula **9** to the disaccharide of formula **3-A** by treatment with an oxidizing agent.

More preferably, the inventive method for preparation of a disaccharide of formula **3-A** comprises the following steps: a6) Reacting the disaccharide of formula **2** with an alcohol HO-W in presence of a catalyst to yield a disaccharide of formula **9** wherein R¹ is selected from -Bz, -ClAc, -Ac and -Piv, R² represents -Bn, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10; b6) Converting the disaccharide of formula **9** to the disaccharide of formula **3-A** by treatment with an oxidizing agent.

More preferably, the inventive method for preparation of a disaccharide of formula **3-A** comprises the following steps: a6) Reacting the disaccharide of formula **2** with an alcohol HO-W in presence of a catalyst to yield a disaccharide of formula **9** wherein R¹ is selected from -Bz, -ClAc, -Ac and -Piv, R² is selected from -Bz, -Ac, -Piv, -Bn and -TBS, R³ represents -Ph, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10; b6) Converting the disaccharide of formula **9** to the disaccharide of formula **3-A** by treatment with an oxidizing agent.

More preferably, the inventive method for preparation of a disaccharide of formula **3-A** comprises the following steps: a6) Reacting the disaccharide of formula **2** with an alcohol HO-W in presence of a catalyst to yield a disaccharide of formula **9** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -N(Bn)Cbz, L represents -C₂H₄-O-C₂H₄-or -(CH₂)ₙ-, n is an integer selected from 2, 3, 4, 5, 6, 7, 8, 9 and 10; and b6) Converting the disaccharide of formula **9** to the disaccharide of formula **3-A** by treatment with an oxidizing agent.

More preferably, the inventive method for preparation of a disaccharide of formula **3-A** comprises the following steps: a6) Reacting the disaccharide of formula **2** with an alcohol HO-W in presence of a catalyst to yield a disaccharide of formula **9** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -(CH₂)ₙ-, n is an integer selected from 2, 3, 4, and 5; b6) Converting the disaccharide of formula **9** to the disaccharide of formula **3-A** by treatment with an oxidizing agent.

More preferably, the inventive method for preparation of a disaccharide of formula **3-A** comprises the following steps: a6) Reacting the disaccharide of formula **2** with an alcohol HO-W in presence of a catalyst to yield a disaccharide of formula **9** wherein R¹ is selected from -Bz and -Ac, R² represents -Bn, R³ represents -Ph, W represents -L-P or -Bn, P is -N(Bn)Cbz or -N₃, L represents -(CH₂)ₙ-, n is an integer selected from 2, 3, 4 and 5; b6) Converting the disaccharide of formula **9** to the disaccharide of formula **3-A** by treatment with an oxidizing agent.

It is preferred that the reaction of disaccharide of formula **2** with an alcohol HO-W to disaccharide of formula **9** is performed in the presence of NIS and catalytic amounts of a strong acid in an aprotic apolar solvent at low temperatures between -30 °C and 10 °C. It is more preferred that the reaction of disaccharide of formula **2** with an alcohol HO-W to disaccharide of formula **9** is performed in the presence of NIS and catalytic amounts of trifluoromethanesulfonic acid in an aprotic apolar solvent at low temperatures between -30 °C and 10 °C. It is more preferred that the reaction of disaccharide of formula **2** with an alcohol HO-W to disaccharide of formula **9** is performed in the presence of NIS and catalytic amounts of trifluoromethanesulfonic acid in methylene chloride at low temperatures between -30 °C and 10 °C. It is even more preferred that the reaction of disaccharide of formula **2** with an alcohol HO-W to disaccharide of formula **9** is performed in the presence of NIS and catalytic amounts of trifluoromethanesulfonic acid in methylene chloride at low temperatures between -20 °C and 0 °C. It is especially preferred that the reaction of disaccharide of formula **2** with an alcohol HO-W to disaccharide of formula **9** is performed in the presence of NIS and catalytic amounts of trifluoromethanesulfonic acid in methylene chloride at low temperatures between -10 °C and 0 °C. According to the present invention the disaccharide of formula **9** is preferably obtained in form of crystals by recrystallization.

Preferably, the conversion of disaccharide of formula **9** to disaccharide of formula **3-A** is performed by treatment with an oxidizing agent in an aprotic apolar solvent at lowered temperatures between -10 °C and 20 °C. More preferably, the conversion of disaccharide of formula **9** to disaccharide of formula **3-A** is performed by treatment with DDQ in an aprotic apolar solvent at lowered temperatures between -10 °C and 20 °C. More preferably, the conversion of disaccharide of formula **9** to disaccharide of formula **3-A** is performed by treatment with DDQ in methylene chloride at lowered temperatures between -10 °C and 20 °C. Even more preferably, the conversion of disaccharide of formula **9** to disaccharide of formula **3-A** is performed by treatment with DDQ in methylene chloride at lowered temperatures between 0 °C and 10 °C.

Thus, it is especially preferred that the inventive method for preparation of a disaccharide of formula **3-A** comprises the following steps: a6) Reacting the disaccharide of formula **2** with an alcohol HO-W; in presence of NIS and catalytic amounts of trifluoromethanesulfonic acid to yield a disaccharide of formula **9** wherein R¹ represents -Bz, R² is -Bn, R³ is a phenyl group, W represents -L-P or -Bn, P is -N(Bn)Cbz, L represents -(CH₂)ₙ-, n is an integer selected from 2, 3, 4 and 5; and b6) Converting the disaccharide of formula **9** to the disaccharide of formula **3-A** by treatment with DDQ.

### Coupling of disaccharide of formula 2 and saccharide of formula 3 to protected saccharide of formula 1

The preparation of protected saccharide of formula **1** according to a first aspect of the present invention comprises the following steps:
A) Providing a disaccharide of formula **2** 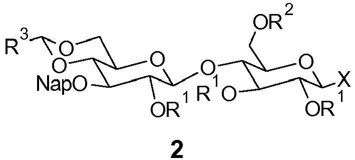 wherein
   Nap represents 2-naphthylmethyl group,
   R¹ represents an orthogonal oxidation-stable and acid-stable protecting group,
   R² represents an orthogonal oxidation-stable and acid-stable protecting group,
   R³ is an aryl or alkyl group, and
   X represents -OAc or -SR⁵, with R⁵ being selected from -Me, -Et, -Pr, -Bu, -Ph and -Tol;
   and a saccharide of formula **3** 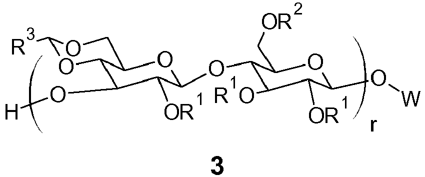 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
   R' and R" form together phthaloyl,
   L represents -(CH₂)ₙ- or -C₂H₄-O-C₂H₄-,
   n is an integer selected from 2, 3, 4, 5, 6, 7, 8, 9 and 10,
   r is 1, 2 or 3;
B) Coupling of the disaccharide of formula **2** with the saccharide of formula **3** in presence of a catalyst to obtain a saccharide of formula **1** 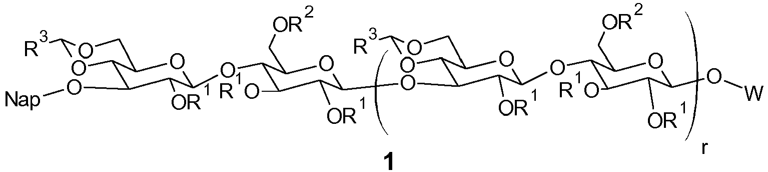 wherein
   Nap represents 2-naphthylmethyl group,
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
   R' and R" form together phthaloyl,
   L represents -(CH₂)ₙ- or -C₂H₄-O-C₂H₄-,
   n is an integer selected from 2, 3, 4, 5, 6, 7, 8, 9 and 10, and
   r is 1, 2 or 3.

### Preparation of saccharide of formula 4 or saccharide of formula 11

In a second aspect, the inventive method according to aspect 1 further comprises performing selective removal of the acetal groups by treatment of the saccharide of formula **1** with a nucleophile in presence of a catalytic amount of an acid to yield a saccharide of formula **4**, 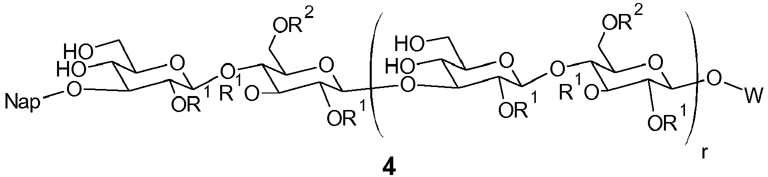 wherein
Nap represents 2-naphthylmethyl group,
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3;
or
performing reductive removal of the acetal groups by treatment of the saccharide of formula **1** according to aspect 1 with a borane in presence of a catalytic amount of a Lewis acid to yield a saccharide of formula **11**, 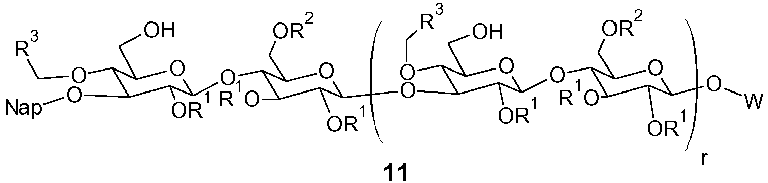 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3.

### Preparation of saccharide of formula 5 or saccharide of formula 12

In a third aspect, the inventive method according to aspect 2 further comprises performing oxidation of the saccharide of formula **4** to yield a saccharide of formula **5**, 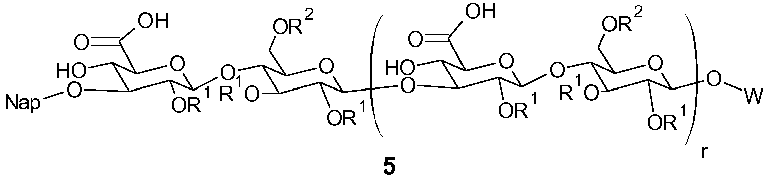 wherein
Nap represents 2-naphthylmethyl group,
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-, n is an integer selected from 2, 3, 4, 5, 6, 7, 8, 9 and 10, and
r is 1, 2 or 3;
or
performing oxidation of the saccharide of formula **11** according to aspect 2 to yield a saccharide of formula **12**, 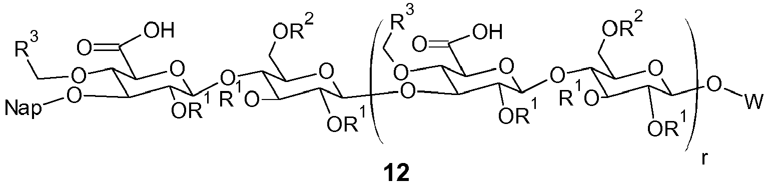 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3.

### Preparation of saccharide of formula 7 or saccharide of formula 13

In a fourth aspect, the inventive method according to aspect 3 further comprises performing removal of protecting groups R¹ of saccharide of formula **5** according to aspect 3 in presence of a base to yield a saccharide of formula **7**, 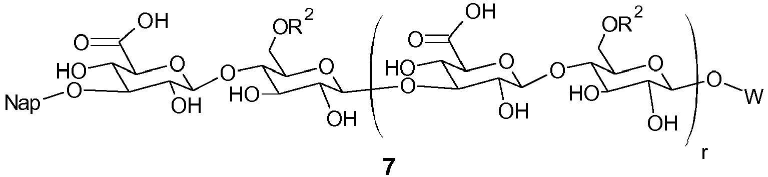 wherein
Nap represents 2-naphthylmethyl group,
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3;
or
performing removal of protecting groups R¹ of saccharide of formula 12 according to aspect 3 in presence of a base to yield a saccharide of formula **13**, 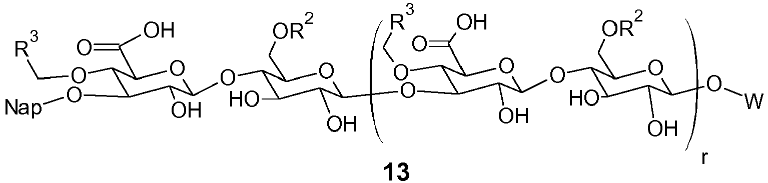 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3.

### Preparation of saccharide of formula 8 or saccharide of formula 8'

In a fifth aspect, the inventive method according to aspect 4 further comprises hydrogenating the saccharide of formula 7 or formula **13** in presence of a catalyst to yield a saccharide of formula **8** or a saccharide of formula **8'**, 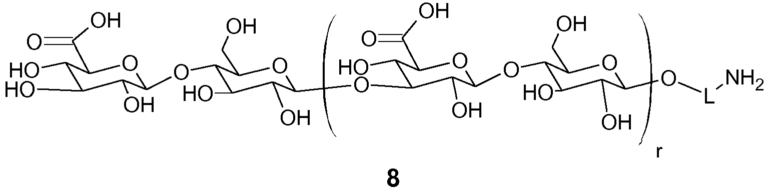 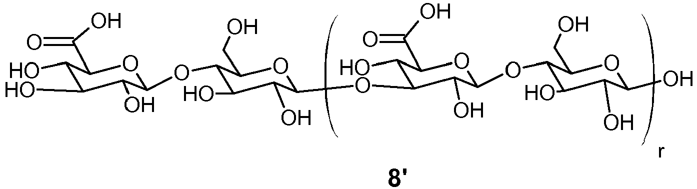
wherein L represents -(CH₂)ₙ- or -C₂H₄-O-C₂H₄-,
n is an integer selected from 2, 3, 4, 5, 6, 7, 8, 9 and 10, and
r is 1, 2 or 3.

The inventive method according to any one of aspects 1 to 5 further comprises converting the saccharide of formula **1** to the saccharide of formula **3** by treatment with an oxidizing agent.

### Coupling of disaccharide of formula 2 and disaccharide of formula 3-A to protected tetrasaccharide of formula 1-A

The preparation of protected tetrasaccharide of formula **1-A** according to the present invention comprises the following steps:
A) Providing a disaccharide of formula **2** 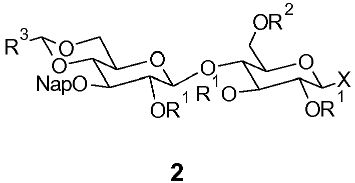 wherein
   R¹ represents an orthogonal oxidation-stable and acid-stable protecting group,
   R² represents an orthogonal oxidation-stable and acid-stable protecting group,
   R³ is an aryl or alkyl group, and
   X represents -OAc or -SR⁵, with R⁵ being selected from -Me, -Et, -Pr, -Bu, -Ph and -Tol;
   and a disaccharide of formula **3-A** 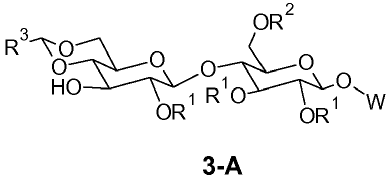 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
   R' and R" form together phthaloyl,
   L represents -(CH₂)ₙ- or -C₂H₄-O-C₂H₄-,
   n is an integer selected from 2, 3, 4, 5, 6, 7, 8, 9 and 10;
B) Coupling of the disaccharide of formula **2** with the disaccharide of formula **3-A** in the presence of a catalyst to obtain a tetrasaccharide of formula **1-A** 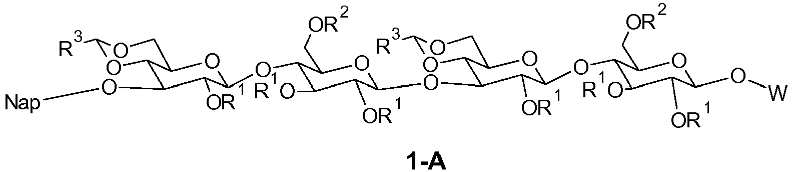 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
   R' and R" form together phthaloyl,
   L represents -(CH₂)ₙ- or -C₂H₄-O-C₂H₄-,,
   n is an integer selected from 2, 3, 4, 5, 6, 7, 8, 9 and 10.

In another aspect of the present invention the preparation of protected tetrasaccharide of formula **1*** comprises the following steps:
A) Providing a disaccharide of formula **2** 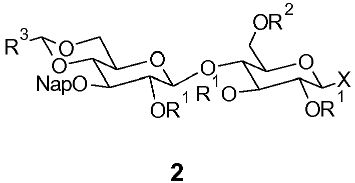 wherein
   R¹ represents an oxidation-stable and acid-stable protecting group,
   R² represents an oxidation-stable and acid-stable protecting group,
   R³ is an aryl or alkyl group, and
   X represents -OAc or -SR⁵, with R⁵ being selected from -Me, -Et, -Pr, -Bu, -Ph and -Tol;
   and a disaccharide of formula **3*** 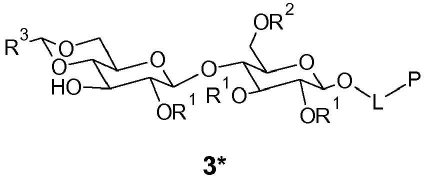 wherein
   R¹ represents an oxidation-stable and acid-stable protecting group,
   R² represents an oxidation-stable and acid-stable protecting group,
   R³ is an aryl or alkyl group,
   P is -NR'R" or -N₃,
   R' and R" represent independently of each other -Bn, -Cbz, -Allyl or
   R' and R" form together phthaloyl,
   L represents -(CH₂)ₙ-,
   n is an integer selected from 2, 3, 4, 5, 6, 7, 8, 9 and 10;
B) Coupling of the disaccharide of formula **2** with the disaccharide of formula **3*** in the presence of a catalyst to obtain a tetrasaccharide of formula **1*** 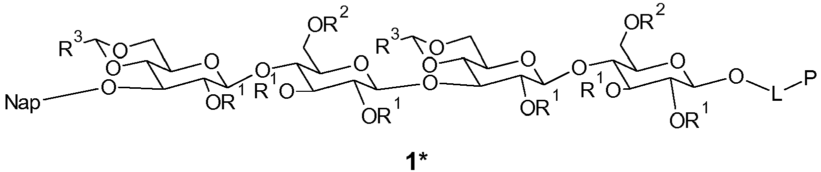 wherein
   R¹ represents an oxidation-stable and acid-stable protecting group,
   R² represents an oxidation-stable and acid-stable protecting group,
   R³ is an aryl or alkyl group,
   P is -NR'R" or -N₃,
   R' and R" represent independently of each other -Bn, -Cbz, -Allyl or R' and R" form together phthaloyl,
   L represents -(CH₂)ₙ-,
   n is an integer selected from 2, 3, 4, 5, 6, 7, 8, 9 and 10.

It is preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a disaccharide of formula **3-A** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the disaccharide of formula **3-A** in the presence of a catalyst to obtain a tetrasaccharide of formula **1-A.**

It is more preferred that the inventive method comprises the following steps:
A) Providing a disaccharide of formula **2** wherein R¹ represents -Bz, R² is selected from -Bz, -Ac, -Piv, -Bn and -TBS, R³ is selected from -Me, -Et, -Ph, -tBu and -PMP, X represents -OAc or -SR⁵, and R⁵ is selected from -Me, -Et, -Pr, -Bu, -Ph, and -Tol and a disaccharide of formula **3-A** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the disaccharide of formula **3-A** in the presence of a catalyst to obtain a tetrasaccharide of formula **1-A.**

It is more preferred that the inventive method comprises the following steps:
A) Providing a disaccharide of formula **2** wherein R¹ is selected from -Bz, -ClAc, -Ac and -Piv, R² represents -Bn, R³ is selected from -Me, -Et, -Ph, -tBu or -PMP, X represents -OAc or -SR⁵ and R⁵ is selected from -Me, -Et, -Pr, -Bu, -Ph, and -Tol and a disaccharide of formula **3-A** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the disaccharide of formula **3-A** in the presence of a catalyst to obtain a tetrasaccharide of formula **1-A.**

It is more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ represents -Ph, X represents -OAc or -SR⁵ and R⁵ is selected from -Me, -Et, -Pr, -Bu, -Ph, and -Tol and a disaccharide of formula **3-A** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the disaccharide of formula **3-A** in the presence of a catalyst to obtain a tetrasaccharide of formula **1-A.**

It is even more preferred that the inventive method comprises the following steps: A) Providing a disaccharide of formula **2** wherein R¹ is selected from -Bz, -ClAc, -Ac or -Piv, R² is selected from -Bz, -Ac, -Piv, -Bn and -TBS, R³ is selected from -Me, -Et, -Ph, -tBu and -PMP, X represents -OAc or -SEt and a disaccharide of formula **3-A** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the disaccharide of formula **3-A** in the presence of a catalyst to obtain a tetrasaccharide of formula **1-A.**

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a disaccharide of formula **3-A** with W representing -L-P or -Bn, P is -N(Bn)Cbz, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the disaccharide of formula **3-A** in the presence of a catalyst to obtain a tetrasaccharide of formula **1-A**.

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a disaccharide of formula **3-A** with W representing -L-P or -Bn, P is -NR'R", R' and R" represent independently of each other -Bn, -Cbz, -Allyl or R' and R" form together phthaloyl, L represents -(CH₂)ₙ-, n is an integer selected from 2, 3, 4, and 5; B) Coupling of the disaccharide of formula **2** with the disaccharide of formula **3-A** in the presence of a catalyst to obtain a tetrasaccharide of formula **1-A**.

It is even more preferred that the inventive method comprises the following steps:
A) Providing a disaccharide of formula **2** wherein R¹ is selected from -Bz and -Ac, R² represents -Bn, R³ represents -Ph, X represents -OAc or -SEt and a disaccharide of formula **3-A** wherein W represents -L-P or -Bn, P is -N(Bn)Cbz or N₃, L represents -(CH₂)ₙ-, n is an integer selected from 2, 3, 4, and 5; B) Coupling of the disaccharide of formula **2** with the disaccharide of formula **3-A** in the presence of a catalyst to obtain a tetrasaccharide of formula **1-A**.

Preferably, the coupling reaction of disaccharide of formula **2** with the disaccharide of formula **3-A** is performed in the presence of NIS and catalytic amounts of a strong acid in an aprotic apolar solvent at low temperatures between -20 °C and 10 °C. More preferably, the coupling reaction of disaccharide of formula **2** with the disaccharide of formula **3-A** is performed in the presence of NIS and catalytic amounts of trifluoromethanesulfonic acid in an aprotic apolar solvent at low temperatures between -20 °C and 10 °C. More preferably, the coupling reaction of disaccharide of formula **2** with the disaccharide of formula **3-A** is performed in the presence of NIS and catalytic amounts of trifluoromethanesulfonic acid in methylene chloride at low temperatures between -20 °C and 5 °C. Even more preferably, the coupling reaction of disaccharide of formula **2** with the disaccharide of formula **3-A** is performed in the presence of NIS and catalytic amounts of trifluoromethanesulfonic acid in methylene chloride at low temperatures between -10 °C and 0 °C. According to the present invention the protected tetrasaccharide of formula **1-A** is preferably obtained in form of crystals by recrystallization or trituration.

Thus, it is especially preferred that the inventive method for preparation of protected tetrasaccharide of formula **1-A** comprises the following steps: A) Providing a disaccharide of formula **2** with R¹ is -Bz, R² is -Bn, R³ is a phenyl group, X represents -OAc or -SEt and a disaccharide of formula **3-A** with W representing -L-P or -Bn, P is -N(Bn)Cbz, L represents -(CH₂)ₙ-, n is an integer selected from 2, 3, 4 and 5; B) Coupling of the disaccharide of formula **2** with the disaccharide of formula **3-A** in the presence of NIS and catalytic amounts of trifluoromethanesulfonic acid to obtain a tetrasaccharide of formula **1-A.**

### Preparation of tetrasaccharide of formula 4-A

The preparation of tetrasaccharide of formula **4-A** according to the present invention comprises the following step:
Performing selective removal of acetal groups by treatment of the tetrasaccharide of formula **1-A** with a nucleophile in presence of a catalytic amount of an acid to yield a tetrasaccharide of formula **4-A,** 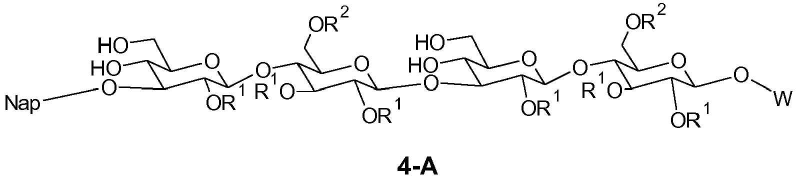 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of tetrasaccharide of formula **4-A** comprises the following step: Performing selective removal of acetal groups by treatment of the tetrasaccharide of formula **1-A** with a nucleophile in presence of a catalytic amount of an acid to yield a tetrasaccharide of formula **4-A,** wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide of formula **4-A** comprises the following step: Performing selective removal of acetal groups by treatment of the tetrasaccharide of formula **1-A** with a nucleophile in presence of a catalytic amount of an acid to yield a tetrasaccharide of formula **4-A,** wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide of formula **4-A** comprises the following step: Performing selective removal of acetal groups by treatment of the tetrasaccharide of formula **1-A** with a nucleophile in presence of a catalytic amount of an acid to yield a tetrasaccharide of formula **4-A,** wherein R¹ is selected from -Bz, -ClAc, -Ac and -Piv, R² represents -Bn, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide of formula **4-A** comprises the following step: Performing selective removal of acetal groups by treatment of the tetrasaccharide of formula **1-A** with a nucleophile in presence of a catalytic amount of an acid to yield a tetrasaccharide of formula **4-A,** wherein R¹ is selected from -Bz, -ClAc, -Ac and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of tetrasaccharide of formula **4-A** comprises the following step: Performing selective removal of acetal groups by treatment of the tetrasaccharide of formula **1-A** with a nucleophile in presence of a catalytic amount of an acid to yield a tetrasaccharide of formula **4-A,** wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of tetrasaccharide of formula **4-A** comprises the following step: Performing selective removal of acetal groups by treatment of the tetrasaccharide of formula **1-A** with a nucleophile in presence of a catalytic amount of an acid to yield a tetrasaccharide of formula **4-A,** wherein R¹ is selected from -Bz and -Ac, R² represents -Bn, P is -N(Bn)Cbz or N₃, L represents -(CH₂)ₙ- and n is an integer selected from 2, 3, 4 and 5.

It is preferred that the selective removal of acetal groups of tetrasaccharide of formula **1-A** is performed in the presence of catalytic amounts of an acid and a nucleophile, such as a thiol, water or an alcohol. It is even more preferred that the selective removal of acetal groups of tetrasaccharide of formula **1-A** is performed in the presence of catalytic amounts of a sulfonic acid and a thiol. It is even more preferred that the selective removal of acetal groups of tetrasaccharide of formula **1-A** is performed in the presence of catalytic amounts of PTSA and a thiol. It is especially preferred that the selective removal of acetal groups of tetrasaccharide of formula **1-A** is performed in the presence of catalytic amounts of PTSA and ethanethiol. According to the present invention the tetrasaccharide of formula **4-A** is preferably obtained in form of crystals by recrystallization or trituration.

Thus, it is especially preferred that the inventive method for preparation of tetrasaccharide of formula **4-A** comprises the following step: Performing selective removal of acetal groups by treatment of the tetrasaccharide of formula **1-A** with ethanethiol in presence of a catalytic amount of PTSA to yield a tetrasaccharide of formula **4-A,** wherein R¹ is -Bz, R² is -Bn, W represents -L-P or -Bn, P is -N(Bn)CBz, L represents -(CH₂)ₙ- and n is an integer selected from 2, 3, 4 and 5.

### Preparation of tetrasaccharide of formula 11-A

In an alternative embodiment of the present invention the tetrasaccharide of formula **11-A** is prepared by
performing reductive removal of the acetal groups by treatment of the tetrasaccharide of formula **1-A** with a borane in presence of a catalytic amount of a Lewis acid to yield a saccharide of formula **11-A**, 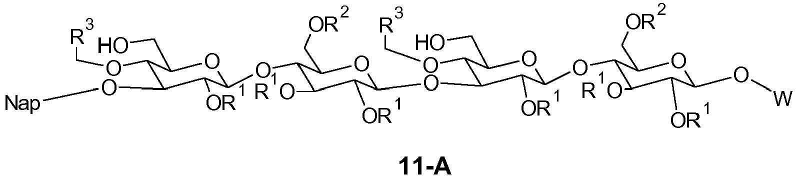 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of tetrasaccharide of formula **11-A** comprises the following step: Performing reductive removal of the acetal groups by treatment of the tetrasaccharide of formula **1-A** with a borane in presence of a catalytic amount of a Lewis acid to yield a saccharide of formula **11-A**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide of formula **11-A** comprises the following step: Performing reductive removal of the acetal groups by treatment of the tetrasaccharide of formula **1-A** with a borane in presence of a catalytic amount of a Lewis acid to yield a saccharide of formula **11-A**, wherein R¹ represents -Bz, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide of formula **11-A** comprises the following step: Performing reductive removal of the acetal groups by treatment of the tetrasaccharide of formula **1-A** with a borane in presence of a catalytic amount of a Lewis acid to yield a saccharide of formula **11-A**, wherein R¹ is selected from -Bz, -ClAc, -Ac and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide of formula **11-A** comprises the following step: Performing reductive removal of the acetal groups by treatment of the tetrasaccharide of formula **1-A** with a borane in presence of a catalytic amount of a Lewis acid to yield a saccharide of formula **11-A**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of tetrasaccharide of formula **11-A** comprises the following step: Performing reductive removal of the acetal groups by treatment of the tetrasaccharide of formula **1-A** with a borane in presence of a catalytic amount of a Lewis acid to yield a saccharide of formula **11-A**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of tetrasaccharide of formula **11-A** comprises the following step: Performing reductive removal of the acetal groups by treatment of the tetrasaccharide of formula **1-A** with a borane in presence of a catalytic amount of a Lewis acid to yield a saccharide of formula **11-A**, wherein R¹ is selected from -Bz and -Ac, R² represents -Bn, P is -N(Bn)Cbz or N₃, R³ is -Ph, L represents -(CH₂)ₙ- and n is an integer selected from 2, 3, 4 and 5.

It is preferred that the reductive removal of acetal groups of tetrasaccharide of formula **1-A** is performed in the presence of catalytic amounts of a Lewis acid and a borane, such as BH₃, BH₃•NMe₂, BH₃•THF. It is even more preferred that the reductive removal of acetal groups of tetrasaccharide of formula **1-A** is performed in the presence of catalytic amounts of silver trifluoromethanesulfonate and BH₃. According to the present invention the tetrasaccharide of formula **11-A** is preferably obtained in form of crystals by recrystallization or trituration.

Thus, it is especially preferred that the inventive method for preparation of tetrasaccharide of formula **11-A** comprises the following step: Performing reductive removal of the acetal groups by treatment of the tetrasaccharide of formula **1-A** with BH₃ in presence of a catalytic amount of silver trifluoromethanesulfonate to yield a saccharide of formula **11-A**, wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of tetrasaccharide diacid of formula 5-A

The preparation of tetrasaccharide diacid of formula **5-A** according to the present invention comprises the following step:
Performing oxidation of the tetrasaccharide of formula **4-A** to yield a tetrasaccharide diacid of formula **5-A,** 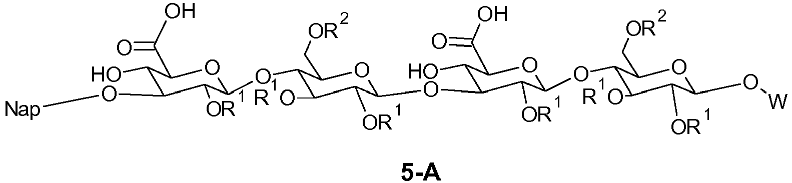 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of tetrasaccharide diacid of formula **5-A** comprises the following step: Performing oxidation of the tetrasaccharide of formula **4-A** to yield a tetrasaccharide diacid of formula **5-A,** wherein R¹ is selected from -Bz, -ClAc, -Ac, and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide diacid of formula **5-A** comprises the following step: Performing oxidation of the tetrasaccharide of formula **4-A** to yield a tetrasaccharide diacid of formula **5-A,** wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

More preferably the inventive method for preparation of tetrasaccharide diacid of formula **5-A** comprises the following step: Performing oxidation of the tetrasaccharide of formula **4-A** to yield a tetrasaccharide diacid of formula **5-A,** wherein R¹ is selected from -Bz, -ClAc, -Ac, and -Piv, R² represents -Bn, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

More preferably the inventive method for preparation of tetrasaccharide diacid of formula **5-A** comprises the following step: Performing oxidation of the tetrasaccharide of formula **4-A** to yield a tetrasaccharide diacid of formula **5-A,** wherein R¹ is selected from -Bz, -ClAc, -Ac, and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of tetrasaccharide diacid of formula **5-A** comprises the following step: Performing oxidation of the tetrasaccharide of formula **4-A** to yield a tetrasaccharide diacid of formula **5-A,** wherein R¹ is selected from -Bz, -ClAc, -Ac, and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of tetrasaccharide diacid of formula **5-A** comprises the following step: Performing oxidation of the tetrasaccharide of formula **4-A** to yield a tetrasaccharide diacid of formula **5-A,** wherein R¹ is selected from -Bz and -Ac, R² represents -Bn, W represents -L-P or -Bn, P is -N(Bn)Cbz or N₃, L represents -(CH₂)ₙ- and n is an integer selected from 2, 3, 4 and 5.

It is preferred that the oxidation of tetrasaccharide of formula **4-A** is performed in the presence of at least one oxidizing agent in a mixture of water and an aprotic apolar solvent at low temperatures between -10 °C and 10 °C. It is even more preferred that the oxidation of tetrasaccharide of formula **4-A** is performed in the presence of a catalytic amount of a first oxidizing agent and a second oxidizing agent in a mixture of water and an aprotic apolar solvent at low temperatures between -10 °C and 10 °C. It is even more preferred that the oxidation of tetrasaccharide of formula **4-A** is performed in the presence of a catalytic amount of TEMPO and a second oxidizing agent in a mixture of water and an aprotic apolar solvent at low temperatures between -10 °C and 10 °C. It is even more preferred that the oxidation of tetrasaccharide of formula **4-A** is performed in the presence of a catalytic amount of TEMPO and bis(acetoxy)iodobenzene (BAIB) in a mixture of water and an aprotic apolar solvent at low temperatures between -10 °C and 10 °C. It is even more preferred that the oxidation of tetrasaccharide of formula **4-A** is performed in the presence of a catalytic amount of TEMPO and bis(acetoxy)iodobenzene (BAIB) in a mixture of water and methylene chloride at low temperatures between -10 °C and 10 °C. It is especially preferred that the oxidation of tetrasaccharide of formula **4-A** is performed in the presence of a catalytic amount of TEMPO and bis(acetoxy)iodobenzene (BAIB) in a mixture of water and methylene chloride at 0 °C. According to the present invention the tetrasaccharide diacid of formula **5-A** is preferably obtained in form of crystals by recrystallization or trituration.

Thus, it is especially preferred that the inventive method for preparation of tetrasaccharide diacid of formula **5-A** comprises the following step: Performing oxidation of the tetrasaccharide of formula **4-A** with a catalytic amount of TEMPO and BAIB to yield a tetrasaccharide diacid of formula **5-A**, wherein R¹ is -Bz, R² is -Bn, W represents -L-P or -Bn, P is -N(Bn)CBz, L represents -(CH₂)ₙ- and n is an integer selected from 2, 3, 4 and 5.

### Preparation of tetrasaccharide diacid of formula 12-A

The preparation of tetrasaccharide diacid of formula **12-A** according to the present invention comprises the following step:
Performing oxidation of the tetrasaccharide of formula **11-A** to yield a tetrasaccharide diacid of formula **12-A,** 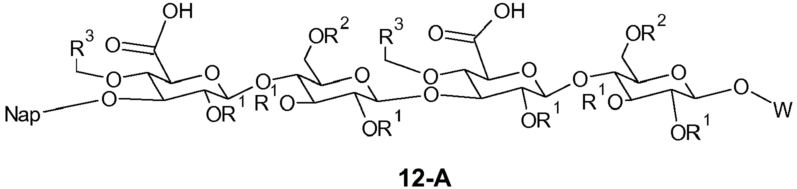 wherein
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of tetrasaccharide diacid of formula **12-A** comprises the following step: Performing oxidation of the tetrasaccharide of formula **11-A** to yield a tetrasaccharide diacid of formula **12-A,** wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide diacid of formula **12-A** comprises the following step: Performing oxidation of the tetrasaccharide of formula **11-A** to yield a tetrasaccharide diacid of formula **12-A,** wherein R¹ represents -Bz, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide diacid of formula **12-A** comprises the following step: Performing oxidation of the tetrasaccharide of formula **11-A** to yield a tetrasaccharide diacid of formula **12-A,** wherein R¹ is selected from -Bz, -ClAc, -Ac, and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of tetrasaccharide diacid of formula **12-A** comprises the following step: Performing oxidation of the tetrasaccharide of formula **11-A** to yield a tetrasaccharide diacid of formula **12-A,** wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of tetrasaccharide diacid of formula **12-A** comprises the following step: Performing oxidation of the tetrasaccharide of formula **11-A** to yield a tetrasaccharide diacid of formula **12-A,** wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of tetrasaccharide diacid of formula **12-A** comprises the following step: Performing oxidation of the tetrasaccharide of formula **11-A** to yield a tetrasaccharide diacid of formula **12-A,** wherein R¹ is selected from -Bz and -Ac, R² represents -Bn, R³ is -Ph, W represents -L-P or -Bn, P is -N(Bn)Cbz or N₃, L represents -(CH₂)ₙ- and n is an integer selected from 2, 3, 4 and 5.

It is preferred that the oxidation of tetrasaccharide of formula **11-A** is performed in the presence of at least one oxidizing agent in a mixture of water and an aprotic apolar solvent at low temperatures between -10 °C and 10 °C. It is even more preferred that the oxidation of tetrasaccharide of formula **11-A** is performed in the presence of a catalytic amount of a first oxidizing agent and a second oxidizing agent in a mixture of water and an aprotic apolar solvent at low temperatures between -10 °C and 10 °C. It is even more preferred that the oxidation of tetrasaccharide of formula **11-A** is performed in the presence of a catalytic amount of TEMPO and a second oxidizing agent in a mixture of water and an aprotic apolar solvent at low temperatures between -10 °C and 10 °C. It is even more preferred that the oxidation of tetrasaccharide of formula **11-A** is performed in the presence of a catalytic amount of TEMPO and bis(acetoxy)iodobenzene (BAIB) in a mixture of water and an aprotic apolar solvent at low temperatures between -10 °C and 10 °C. It is even more preferred that the oxidation of tetrasaccharide of formula **11-A** is performed in the presence of a catalytic amount of TEMPO and bis(acetoxy)iodobenzene (BAIB) in a mixture of water and methylene chloride at low temperatures between -10 °C and 10 °C. It is especially preferred that the oxidation of tetrasaccharide of formula **11-A** is performed in the presence of a catalytic amount of TEMPO and bis(acetoxy)iodobenzene (BAIB) in a mixture of water and methylene chloride at 0 °C. According to the present invention the tetrasaccharide diacid of formula **12-A** is preferably obtained in form of crystals by recrystallization or trituration.

Thus, it is especially preferred that the inventive method for preparation of tetrasaccharide diacid of formula **12-A** comprises the following step: Performing oxidation of the tetrasaccharide of formula **11-A** with a catalytic amount of TEMPO and BAIB to yield a tetrasaccharide diacid of formula **12-A**, wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of tetrasaccharide of formula 7-A

The preparation of tetrasaccharide of formula **7-A** according to the present invention comprises the following step: Performing removal of protecting groups R¹ of tetrasaccharide of formula **5-A** in presence of a base to yield tetrasaccharide of formula **7-A**, 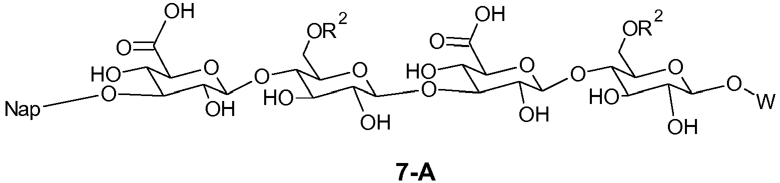 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10.

It is preferred that the inventive method for preparation of tetrasaccharide of formula **7-A** comprises the following step: Performing removal of protecting groups R¹ of tetrasaccharide of formula **5-A** in presence of a base to yield tetrasaccharide of formula **7-A**, wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of tetrasaccharide of formula **7-A** comprises the following step: Performing removal of protecting groups R¹ of tetrasaccharide of formula **5-A** in presence of a base to yield tetrasaccharide of formula **7-A**, wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of tetrasaccharide of formula **7-A** comprises the following step: Performing removal of protecting groups R¹ of tetrasaccharide of formula **5-A** in presence of a base to yield tetrasaccharide of formula **7-A**, wherein R¹ is selected from -Bz, -ClAc, -Ac and -Piv, R² represents -Bn, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of tetrasaccharide of formula **7-A** comprises the following step: Performing removal of protecting groups R¹ of tetrasaccharide of formula **5-A** in presence of a base to yield tetrasaccharide of formula **7-A**, wherein R¹ is selected from -Bz, -ClAc, -Ac and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of tetrasaccharide of formula **7-A** comprises the following step: Performing removal of protecting groups R¹ of tetrasaccharide of formula **5-A** in presence of a base to yield tetrasaccharide of formula **7-A**, wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

It is even more preferred that the inventive method for preparation of tetrasaccharide of formula **7-A** comprises the following step: Performing removal of protecting groups R¹ of tetrasaccharide of formula **5-A** in presence of a base to yield tetrasaccharide of formula **7-A**, wherein R¹ is selected from -Bz and -Ac, R² represents -Bn, W represents -L-P or -Bn, P is -N(Bn)Cbz or N₃, L represents -(CH₂)ₙ- and n is an integer selected from 2, 3, 4 and 5.

Preferably, the removal of protecting groups R¹ of tetrasaccharide of formula **5-A** to yield tetrasaccharide of formula **7-A** is performed in presence of a base in a protic solvent at elevated temperatures between 25 °C and 60 °C. More preferably, the removal of protecting groups R¹ of tetrasaccharide of formula **5-A** to yield tetrasaccharide of formula **7-A** is performed in presence of sodium methoxide in a protic solvent at elevated temperatures between 25 °C and 60 °C. More preferably, the removal of protecting groups R¹ of tetrasaccharide of formula **5-A** to yield tetrasaccharide of formula **7-A** is performed in presence of sodium methoxide in an alcohol at elevated temperatures between 30 °C and 50 °C. Even more preferably, the removal of protecting groups R¹ of tetrasaccharide of formula **5-A** to yield tetrasaccharide of formula **7-A** is performed in presence of sodium methoxide in methanol at 40 °C. According to the present invention the tetrasaccharide of formula **7-A** is preferably obtained in form of crystals by recrystallization or trituration.

Thus, it is especially preferred that the inventive method for preparation of tetrasaccharide of formula **7-A** comprises the following step: Performing removal of protecting groups R¹ of tetrasaccharide of formula **5-A** in presence of sodium methoxide in methanol to yield tetrasaccharide of formula **7-A**, wherein R¹ is -Bz, R² is -Bn, W represents -L-P or -Bn, P is -N(Bn)CBz, L represents -(CH₂)ₙ- and n is an integer selected from 2, 3, 4 and 5.

### Preparation of tetrasaccharide of formula 13-A

The preparation of tetrasaccharide of formula **13-A** according to the present invention comprises the following step: Performing removal of protecting groups R¹ of tetrasaccharide of formula **12-A** in presence of a base to yield tetrasaccharide of formula **13-A**, 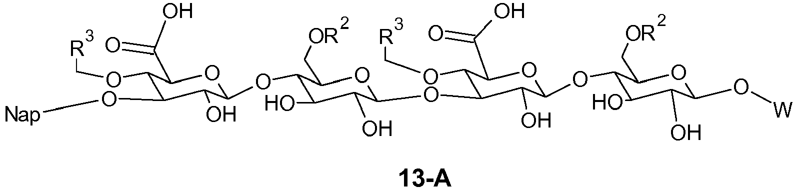 wherein
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

It is preferred that the inventive method for preparation of tetrasaccharide of formula **13-A** comprises the following step: Performing removal of protecting groups R¹ of tetrasaccharide of formula **12-A** in presence of a base to yield tetrasaccharide of formula **13-A**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of tetrasaccharide of formula **13-A** comprises the following step: Performing removal of protecting groups R¹ of tetrasaccharide of formula **12-A** in presence of a base to yield tetrasaccharide of formula **13-A**, wherein R¹ represents -Bz, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of tetrasaccharide of formula **13-A** comprises the following step: Performing removal of protecting groups R¹ of tetrasaccharide of formula **12-A** in presence of a base to yield tetrasaccharide of formula **13-A**, wherein R¹ is selected from -Bz, -ClAc, -Ac and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of tetrasaccharide of formula **13-A** comprises the following step: Performing removal of protecting groups R¹ of tetrasaccharide of formula **12-A** in presence of a base to yield tetrasaccharide of formula **13-A**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of tetrasaccharide of formula **13-A** comprises the following step: Performing removal of protecting groups R¹ of tetrasaccharide of formula **12-A** in presence of a base to yield tetrasaccharide of formula **13-A**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

It is even more preferred that the inventive method for preparation of tetrasaccharide of formula **13-A** comprises the following step: Performing removal of protecting groups R¹ of tetrasaccharide of formula **12-A** in presence of a base to yield tetrasaccharide of formula **13-A**, wherein R¹ is selected from -Bz and -Ac, R² represents -Bn, R³ is -Ph, W represents -L-P or -Bn, P is -N(Bn)Cbz or N₃, L represents -(CH₂)ₙ- and n is an integer selected from 2, 3, 4 and 5.

Preferably, the removal of protecting groups R¹ of tetrasaccharide of formula **12-A** to yield tetrasaccharide of formula **13-A** is performed in presence of a base in a protic solvent at elevated temperatures between 25 °C and 60 °C. More preferably, the removal of protecting groups R¹ of tetrasaccharide of formula **12-A** to yield tetrasaccharide of formula **13-A** is performed in presence of sodium methoxide in a protic solvent at elevated temperatures between 25 °C and 60 °C. More preferably, the removal of protecting groups R¹ of tetrasaccharide of formula **12-A** to yield tetrasaccharide of formula **13-A** is performed in presence of sodium methoxide in an alcohol at elevated temperatures between 30 °C and 50 °C. Even more preferably, the removal of protecting groups R¹ of tetrasaccharide of formula **12-A** to yield tetrasaccharide of formula **13-A** is performed in presence of sodium methoxide in methanol at 40 °C. According to the present invention the tetrasaccharide of formula **13-A** is preferably obtained in form of crystals by recrystallization or trituration.

Thus, it is especially preferred that the inventive method for preparation of tetrasaccharide of formula **13-A** comprises the following step: Performing removal of protecting groups R¹ of tetrasaccharide of formula **12-A** in presence of sodium methoxide in methanol to yield tetrasaccharide of formula **13-A,** wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of tetrasaccharide of formula 8-A

The preparation of tetrasaccharide of formula **8-A** according to the present invention comprises the following step:
Hydrogenating the tetrasaccharide of formula **7-A** in presence of a catalyst to yield a tetrasaccharide of formula **8-A** or a tetrasaccharide of formula **8'-A**, 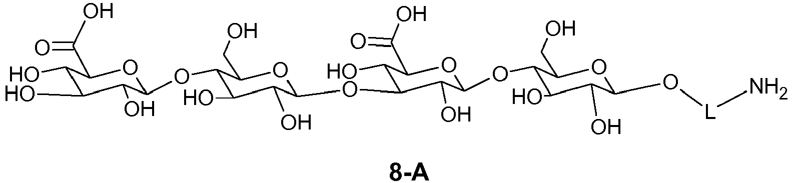 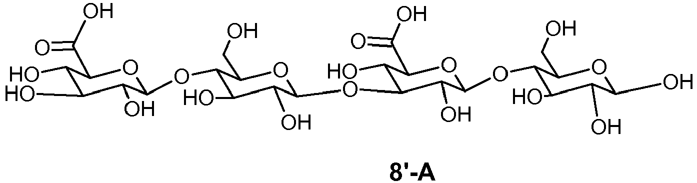
wherein L represents -(CH₂)ₙ- or -C₂H₄-O-C₂H₄- and
n is an integer selected from 2, 3, 4, 5, 6, 7, 8, 9 and 10.

Preferably, the inventive method for preparation of tetrasaccharide of formula **8-A** comprises the following step: Hydrogenating the tetrasaccharide of formula **7-A** in presence of a catalyst to yield a tetrasaccharide of formula **8-A**, wherein L represents -C₂H₄-O-C₂H₄- or -(CH₂)ₙ- and n is an integer selected from 2, 3, 4, 5, 6, 7 and 8. More preferably, the inventive method for preparation of tetrasaccharide of formula **8-A** comprises the following step: Performing a hydrogenation reaction of the tetrasaccharide of formula **7-A** in the presence of a catalyst to yield tetrasaccharide of formula **8-A**, wherein L represents -(CH₂)ₙ- and n is an integer selected from 2, 3, 4, and 5.

It is preferred that the hydrogenation reaction of the tetrasaccharide of formula **7-A** to yield tetrasaccharide of formula **8-A** is performed in the presence of a catalyst in a mixture of an alcohol and an aprotic apolar solvent. It is more preferred that the hydrogenation reaction of the tetrasaccharide of formula **7-A** to yield tetrasaccharide of formula **8-A** is performed in the presence of a palladium catalyst in a mixture of an alcohol and an aprotic apolar solvent It is even more preferred that the hydrogenation reaction of the tetrasaccharide of formula **7-A** to yield tetrasaccharide of formula **8-A** is performed in the presence of palladium on carbon (Pd/C) and/or Pd(OH)₂/C in a mixture of an alcohol and an aprotic apolar solvent. It is even more preferred that the hydrogenation reaction of the tetrasaccharide of formula **7-A** to yield tetrasaccharide of formula **8-A** is performed in the presence of palladium on carbon (Pd/C) and/or Pd(OH)₂/C in a mixture of tBuOH and methylene chloride.

Thus, it is especially preferred that the inventive method for preparation of tetrasaccharide of formula **8-A** comprises the following step: Hydrogenating the tetrasaccharide of formula **7-A** in the presence of Pd/C and/or Pd(OH)₂/C to yield a tetrasaccharide of formula **8-A**, wherein L represents -(CH₂)ₙ- and n is an integer selected from 2, 3, 4, and 5.

In an alternative embodiment of the present invention the preparation of tetrasaccharide of formula **8-A** comprises the following step:
Hydrogenating the tetrasaccharide of formula **13-A** in presence of a catalyst to yield a tetrasaccharide of formula **8-A** or a tetrasaccharide of formula **8'-A**, 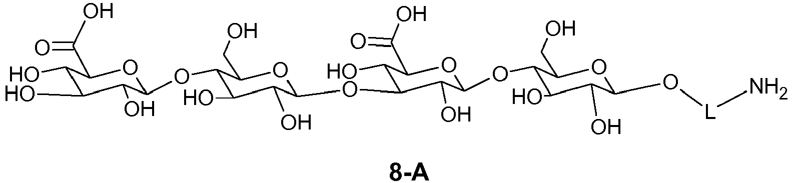 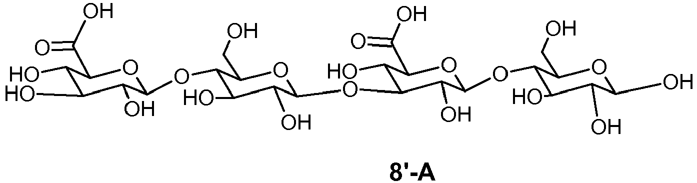
wherein L represents -(CH₂)ₙ- or -C₂H₄-O-C₂H₄- and
n is an integer selected from 2, 3, 4, 5, 6, 7, 8, 9 and 10.

Preferably, the inventive method for preparation of tetrasaccharide of formula **8-A** comprises the following step: Hydrogenating the tetrasaccharide of formula **13-A** in presence of a catalyst to yield a tetrasaccharide of formula **8-A**, wherein L represents -C₂H₄-O-C₂H₄- or -(CH₂)ₙ- and n is an integer selected from 2, 3, 4, 5, 6, 7 and 8. More preferably, the inventive method for preparation of tetrasaccharide of formula **8-A** comprises the following step: Performing a hydrogenation reaction of the tetrasaccharide of formula **13-A** in the presence of a catalyst to yield tetrasaccharide of formula **8-A**, wherein L represents -(CH₂)ₙ- and n is an integer selected from 2, 3, 4, and 5.

It is preferred that the hydrogenation reaction of the tetrasaccharide of formula **13-A** to yield tetrasaccharide of formula **8-A** is performed in the presence of a catalyst in a mixture of an alcohol and an aprotic apolar solvent. It is more preferred that the hydrogenation reaction of the tetrasaccharide of formula **13-A** to yield tetrasaccharide of formula **8-A** is performed in the presence of a palladium catalyst in a mixture of an alcohol and an aprotic apolar solvent It is even more preferred that the hydrogenation reaction of the tetrasaccharide of formula **13-A** to yield tetrasaccharide of formula **8-A** is performed in the presence of palladium on carbon (Pd/C) and/or Pd(OH)₂/C in a mixture of an alcohol and an aprotic apolar solvent. It is even more preferred that the hydrogenation reaction of the tetrasaccharide of formula **13-A** to yield tetrasaccharide of formula **8-A** is performed in the presence of palladium on carbon (Pd/C) and/or Pd(OH)₂/C in a mixture of tBuOH and methylene chloride.

Thus, it is especially preferred that the inventive method for preparation of tetrasaccharide of formula **8-A** comprises the following step: Hydrogenating the tetrasaccharide of formula **13-A** in the presence of Pd/C and/or Pd(OH)₂/C to yield a tetrasaccharide of formula **8-A**, wherein L represents -(CH₂)ₙ- and n is an integer selected from 2, 3, 4, and 5.

### Preparation of tetrasaccharide of formula 3-B

The preparation of a tetrasaccharide of formula **3-B** according to the present invention comprises the following step:
b6) Converting the tetrasaccharide of formula **1-A** to the tetrasaccharide of formula **3-B** by treatment with an oxidizing agent; 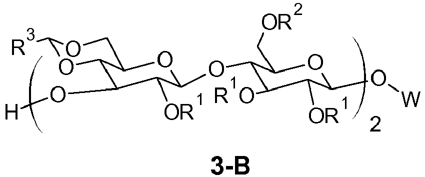 wherein
R¹ represents an orthogonal oxidation-stable and acid-stable protecting group,
R² represents an orthogonal oxidation-stable and acid-stable protecting group,
R³ is an aryl or alkyl group,
W represents -L-P or -Bn
P is -NR'R" or -N₃,
R' and R" represent independently of each other -Bn, -Cbz, -Allyl or R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably, the inventive method for preparation of a tetrasaccharide of formula **3-B** comprises the following step: b6) Converting the tetrasaccharide of formula **1-A** to the tetrasaccharide of formula **3-B** by treatment with an oxidizing agent, wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10.

More preferably, the inventive method for preparation of a tetrasaccharide of formula **3-A** comprises the following step b6) Converting the tetrasaccharide of formula **1-A** to the tetrasaccharide of formula **3-B** by treatment with an oxidizing agent, wherein R¹ represents -Bz, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10.

More preferably, the inventive method for preparation of a tetrasaccharide of formula **3-B** comprises the following step: b6) Converting the tetrasaccharide of formula **1-A** to the tetrasaccharide of formula **3-B** by treatment with an oxidizing agent, wherein R¹ is selected from -Bz, -ClAc, -Ac and -Piv, R² represents -Bn, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10.

More preferably, the inventive method for preparation of a tetrasaccharide of formula **3-B** comprises the following step: b6) Converting the tetrasaccharide of formula **1-A** to the tetrasaccharide of formula **3-B** by treatment with an oxidizing agent, wherein R¹ is selected from -Bz, -ClAc, -Ac and -Piv, R² is selected from -Bz, -Ac, -Piv, -Bn and -TBS, R³ represents -Ph, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10.

More preferably, the inventive method for preparation of a tetrasaccharide of formula **3-B** comprises the following step: b6) Converting the tetrasaccharide of formula **1-A** to the tetrasaccharide of formula **3-B** by treatment with an oxidizing agent, wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -N(Bn)Cbz, L represents -C₂H₄-O-C₂H₄-or -(CH₂)ₙ-, n is an integer selected from 2, 3, 4, 5, 6, 7, 8, 9 and 10.

More preferably, the inventive method for preparation of a tetrasaccharide of formula **3-B** comprises the following step: b6) Converting the tetrasaccharide of formula **1-A** to the tetrasaccharide of formula **3-B** by treatment with an oxidizing agent, wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -(CH₂)ₙ-, n is an integer selected from 2, 3, 4, and 5.

More preferably, the inventive method for preparation of a tetrasaccharide of formula **3-B** comprises the following step: b6) Converting the tetrasaccharide of formula **1-A** to the tetrasaccharide of formula **3-B** by treatment with an oxidizing agent, wherein R¹ is selected from -Bz and -Ac, R² represents -Bn, R³ represents -Ph, W represents -L-P or -Bn, P is -N(Bn)Cbz or -N₃, L represents -(CH₂)ₙ-, n is an integer selected from 2, 3, 4 and 5.

Preferably, the conversion of tetrasaccharide of formula **1-A** to tetrasaccharide of formula **3-B** is performed by treatment with an oxidizing agent in an aprotic apolar solvent at lowered temperatures between -10 °C and 20 °C. More preferably, the conversion of tetrasaccharide of formula **1-A** to tetrasaccharide of formula **3-B** is performed by treatment with DDQ in an aprotic apolar solvent at lowered temperatures between -10 °C and 20 °C. More preferably, the conversion of tetrasaccharide of formula **1-A** to tetrasaccharide of formula **3-B** is performed by treatment with DDQ in methylene chloride at lowered temperatures between -10 °C and 20 °C. Even more preferably, the conversion of tetrasaccharide of formula **1-A** to tetrasaccharide of formula **3-B** is performed by treatment with DDQ in methylene chloride at lowered temperatures between 0 °C and 10 °C.

Thus, it is especially preferred that the inventive method for preparation of a tetrasaccharide of formula **3-B** comprises the following step: b6) Converting the tetrasaccharide of formula **1-A** to the tetrasaccharide of formula **3-B** by treatment with DDQ, wherein R¹ represents -Bz, R² is -Bn, R³ is a phenyl group, W represents -L-P or -Bn, P is -N(Bn)Cbz, L represents -(CH₂)ₙ-, n is an integer selected from 2, 3, 4 and 5.

### Coupling of disaccharide of formula 2 and tetrasaccharide of formula 3-B to protected hexasaccharide of formula 1-B

The preparation of protected hexasaccharide of formula **1-B** according to the present invention comprises the following steps:
A) Providing a disaccharide of formula **2** 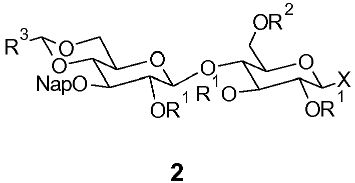 wherein
   R¹ represents an orthogonal oxidation-stable and acid-stable protecting group,
   R² represents an orthogonal oxidation-stable and acid-stable protecting group,
   R³ is an aryl or alkyl group, and
   X represents -OAc or -SR⁵, with R⁵ being selected from -Me, -Et, -Pr, -Bu, -Ph and -Tol;
   and a tetrasaccharide of formula **3-B** 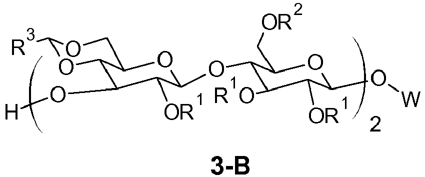 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
   R' and R" form together phthaloyl,
   L represents -(CH₂)ₙ- or -C₂H₄-O-C₂H₄-,
   n is an integer selected from 2, 3, 4, 5, 6, 7, 8, 9 and 10;
B) Coupling of the disaccharide of formula **2** with the tetrasaccharide of formula **3-B** in the presence of a catalyst to obtain a hexasaccharide of formula **1-B** 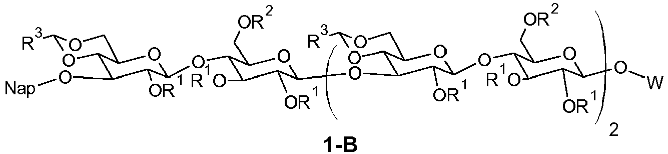 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
   R' and R" form together phthaloyl,
   L represents -(CH₂)ₙ- or -C₂H₄-O-C₂H₄-,,
   n is an integer selected from 2, 3, 4, 5, 6, 7, 8, 9 and 10.

It is preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a tetrasaccharide of formula **3-B** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the tetrasaccharide of formula **3-B** in the presence of a catalyst to obtain a hexasaccharide of formula **1-B.**

It is more preferred that the inventive method comprises the following steps:
A) Providing a disaccharide of formula **2** wherein R¹ represents -Bz, R² is selected from -Bz, -Ac, -Piv, -Bn and -TBS, R³ is selected from -Me, -Et, -Ph, -tBu and -PMP, X represents -OAc or -SR⁵, and R⁵ is selected from -Me, -Et, -Pr, -Bu, -Ph, and -Tol and a tetrasaccharide of formula **3-B** with W representing -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10; B) Coupling of the disaccharide of formula **2** with the tetrasaccharide of formula **3-B** in the presence of a catalyst to obtain a hexasaccharide of formula **1-B.**

It is more preferred that the inventive method comprises the following steps:
A) Providing a disaccharide of formula **2** wherein R¹ is selected from -Bz, -ClAc, -Ac and -Piv, R² represents -Bn, R³ is selected from -Me, -Et, -Ph, -tBu or -PMP, X represents -OAc or -SR⁵ and R⁵ is selected from -Me, -Et, -Pr, -Bu, -Ph, and -Tol and a tetrasaccharide of formula **3-B** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the tetrasaccharide of formula **3-B** in the presence of a catalyst to obtain a hexasaccharide of formula **1-B.**

It is more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ represents -Ph, X represents -OAc or -SR⁵ and R⁵ is selected from -Me, -Et, -Pr, -Bu, -Ph, and -Tol and a tetrasaccharide of formula **3-B** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the tetrasaccharide of formula **3-B** in the presence of a catalyst to obtain a hexasaccharide of formula **1-B.**

It is even more preferred that the inventive method comprises the following steps: A) Providing a disaccharide of formula **2** wherein R¹ is selected from -Bz, -ClAc, -Ac or -Piv, R² is selected from -Bz, -Ac, -Piv, -Bn and -TBS, R³ is selected from -Me, -Et, -Ph, -tBu and -PMP, X represents -OAc or -SEt and a tetrasaccharide of formula **3-B** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the tetrasaccharide of formula **3-B** with the disaccharide of formula **2** in the presence of a catalyst to obtain a hexasaccharide of formula **1-B.**

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a tetrasaccharide of formula **3-B** with W representing -L-P or -Bn, P is -N(Bn)Cbz, L represents -C₂H₄-O-C₂H₄- or -(CH₂)ₙ-, n is an integer selected from 2, 3, 4, 5, 6, 7, 8, 9 and 10;
B) Coupling of the disaccharide of formula **2** with the tetrasaccharide of formula **3-B** in the presence of a catalyst to obtain a hexasaccharide of formula **1-B.**

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a tetrasaccharide of formula **3-B** with W representing -L-P or -Bn, P is -NR'R", R' and R" represent independently of each other -Bn, -Cbz, -Allyl or R' and R" form together phthaloyl, L represents -(CH₂)ₙ-, n is an integer selected from 2, 3, 4, and 5; B) Coupling of the disaccharide of formula **2** with the tetrasaccharide of formula **3-B** in the presence of a catalyst to obtain a hexasaccharide of formula **1-B.**

It is even more preferred that the inventive method comprises the following steps:
A) Providing a disaccharide of formula **2** wherein R¹ is selected from -Bz and -Ac, R² represents -Bn, R³ represents -Ph, X represents -OAc or -SEt and a tetrasaccharide of formula **3-B** wherein W represents -L-P or -Bn, P is -N(Bn)Cbz or N₃, L represents -(CH₂)ₙ-, n is an integer selected from 2, 3, 4, and 5; B) Coupling of the disaccharide of formula **2** with tetrasaccharide of formula **3-B** in the presence of a catalyst to obtain a hexasaccharide of formula **1-B.**

Preferably, the coupling reaction of disaccharide of formula **2** with the tetrasaccharide of formula **3-B** is performed in the presence of NIS and catalytic amounts of a strong acid in an aprotic apolar solvent at low temperatures between -20 °C and 10 °C. More preferably, the coupling reaction of disaccharide of formula **2** with the tetrasaccharide of formula **3-B** is performed in the presence of NIS and catalytic amounts of trifluoromethanesulfonic acid in an aprotic apolar solvent at low temperatures between -20 °C and 10 °C. More preferably, the coupling reaction of disaccharide of formula **2** with the tetrasaccharide of formula **3-B** is performed in the presence of NIS and catalytic amounts of trifluoromethanesulfonic acid in methylene chloride at low temperatures between -20 °C and 5 °C.

Even more preferably, the coupling reaction of disaccharide of formula **2** with the tetrasaccharide of formula **3-B** is performed in the presence of NIS and catalytic amounts of trifluoromethanesulfonic acid in methylene chloride at low temperatures between -10 °C and 0 °C. According to the present invention the protected hexasaccharide of formula **1-B** is preferably obtained in form of crystals by recrystallization or trituration.

Thus, it is especially preferred that the inventive method for preparation of protected hexasaccharide of formula **1-B** comprises the following steps: A) Providing a disaccharide of formula **2** with R¹ is -Bz, R² is -Bn, R³ is a phenyl group, X represents -OAc or -SEt and a tetrasaccharide of formula **3-B** with W representing -L-P or -Bn, P is -N(Bn)Cbz, L represents -(CH₂)ₙ-, n is an integer selected from 2, 3, 4 and 5; B) Coupling of the disaccharide of formula **2** with the tetrasaccharide of formula **3-B** in the presence of NIS and catalytic amounts of trifluoromethanesulfonic acid to obtain a hexasaccharide of formula **1-B.**

### Preparation of hexasaccharide of formula 4-B

The preparation of hexasaccharide of formula **4-B** according to the present invention comprises the following step:
Performing selective removal of acetal groups by treatment of the hexasaccharide of formula **1-B** with a nucleophile in presence of a catalytic amount of an acid to yield a hexasaccharide of formula **4-B**, 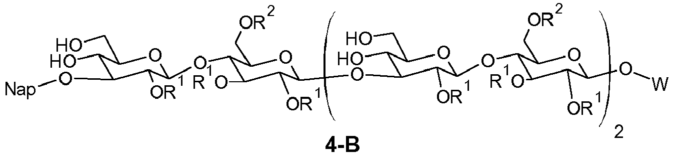 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of hexasaccharide of formula **4-B** comprises the following step: Performing selective removal of acetal groups by treatment of the hexasaccharide of formula **1-B** with a nucleophile in presence of a catalytic amount of an acid to yield a hexasaccharide of formula **4-B**, wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide of formula **4-B** comprises the following step: Performing selective removal of acetal groups by treatment of the hexasaccharide of formula **1-B** with a nucleophile in presence of a catalytic amount of an acid to yield a hexasaccharide of formula **4-B**, wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation hexasaccharide of formula **4-B** comprises the following step: Performing selective removal of acetal groups by treatment of the hexasaccharide of formula **1-B** with a nucleophile in presence of a catalytic amount of an acid to yield a hexasaccharide of formula **4-B**, wherein R¹ is selected from -Bz, -ClAc, -Ac and -Piv, R² represents -Bn, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide of formula **4-B** comprises the following step: Performing selective removal of acetal groups by treatment of the hexasaccharide of formula **1-B** with a nucleophile in presence of a catalytic amount of an acid to yield a hexasaccharide of formula **4-B**, wherein R¹ is selected from -Bz, -ClAc, -Ac and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of hexasaccharide of formula **4-B** comprises the following step: Performing selective removal of acetal groups by treatment of the hexasaccharide of formula **1-B** with a nucleophile in presence of a catalytic amount of an acid to yield a hexasaccharide of formula **4-B**, wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of hexasaccharide of formula **4-B** comprises the following step: Performing selective removal of acetal groups by treatment of the hexasaccharide of formula **1-B** with a nucleophile in presence of a catalytic amount of an acid to yield a hexasaccharide of formula **4-B**, wherein R¹ is selected from -Bz and -Ac, R² represents -Bn, P is -N(Bn)Cbz or N₃, L represents -(CH₂)ₙ- and n is an integer selected from 2, 3, 4 and 5.

It is preferred that the selective removal of acetal groups of hexasaccharide of formula **1-B** is performed in the presence of catalytic amounts of an acid and a nucleophile, such as a thiol, water or an alcohol. It is even more preferred that the selective removal of acetal groups of hexasaccharide of formula **1-B** is performed in the presence of catalytic amounts of a sulfonic acid and a thiol. It is even more preferred that the selective removal of acetal groups of hexasaccharide of formula **1-B** is performed in the presence of catalytic amounts of PTSA and a thiol. It is especially preferred that the selective removal of acetal groups of hexasaccharide of formula **1-B** is performed in the presence of catalytic amounts of PTSA and ethanethiol. According to the present invention the hexasaccharide of formula **4-B** is preferably obtained in form of crystals by recrystallization or trituration.

Thus, it is especially preferred that the inventive method for preparation of hexasaccharide of formula **4-B** comprises the following step: Performing selective removal of acetal groups by treatment of the hexasaccharide of formula **1-B** with ethanethiol in presence of a catalytic amount of PTSA to yield a hexasaccharide of formula **4-B**, wherein R¹ is -Bz, R² is -Bn, W represents -L-P or -Bn, P is -N(Bn)CBz, L represents -(CH₂)ₙ- and n is an integer selected from 2, 3, 4 and 5.

### Preparation of hexasaccharide of formula 11-B

In an alternative embodiment of the present invention the hexasaccharide of formula **11-B** is prepared by
performing reductive removal of the acetal groups by treatment of the hexasaccharide of formula **1-B** with a borane in presence of a catalytic amount of a Lewis acid to yield a hexasaccharide of formula **11-B**, 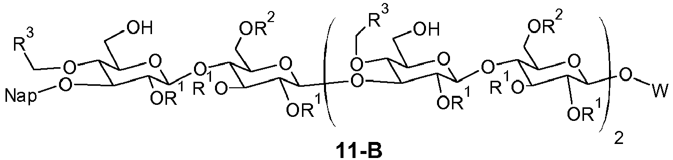 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of hexasaccharide of formula **11-B** comprises the following step: Performing reductive removal of the acetal groups by treatment of the hexasaccharide of formula **1-B** with a borane in presence of a catalytic amount of a Lewis acid to yield a saccharide of formula **11-B**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide of formula **11-B** comprises the following step: Performing reductive removal of the acetal groups by treatment of the hexasaccharide of formula **1-B** with a borane in presence of a catalytic amount of a Lewis acid to yield a saccharide of formula **11-B**, wherein R¹ represents -Bz, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide of formula **11-B** comprises the following step: Performing reductive removal of the acetal groups by treatment of the hexasaccharide of formula **1-B** with a borane in presence of a catalytic amount of a Lewis acid to yield a saccharide of formula **11-B**, wherein R¹ is selected from -Bz, -ClAc, -Ac and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide of formula **11-B** comprises the following step: Performing reductive removal of the acetal groups by treatment of the hexasaccharide of formula **1-B** with a borane in presence of a catalytic amount of a Lewis acid to yield a saccharide of formula **11-B**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of hexasaccharide of formula **11-B** comprises the following step: Performing reductive removal of the acetal groups by treatment of the hexasaccharide of formula **1-B** with a borane in presence of a catalytic amount of a Lewis acid to yield a saccharide of formula **11-B**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of hexasaccharide of formula **11-B** comprises the following step: Performing reductive removal of the acetal groups by treatment of the hexasaccharide of formula **1-B** with a borane in presence of a catalytic amount of a Lewis acid to yield a saccharide of formula **11-B**, wherein R¹ is selected from -Bz and -Ac, R² represents -Bn, P is -N(Bn)Cbz or N₃, R³ is -Ph, L represents -(CH₂)ₙ- and n is an integer selected from 2, 3, 4 and 5.

It is preferred that the reductive removal of acetal groups hexasaccharide of formula **1-B** is performed in the presence of catalytic amounts of a Lewis acid and a borane, such as BH₃, BH₃•NMe₂, BH₃•THF. It is even more preferred that the reductive removal of acetal groups of hexasaccharide of formula **1-B** is performed in the presence of catalytic amounts of silver trifluoromethanesulfonate and BH₃. According to the present invention the hexasaccharide of formula **11-B** is preferably obtained in form of crystals by recrystallization or trituration.

Thus, it is especially preferred that the inventive method for preparation of hexasaccharide of formula **11-B** comprises the following step: Performing reductive removal of the acetal groups by treatment of the hexasaccharide of formula **1-B** with BH₃ in presence of a catalytic amount of silver trifluoromethanesulfonate to yield a saccharide of formula **11-B**, wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of hexasaccharide diacid of formula 5-B

The preparation of hexasaccharide diacid of formula **5-B** according to the present invention comprises the following step:
Performing oxidation of the hexasaccharide of formula **4-B** to yield a hexasaccharide diacid of formula **5-B,** 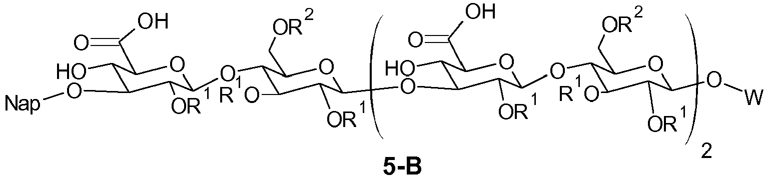 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of hexasaccharide diacid of formula **5-B** comprises the following step: Performing oxidation of the hexasaccharide of formula **4-B** to yield a hexasaccharide diacid of formula **5-B,** wherein R¹ is selected from -Bz, -ClAc, -Ac, and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide diacid of formula **5-B** comprises the following step: Performing oxidation of the hexasaccharide of formula **4-B** to yield a hexasaccharide diacid of formula **5-B,** wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

More preferably the inventive method for preparation of hexasaccharide diacid of formula **5-B** comprises the following step: Performing oxidation of the hexasaccharide of formula **4-B** to yield a hexasaccharide diacid of formula **5-B,** wherein R¹ is selected from -Bz, -ClAc, -Ac, and -Piv, R² represents -Bn, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

More preferably the inventive method for preparation of hexasaccharide diacid of formula **5-B** comprises the following step: Performing oxidation of the hexasaccharide of formula **4-B** to yield a hexasaccharide diacid of formula **5-B**, wherein R¹ is selected from -Bz, -ClAc, -Ac, and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of hexasaccharide diacid of formula **5-B** comprises the following step: Performing oxidation of the hexasaccharide of formula **4-B** to yield a hexasaccharide diacid of formula **5-B**, wherein R¹ is selected from -Bz, -ClAc, -Ac, and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of hexasaccharide diacid of formula **5-B** comprises the following step: Performing oxidation of the hexasaccharide of formula **4-B** to yield a hexasaccharide diacid of formula **5-B**, wherein R¹ is selected from -Bz and -Ac, R² represents -Bn, W represents -L-P or -Bn, P is -N(Bn)Cbz or N₃, L represents -(CH₂)ₙ- and n is an integer selected from 2, 3, 4 and 5.

It is preferred that the oxidation of hexasaccharide of formula **4-B** is performed in the presence of at least one oxidizing agent in a mixture of water and an aprotic apolar solvent at low temperatures between -10 °C and 10 °C. It is even more preferred that the oxidation of hexasaccharide of formula **4-B** is performed in the presence of a catalytic amount of a first oxidizing agent and a second oxidizing agent in a mixture of water and an aprotic apolar solvent at low temperatures between -10 °C and 10 °C. It is even more preferred that the oxidation of hexasaccharide of formula **4-B** is performed in the presence of a catalytic amount of TEMPO and a second oxidizing agent in a mixture of water and an aprotic apolar solvent at low temperatures between -10 °C and 10 °C. It is even more preferred that the oxidation of hexasaccharide of formula **4-B** is performed in the presence of a catalytic amount of TEMPO and bis(acetoxy)iodobenzene (BAIB) in a mixture of water and an aprotic apolar solvent at low temperatures between -10 °C and 10 °C. It is even more preferred that the oxidation of hexasaccharide of formula **4-B** is performed in the presence of a catalytic amount of TEMPO and bis(acetoxy)iodobenzene (BAIB) in a mixture of water and methylene chloride at low temperatures between -10 °C and 10 °C. It is especially preferred that the oxidation of hexasaccharide of formula **4-B** is performed in the presence of a catalytic amount of TEMPO and bis(acetoxy)iodobenzene (BAIB) in a mixture of water and methylene chloride at 0 °C. According to the present invention the hexasaccharide diacid of formula **5-B** is preferably obtained in form of crystals by recrystallization or trituration.

Thus, it is especially preferred that the inventive method for preparation of hexasaccharide diacid of formula **5-B** comprises the following step: Performing oxidation of the hexasaccharide of formula **4-B** with a catalytic amount of TEMPO and BAIB to yield a hexasaccharide diacid of formula **5-B**, wherein R¹ is -Bz, R² is -Bn, W represents -L-P or -Bn, P is -N(Bn)CBz, L represents -(CH₂)ₙ- and n is an integer selected from 2, 3, 4 and 5.

### Preparation of hexasaccharide diacid of formula 12-B

The preparation of hexasaccharide diacid of formula **12-B** according to the present invention comprises the following step:
Performing oxidation of the hexasaccharide of formula **11-B** to yield a hexasaccharide diacid of formula **12-B,** 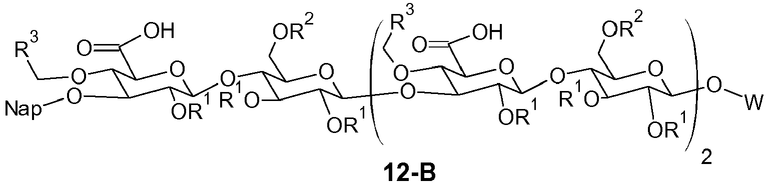 wherein
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of hexasaccharide diacid of formula **12-B** comprises the following step: Performing oxidation of the hexasaccharide of formula **11-B** to yield a hexasaccharide diacid of formula **12-B**, wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide diacid of formula **12-B** comprises the following step: Performing oxidation of the hexasaccharide of formula **11-B** to yield a hexasaccharide diacid of formula **12-B**, wherein R¹ represents -Bz, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide diacid of formula **12-B** comprises the following step: Performing oxidation of the hexasaccharide of formula **11-B** to yield a hexasaccharide diacid of formula **12-B**, wherein R¹ is selected from -Bz, -ClAc, -Ac, and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of hexasaccharide diacid of formula **12-B** comprises the following step: Performing oxidation of the hexasaccharide of formula **11-B** to yield a hexasaccharide diacid of formula **12-B**, wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for hexasaccharide diacid of formula **12-B** comprises the following step: Performing oxidation of the hexasaccharide of formula **11-B** to yield a hexasaccharide diacid of formula **12-B**, wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of hexasaccharide diacid of formula **12-B** comprises the following step: Performing oxidation of the hexasaccharide of formula **11-B** to yield a hexasaccharide diacid of formula **12-B**, wherein R¹ is selected from -Bz and -Ac, R² represents -Bn, R³ is -Ph, W represents -L-P or -Bn, P is -N(Bn)Cbz or N₃, L represents -(CH₂)ₙ- and n is an integer selected from 2, 3, 4 and 5.

It is preferred that the oxidation of hexasaccharide of formula **11-B** is performed in the presence of at least one oxidizing agent in a mixture of water and an aprotic apolar solvent at low temperatures between -10 °C and 10 °C. It is even more preferred that the oxidation of hexasaccharide of formula **11-B** is performed in the presence of a catalytic amount of a first oxidizing agent and a second oxidizing agent in a mixture of water and an aprotic apolar solvent at low temperatures between -10 °C and 10 °C. It is even more preferred that the oxidation of hexasaccharide of formula **11-B** is performed in the presence of a catalytic amount of TEMPO and a second oxidizing agent in a mixture of water and an aprotic apolar solvent at low temperatures between -10 °C and 10 °C. It is even more preferred that the oxidation of hexasaccharide of formula **11-B** is performed in the presence of a catalytic amount of TEMPO and bis(acetoxy)iodobenzene (BAIB) in a mixture of water and an aprotic apolar solvent at low temperatures between -10 °C and 10 °C. It is even more preferred that the oxidation of hexasaccharide of formula **11-B** is performed in the presence of a catalytic amount of TEMPO and bis(acetoxy)iodobenzene (BAIB) in a mixture of water and methylene chloride at low temperatures between -10 °C and 10 °C. It is especially preferred that the oxidation of hexasaccharide of formula **11-B** is performed in the presence of a catalytic amount of TEMPO and bis(acetoxy)iodobenzene (BAIB) in a mixture of water and methylene chloride at 0 °C. According to the present invention the hexasaccharide diacid of formula **12-B** is preferably obtained in form of crystals by recrystallization or trituration.

Thus, it is especially preferred that the inventive method for preparation of hexasaccharide diacid of formula **12-B** comprises the following step: Performing oxidation of the hexasaccharide of formula **11-B** with a catalytic amount of TEMPO and BAIB to yield a hexasaccharide diacid of formula **12-B**, wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of hexasaccharide of formula 7-B

The preparation of hexasaccharide of formula **7-B** according to the present invention comprises the following step: Performing removal of protecting groups R¹ of hexasaccharide of formula **5-B** in presence of a base to yield hexasaccharide of formula **7-B**, 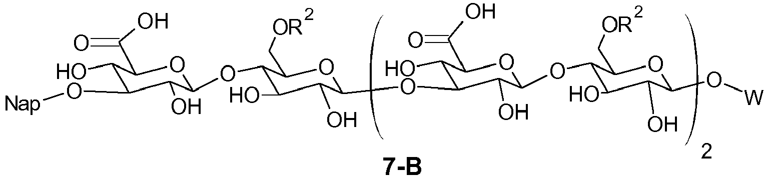 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10.

It is preferred that the inventive method for preparation of hexasaccharide of formula **7-B** comprises the following step: Performing removal of protecting groups R¹ of hexasaccharide of formula **5-B** in presence of a base to yield hexasaccharide of formula **7-B,** wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of hexasaccharide of formula **7-B** comprises the following step: Performing removal of protecting groups R¹ of hexasaccharide of formula **5-B** in presence of a base to yield hexasaccharide of formula **7-B**, wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation hexasaccharide of formula **7-B** comprises the following step: Performing removal of protecting groups R¹ of hexasaccharide of formula **5-B** in presence of a base to yield hexasaccharide of formula **7-B**, wherein R¹ is selected from -Bz, -ClAc, -Ac and -Piv, R² represents -Bn, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of hexasaccharide of formula **7-B** comprises the following step: Performing removal of protecting groups R¹ of hexasaccharide of formula **5-B** in presence of a base to yield hexasaccharide of formula **7-B**, wherein R¹ is selected from -Bz, -ClAc, -Ac and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of hexasaccharide of formula **7-B** comprises the following step: Performing removal of protecting groups R¹ of hexasaccharide of formula **5-B** in presence of a base to yield hexasaccharide of formula **7-B**, wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, - Allyl or R' and R" form together phthaloyl, L represents -(CH₂)ₙ- and n is an integer selected from 2, 3, 4 and 5.

It is even more preferred that the inventive method for preparation of hexasaccharide of formula **7-B** comprises the following step: Performing removal of protecting groups R¹ of hexasaccharide of formula **5-B** in presence of a base to yield hexasaccharide of formula **7-B**, wherein R¹ is selected from -Bz and -Ac, R² represents -Bn, W represents -L-P or -Bn, P is -N(Bn)Cbz or N₃, L represents -(CH₂)ₙ- and n is an integer selected from 2, 3, 4 and 5.

Preferably, the removal of protecting groups R¹ of hexasaccharide of formula **5-B** to yield hexasaccharide of formula **7-B** is performed in presence of a base in a protic solvent at elevated temperatures between 25 °C and 60 °C. More preferably, the removal of protecting groups R¹ of hexasaccharide of formula **5-B** to yield hexasaccharide of formula **7-B** is performed in presence of sodium methoxide in a protic solvent at elevated temperatures between 25 °C and 60 °C. More preferably, the removal of protecting groups R¹ of hexasaccharide of formula **5-B** to yield hexasaccharide of formula **7-B** is performed in presence of sodium methoxide in an alcohol at elevated temperatures between 30 °C and 50 °C. Even more preferably, the removal of protecting groups R¹ of hexasaccharide of formula **5-B** to yield hexasaccharide of formula **7-B** is performed in presence of sodium methoxide in methanol at 40 °C. According to the present invention the hexasaccharide of formula **7-B** is preferably obtained in form of crystals by recrystallization or trituration.

Thus, it is especially preferred that the inventive method for preparation of hexasaccharide of formula **7-B** comprises the following step: Performing removal of protecting groups R¹ of hexasaccharide of formula **5-B** in presence of sodium methoxide in methanol to yield hexasaccharide of formula **7-B,** wherein R¹ is -Bz, R² is -Bn, W represents -L-P or -Bn, P is -N(Bn)CBz, L represents -(CH₂)ₙ- and n is an integer selected from 2, 3, 4 and 5.

### Preparation of hexasaccharide of formula 13-B

The preparation of hexasaccharide of formula **13-B** according to the present invention comprises the following step: Performing removal of protecting groups R¹ of hexasaccharide of formula **12-B** in presence of a base to yield hexasaccharide of formula **13-B**, 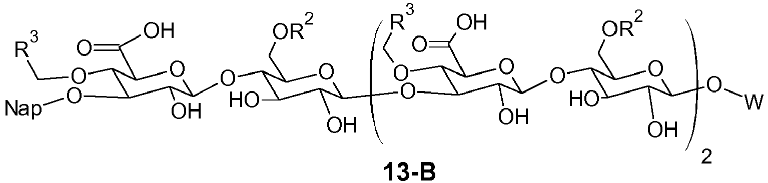 wherein
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

It is preferred that the inventive method for preparation of hexasaccharide of formula **13-B** comprises the following step: Performing removal of protecting groups R¹ of hexasaccharide of formula **12-B** in presence of a base to yield hexasaccharide of formula **13-B**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

It is more preferred that the inventive method for preparation of hexasaccharide of formula **13-B** comprises the following step: Performing removal of protecting groups R¹ of hexasaccharide of formula **12-B** in presence of a base to yield hexasaccharide of formula **13-B,** wherein R¹ represents -Bz, R² is selected from -Bz, -Ac, -Piv, -Bn and -TBS, R³ is an aryl group, W represents -L-P or -Bn, P is - NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of hexasaccharide of formula **13-B** comprises the following step: Performing removal of protecting groups R¹ of hexasaccharide of formula **12-B** in presence of a base to yield hexasaccharide of formula **13-B**, wherein R¹ is selected from -Bz, -ClAc, -Ac and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of hexasaccharide of formula **13-B** comprises the following step: Performing removal of protecting groups R¹ of hexasaccharide of formula **12-B** in presence of a base to yield hexasaccharide of formula **13-B**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of hexasaccharide of formula **13-B** comprises the following step: Performing removal of protecting groups R¹ of hexasaccharide of formula **12-B** in presence of a base to yield hexasaccharide of formula **13-B**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

It is even more preferred that the inventive method for preparation of hexasaccharide of formula **13-B** comprises the following step: Performing removal of protecting groups R¹ of hexasaccharide of formula **12-B** in presence of a base to yield hexasaccharide of formula **13-B**, wherein R¹ is selected from -Bz and -Ac, R² represents -Bn, R³ is -Ph, W represents -L-P or -Bn, P is-N(Bn)Cbz or N₃, L represents -(CH₂)ₙ- and n is an integer selected from 2, 3, 4 and 5.

Preferably, the removal of protecting groups R¹ of hexasaccharide of formula **12-B** to yield hexasaccharide of formula **13-B** is performed in presence of a base in a protic solvent at elevated temperatures between 25 °C and 60 °C. More preferably, the removal of protecting groups R¹ hexasaccharide of formula **12-B** to yield hexasaccharide of formula **13-B** is performed in presence of sodium methoxide in a protic solvent at elevated temperatures between 25 °C and 60 °C. More preferably, the removal of protecting groups R¹ of hexasaccharide of formula **12-B** to yield hexasaccharide of formula **13-B** is performed in presence of sodium methoxide in an alcohol at elevated temperatures between 30 °C and 50 °C. Even more preferably, the removal of protecting groups R¹ of hexasaccharide of formula **12-B** to yield hexasaccharide of formula **13-B** is performed in presence of sodium methoxide in methanol at 40 °C. According to the present invention the hexasaccharide of formula **13-B** is preferably obtained in form of crystals by recrystallization or trituration.

Thus, it is especially preferred that the inventive method for preparation of hexasaccharide of formula **13-B** comprises the following step: Performing removal of protecting groups R¹ of hexasaccharide of formula **12-B** in presence of sodium methoxide in methanol to yield hexasaccharide of formula **13-B**, wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of hexasaccharide of formula 8-B

The preparation of hexasaccharide of formula **8-B** or hexasaccharide of formula **8'-B** according to the present invention comprises the following step: Hydrogenating the hexasaccharide of formula **7-B** in presence of a catalyst to yield a hexasaccharide of formula **8-B** or a hexasaccharide of formula **8'-B**, 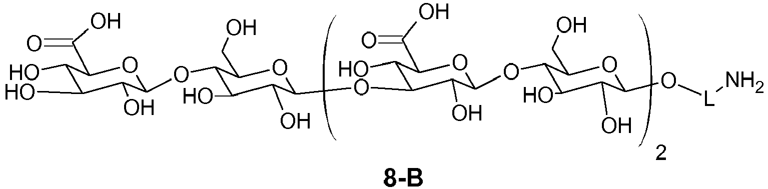 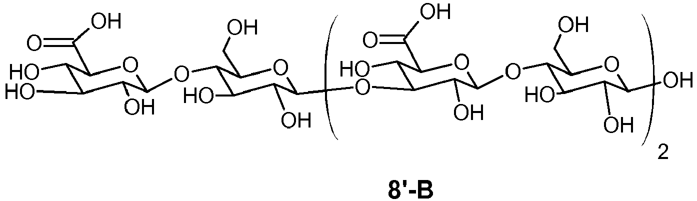
wherein L represents -(CH₂)ₙ- or -C₂H₄-O-C₂H₄- and
n is an integer selected from 2, 3, 4, 5, 6, 7, 8, 9 and 10.

Preferably, the inventive method for preparation of hexasaccharide of formula **8-B** or hexasaccharide of formula **8'-B** comprises the following step: Hydrogenating the hexasaccharide of formula **7-B** in presence of a catalyst to yield a hexasaccharide of formula **8-B** or a hexasaccharide of formula **8'-B**, wherein L represents -C₂H₄-O-C₂H₄- or -(CH₂)ₙ- and n is an integer selected from 2, 3, 4, 5, 6, 7 and 8. More preferably, the inventive method for preparation of hexasaccharide of formula **8-B** or hexasaccharide of formula **8'-B** comprises the following step: Performing a hydrogenation reaction of the hexasaccharide of formula **7-B** in presence of a catalyst to yield a hexasaccharide of formula **8-B** or a hexasaccharide of formula **8'-B**, wherein L represents -(CH₂)ₙ- and n is an integer selected from 2, 3, 4, and 5.

It is preferred that the hydrogenation reaction of the hexasaccharide of formula **7-B** to yield a hexasaccharide of formula **8-B** or a hexasaccharide of formula **8'-B** is performed in the presence of a catalyst in a mixture of an alcohol and an aprotic apolar solvent. It is more preferred that the hydrogenation reaction of the hexasaccharide of formula **7-B** to yield a hexasaccharide of formula **8-B** or a hexasaccharide of formula **8'-B** is performed in the presence of a palladium catalyst in a mixture of an alcohol and an aprotic apolar solvent It is even more preferred that the hydrogenation reaction of the hexasaccharide of formula **7-B** to yield a hexasaccharide of formula **8-B** or a hexasaccharide of formula **8'-B** is performed in the presence of palladium on carbon (Pd/C) and/or Pd(OH)₂/C in a mixture of an alcohol and an aprotic apolar solvent. It is even more preferred that the hydrogenation reaction of the hexasaccharide of formula **7-B** to yield a hexasaccharide of formula **8-B** or a hexasaccharide of formula **8'-B** is performed in the presence of palladium on carbon (Pd/C) and/or Pd(OH)₂/C in a mixture of tBuOH and methylene chloride.

Thus, it is especially preferred that the inventive method for preparation of hexasaccharide of formula **8-B** or a hexasaccharide of formula **8'-B** comprises the following step: Hydrogenating the hexasaccharide of formula **7-B** in the presence of Pd/C and/or Pd(OH)₂/C to yield a hexasaccharide of formula **8-B**, wherein L represents -(CH₂)ₙ- and n is an integer selected from 2, 3, 4, and 5 or a hexasaccharide of formula **8'-B**.

In an alternative embodiment of the present invention the preparation of hexasaccharide of formula **8-B** or a hexasaccharide of formula **8'-B** comprises the following step:
Hydrogenating the hexasaccharide of formula **13-B** in presence of a catalyst to yield a hexasaccharide of formula **8-B** or a hexasaccharide of formula **8'-B**, 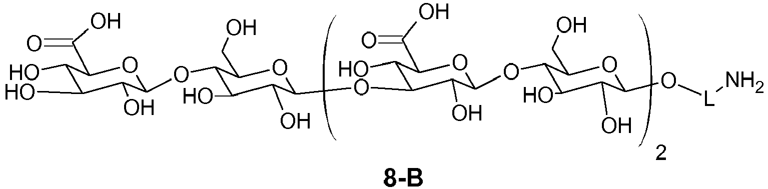 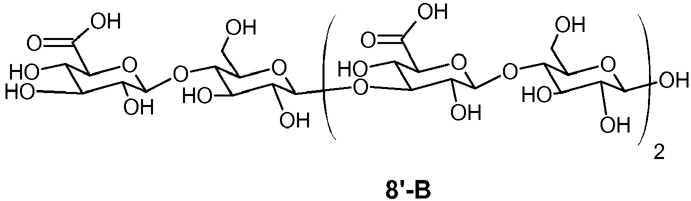
wherein L represents -(CH₂)ₙ- or -C₂H₄-O-C₂H₄- and
n is an integer selected from 2, 3, 4, 5, 6, 7, 8, 9 and 10.

Preferably, the inventive method for preparation of hexasaccharide of formula **8-B** or hexasaccharide of formula **8'-B** comprises the following step: Hydrogenating the hexasaccharide of formula **13-B** in presence of a catalyst to yield a hexasaccharide of formula **8-B** or a hexasaccharide of formula **8'-B,** wherein L represents -C₂H₄-O-C₂H₄- or -(CH₂)ₙ- and n is an integer selected from 2, 3, 4, 5, 6, 7 and 8. More preferably, the inventive method for preparation of hexasaccharide of formula **8-B** or hexasaccharide of formula **8'-B** comprises the following step: Performing a hydrogenation reaction of the hexasaccharide of formula **13-B** in presence of a catalyst to yield a hexasaccharide of formula **8-B** or a hexasaccharide of formula **8'-B**, wherein L represents -(CH₂)ₙ- and n is an integer selected from 2, 3, 4, and 5.

It is preferred that the hydrogenation reaction of the hexasaccharide of formula **13-B** to yield a hexasaccharide of formula **8-B** or a hexasaccharide of formula **8'-B** is performed in the presence of a catalyst in a mixture of an alcohol and an aprotic apolar solvent. It is more preferred that the hydrogenation reaction of the hexasaccharide of formula **13-B** to yield a hexasaccharide of formula **8-B** or a hexasaccharide of formula **8'-B** is performed in the presence of a palladium catalyst in a mixture of an alcohol and an aprotic apolar solvent It is even more preferred that the hydrogenation reaction of the hexasaccharide of formula **13-B** to yield a hexasaccharide of formula **8-B** or a hexasaccharide of formula **8'-B** is performed in the presence of palladium on carbon (Pd/C) and/or Pd(OH)₂/C in a mixture of an alcohol and an aprotic apolar solvent. It is even more preferred that the hydrogenation reaction of the hexasaccharide of formula **13-B** to yield a hexasaccharide of formula **8-B** or a hexasaccharide of formula **8'-B** is performed in the presence of palladium on carbon (Pd/C) and/or Pd(OH)₂/C in a mixture of tBuOH and methylene chloride.

Thus, it is especially preferred that the inventive method for preparation of hexasaccharide of formula **8-B** or a hexasaccharide of formula **8'-B** comprises the following step: Hydrogenating the hexasaccharide of formula **13-B** in the presence of Pd/C and/or Pd(OH)₂/C to yield a hexasaccharide of formula **8-B**, wherein L represents -(CH₂)ₙ- and n is an integer selected from 2, 3, 4, and 5 or a hexasaccharide of formula **8'-B**.

### Preparation of hexasaccharide of formula 3-C

The preparation of a hexasaccharide of formula **3-C** according to the present invention comprises the following step:
b6) Converting the hexasaccharide of formula **1-B** to the hexasaccharide of formula **3-C** by treatment with an oxidizing agent; 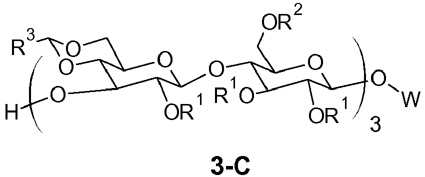 wherein
R¹ represents an orthogonal oxidation-stable and acid-stable protecting group,
R² represents an orthogonal oxidation-stable and acid-stable protecting group,
R³ is an aryl or alkyl group,
W represents -L-P or -Bn
P is -NR'R" or -N₃,
R' and R" represent independently of each other -Bn, -Cbz, -Allyl
   or R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably, the inventive method for preparation of a hexasaccharide of formula **3-C** comprises the following step: b6) Converting the hexasaccharide of formula **1-B** to the hexasaccharide of formula **3-C** by treatment with an oxidizing agent, wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10.

More preferably, the inventive method for preparation of a hexasaccharide of formula **3-C** comprises the following step: b6) Converting the hexasaccharide of formula **1-B** to the hexasaccharide of formula **3-C** by treatment with an oxidizing agent, wherein R¹ represents -Bz, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, - Allyl or R' and R" form together phthaloyl, L represents -C₂H₄-O-C₂H₄-or-(CH₂)ₙ-, n is an integer selected from 2, 3, 4, 5, 6, 7, 8, 9 and 10.

More preferably, the inventive method for preparation of a hexasaccharide of formula **3-C** comprises the following step: b6) Converting the hexasaccharide of formula **1-B** to the hexasaccharide of formula **3-C** by treatment with an oxidizing agent, wherein R¹ is selected from -Bz, -ClAc, -Ac and -Piv, R² represents -Bn, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10.

More preferably, the inventive method for preparation of a hexasaccharide of formula **3-C** comprises the following step: b6) Converting the hexasaccharide of formula **1-B** to the hexasaccharide of formula **3-C** by treatment with an oxidizing agent, wherein R¹ is selected from -Bz, -ClAc, -Ac and -Piv, R² is selected from -Bz, -Ac, -Piv, -Bn and -TBS, R³ represents -Ph, W represents -L-P or -Bn, P is -NR'R", R' and R" represent independently of each other -Bn, -Cbz, -Allyl or R' and R" form together phthaloyl, L represents -C₂H₄-O-C₂H₄-or -(CH₂)ₙ-, n is an integer selected from 2, 3, 4, 5, 6, 7, 8, 9 and 10.

More preferably, the inventive method for preparation of a hexasaccharide of formula **3-C** comprises the following step: b6) Converting the hexasaccharide of formula **1-B** to the hexasaccharide of formula **3-C** by treatment with an oxidizing agent, wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -N(Bn)Cbz, L represents -C₂H₄-O-C₂H₄-or -(CH₂)ₙ-, n is an integer selected from 2, 3, 4, 5, 6, 7, 8, 9 and 10.

More preferably, the inventive method for preparation of a hexasaccharide of formula **3-C** comprises the following step: b6) Converting the hexasaccharide of formula **1-B** to the hexasaccharide of formula **3-C** by treatment with an oxidizing agent, wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, W represents -L-P or -Bn, P is -NR'R", R' and R" represent independently of each other -Bn, -Cbz, -Allyl or R' and R" form together phthaloyl, L represents -(CH₂)ₙ-, n is an integer selected from 2, 3, 4, and 5.

More preferably, the inventive method for preparation of a hexasaccharide of formula **3-C** comprises the following step: b6) Converting the hexasaccharide of formula **1-B** to the hexasaccharide of formula **3-C** by treatment with an oxidizing agent, wherein R¹ is selected from -Bz and -Ac, R² represents -Bn, R³ represents -Ph, W represents -L-P or -Bn, P is -N(Bn)Cbz or -N₃, L represents -(CH₂)ₙ-, n is an integer selected from 2, 3, 4 and 5.

Preferably, the conversion of hexasaccharide of formula **1-B** to hexasaccharide of formula **3-C** is performed by treatment with an oxidizing agent in an aprotic apolar solvent at lowered temperatures between -10 °C and 20 °C. More preferably, the conversion of hexasaccharide of formula **1-B** to hexasaccharide of formula **3-C** is performed by treatment with DDQ in an aprotic apolar solvent at lowered temperatures between -10 °C and 20 °C. More preferably, the conversion of hexasaccharide of formula **1-B** to hexasaccharide of formula **3-C** is performed by treatment with DDQ in methylene chloride at lowered temperatures between -10 °C and 20 °C. Even more preferably, the conversion hexasaccharide of formula **1-B** to hexasaccharide of formula **3-C** is performed by treatment with DDQ in methylene chloride at lowered temperatures between 0 °C and 10 °C.

Thus, it is especially preferred that the inventive method for preparation of a hexasaccharide of formula **3-C** comprises the following step: b6) Converting the hexasaccharide of formula **1-B** to the hexasaccharide of formula **3-C** by treatment with DDQ, wherein R¹ represents -Bz, R² is -Bn, R³ is a phenyl group, W represents -L-P or -Bn, P is -N(Bn)Cbz, L represents -(CH₂)ₙ-, n is an integer selected from 2, 3, 4 and 5.

### Coupling of disaccharide of formula 2 and hexasaccharide of formula 3-C to protected octasaccharide of formula 1-C

The preparation of protected octasaccharide of formula **1-C** according to the present invention comprises the following steps:
A) Providing a disaccharide of formula **2** 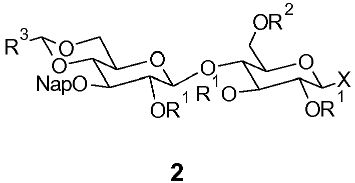 wherein
   R¹ represents an orthogonal oxidation-stable and acid-stable protecting group,
   R² represents an orthogonal oxidation-stable and acid-stable protecting group,
   R³ is an aryl or alkyl group, and
   X represents -OAc or -SR⁵, with R⁵ being selected from -Me, -Et, -Pr, -Bu, -Ph and -Tol;
   and a hexasaccharide of formula **3-C** 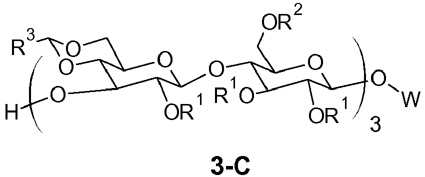 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
   R' and R" form together phthaloyl,
   L represents -(CH₂)ₙ- or -C₂H₄-O-C₂H₄-,
   n is an integer selected from 2, 3, 4, 5, 6, 7, 8, 9 and 10;
B) Coupling of the disaccharide of formula **2** with the hexasaccharide of formula **3-C** in the presence of a catalyst to obtain a octasaccharide of formula **1-C** 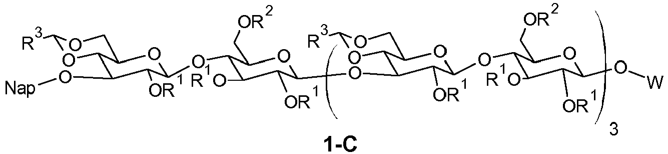 wherein
   R¹ represents an orthogonal oxidation-stable and acid-stable protecting group,
   R² represents an orthogonal oxidation-stable and acid-stable protecting group,
   R³ is an aryl or alkyl group,
   W represents -L-P or -Bn,
   P is -NR'R" or -N₃,
   R' and R" represent independently of each other -Bn, -Cbz, -Allyl or
      R' and R" form together phthaloyl,
   L represents -(CH₂)ₙ- or -C₂H₄-O-C₂H₄-,,
   n is an integer selected from 2, 3, 4, 5, 6, 7, 8, 9 and 10.

It is preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a hexasaccharide of formula **3-C** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the hexasaccharide of formula **3-C** in the presence of a catalyst to obtain an octasaccharide of formula **1-C**.

It is more preferred that the inventive method comprises the following steps:
A) Providing a disaccharide of formula **2** wherein R¹ represents -Bz, R² is selected from -Bz, -Ac, -Piv, -Bn and -TBS, R³ is selected from -Me, -Et, -Ph, -tBu and -PMP, X represents -OAc or -SR⁵, and R⁵ is selected from -Me, -Et, -Pr, -Bu, -Ph, and -Tol and a hexasaccharide of formula **3-C** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the hexasaccharide of formula **3-C** in the presence of a catalyst to obtain an octasaccharide of formula **1-C**.

It is more preferred that the inventive method comprises the following steps:
A) Providing a disaccharide of formula 2 wherein R¹ is selected from -Bz, -ClAc, -Ac and -Piv, R² represents -Bn, R³ is selected from -Me, -Et, -Ph, -tBu or -PMP, X represents -OAc or -SR⁵ and R⁵ is selected from -Me, -Et, -Pr, -Bu, -Ph, and -Tol and a hexasaccharide of formula **3-C** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the hexasaccharide of formula **3-C** in the presence of a catalyst to obtain an octasaccharide of formula **1-C**.

It is more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ represents -Ph, X represents -OAc or -SR⁵ and R⁵ is selected from -Me, -Et, -Pr, -Bu, -Ph, and -Tol and a hexasaccharide of formula **3-C** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the disaccharide of formula **2** with the hexasaccharide of formula **3-C** in the presence of a catalyst to obtain an octasaccharide of formula **1-C**.

It is even more preferred that the inventive method comprises the following steps:
A) Providing a disaccharide of formula **2** wherein R¹ is selected from -Bz, -ClAc, -Ac or -Piv, R² is selected from -Bz, -Ac, -Piv, -Bn and -TBS, R³ is selected from -Me, -Et, -Ph, -tBu and -PMP, X represents -OAc or -SEt and a tetrasaccharide of formula **3-B** with W representing -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ-, n is an integer selected from 2, 3, 4, 5, 6, 7, 8, 9 and 10; B) Coupling of the hexasaccharide of formula **3-C** with the disaccharide of formula **2** in the presence of a catalyst to obtain an octasaccharide of formula **1-C**.

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a hexasaccharide of formula **3-C** with W representing -L-P or -Bn, P is -N(Bn)Cbz, L represents -C₂H₄-O-C₂H₄- or -(CH₂)ₙ-, n is an integer selected from 2, 3, 4, 5, 6, 7, 8, 9 and 10;
B) Coupling of the disaccharide of formula **2** with the hexasaccharide of formula **3-C** in the presence of a catalyst to obtain an octasaccharide of formula **1-C**.

It is even more preferred that the inventive method comprises the following steps:
A) Providing a disaccharide of formula **2** wherein R¹ is selected from -Bz, -ClAc, -Ac and -Piv, R² is selected from -Bz, -Ac, -Piv, -Bn and -TBS, R³ is selected from -Me, -Et, -Ph, -tBu and -PMP, X represents -OAc or -SR⁵ and R⁵ is selected from -Me, -Et, -Pr, -Bu, -Ph, and -Tol and a hexasaccharide of formula **3-C** with W representing -L-P or -Bn, P is -NR'R", R' and R" represent independently of each other -Bn, -Cbz, -Allyl or R' and R" form together phthaloyl, L represents -(CH₂)ₙ-, n is an integer selected from 2, 3, 4, and 5; B) Coupling of the disaccharide of formula **2** with the hexasaccharide of formula **3-C** in the presence of a catalyst to obtain an octasaccharide of formula **1-C**.

It is even more preferred that the inventive method comprises the following steps:
A) Providing a disaccharide of formula **2** wherein R¹ is selected from -Bz and -Ac, R² represents -Bn, R³ represents -Ph, X represents -OAc or -SEt and a hexasaccharide of formula **3-C** wherein W represents -L-P or -Bn, P is -N(Bn)Cbz or N₃, L represents -(CH₂)ₙ-, n is an integer selected from 2, 3, 4, and 5; B) Coupling of the disaccharide of formula **2** with hexasaccharide of formula **3-C** in the presence of a catalyst to obtain an octasaccharide of formula **1-C**.

Preferably, the coupling reaction of disaccharide of formula **2** with the hexasaccharide of formula **3-C** is performed in the presence of NIS and catalytic amounts of a strong acid in an aprotic apolar solvent at low temperatures between -20 °C and 10 °C. More preferably, the coupling reaction of disaccharide of formula **2** with the hexasaccharide of formula **3-C** is performed in the presence of NIS and catalytic amounts of trifluoromethanesulfonic acid in an aprotic apolar solvent at low temperatures between -20 °C and 10 °C. More preferably, the coupling reaction of disaccharide of formula **2** with the hexasaccharide of formula **3-C** is performed in the presence of NIS and catalytic amounts of trifluoromethanesulfonic acid in methylene chloride at low temperatures between -20 °C and 5 °C. Even more preferably, the coupling reaction of disaccharide of formula **2** with the hexasaccharide of formula **3-C** is performed in the presence of NIS and catalytic amounts of trifluoromethanesulfonic acid in methylene chloride at low temperatures between -10 °C and 0 °C. According to the present invention the protected octasaccharide of formula **1-C** is preferably obtained in form of crystals by recrystallization or trituration.

Thus, it is especially preferred that the inventive method for preparation of protected octasaccharide of formula **1-C** comprises the following steps: A) Providing a disaccharide of formula **2** with R¹ is -Bz, R² is -Bn, R³ is a phenyl group, X represents -OAc or -SEt and a hexasaccharide of formula **3-C** with W representing -L-P or -Bn, P is -N(Bn)Cbz, L represents -(CH₂)ₙ-, n is an integer selected from 2, 3, 4 and 5; B) Coupling of the disaccharide of formula **2** with the hexasaccharide of formula **3-C** in the presence of NIS and catalytic amounts of trifluoromethanesulfonic acid to obtain an octasaccharide of formula **1-C**.

### Preparation of octasaccharide of formula 4-C

The preparation of octasaccharide of formula **4-C** according to the present invention comprises the following step:
Performing selective removal of acetal groups by treatment of the octasaccharide of formula **1-C** with a nucleophile in presence of a catalytic amount of an acid to yield a octasaccharide of formula **4-C**, 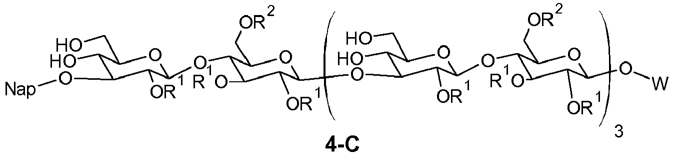 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of octasaccharide of formula **4-C** comprises the following step: Performing selective removal of acetal groups by treatment of the octasaccharide of formula **1-C** with a nucleophile in presence of a catalytic amount of an acid to yield an octasaccharide of formula **4-C**, wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide of formula **4-C** comprises the following step: Performing selective removal of acetal groups by treatment of the octasaccharide of formula **1-C** with a nucleophile in presence of a catalytic amount of an acid to yield an octasaccharide of formula **4-C**, wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation octasaccharide of formula **4-C** comprises the following step: Performing selective removal of acetal groups by treatment of the octasaccharide of formula **1-C** with a nucleophile in presence of a catalytic amount of an acid to yield an octasaccharide of formula **4-C**, wherein R¹ is selected from -Bz, -ClAc, -Ac and -Piv, R² represents -Bn, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide of formula **4-C** comprises the following step: Performing selective removal of acetal groups by treatment of the octasaccharide of formula **1-C** with a nucleophile in presence of a catalytic amount of an acid to yield an octasaccharide of formula **4-C**, wherein R¹ is selected from -Bz, -ClAc, -Ac and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of octasaccharide of formula **4-C** comprises the following step: Performing selective removal of acetal groups by treatment of the octasaccharide of formula **1-C** with a nucleophile in presence of a catalytic amount of an acid to yield an octasaccharide of formula **4-C**, wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of octasaccharide of formula **4-C** comprises the following step: Performing selective removal of acetal groups by treatment of the octasaccharide of formula **1-C** with a nucleophile in presence of a catalytic amount of an acid to yield an octasaccharide of formula **4-C**, wherein R¹ is selected from -Bz and -Ac, R² represents -Bn, P is -N(Bn)Cbz or N₃, L represents -(CH₂)ₙ- and n is an integer selected from 2, 3, 4 and 5.

It is preferred that the selective removal of acetal groups of octasaccharide of formula **1-C** is performed in the presence of catalytic amounts of an acid and a nucleophile, such as a thiol, water or an alcohol. It is even more preferred that the selective removal of acetal groups of octasaccharide of formula **1-C** is performed in the presence of catalytic amounts of a sulfonic acid and a thiol. It is even more preferred that the selective removal of acetal groups of octasaccharide of formula **1-C** is performed in the presence of catalytic amounts of PTSA and a thiol. It is especially preferred that the selective removal of acetal groups of octasaccharide of formula **1-C** is performed in the presence of catalytic amounts of PTSA and ethanethiol. According to the present invention the octasaccharide of formula **4-C** is preferably obtained in form of crystals by recrystallization or trituration.

Thus, it is especially preferred that the inventive method for preparation of octasaccharide of formula **4-C** comprises the following step: Performing selective removal of acetal groups by treatment of the octasaccharide of formula **1-C** with ethanethiol in presence of a catalytic amount of PTSA to yield a octasaccharide of formula **4-C**, wherein R¹ is -Bz, R² is -Bn, W represents -L-P or -Bn, P is - N(Bn)CBz, L represents -(CH₂)ₙ- and n is an integer selected from 2, 3, 4 and 5.

### Preparation of octasaccharide of formula 11-C

In an alternative embodiment of the present invention the octasaccharide of formula **11-C** is prepared by
performing reductive removal of the acetal groups by treatment of the octasaccharide of formula **1-C** with a borane in presence of a catalytic amount of a Lewis acid to yield an octasaccharide of formula **11-C**, 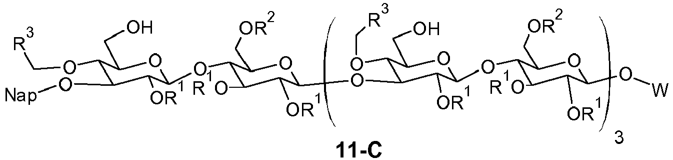 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of octasaccharide of formula **11-C** comprises the following step: Performing reductive removal of the acetal groups by treatment of the octasaccharide of formula **1-C** with a borane in presence of a catalytic amount of a Lewis acid to yield a saccharide of formula **11-C**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide of formula **11-C** comprises the following step: Performing reductive removal of the acetal groups by treatment of the octasaccharide of formula **1-C** with a borane in presence of a catalytic amount of a Lewis acid to yield a saccharide of formula **11-C**, wherein R¹ represents -Bz, R² is selected from -Bz, -Ac, -Piv, -Bn and-TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide of formula **11-C** comprises the following step: Performing reductive removal of the acetal groups by treatment of the octasaccharide of formula **1-C** with a borane in presence of a catalytic amount of a Lewis acid to yield a saccharide of formula **11-C**, wherein R¹ is selected from -Bz, -ClAc, -Ac and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide of formula **11-C** comprises the following step: Performing reductive removal of the acetal groups by treatment of the octasaccharide of formula **1-C** with a borane in presence of a catalytic amount of a Lewis acid to yield a saccharide of formula **11-C**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of octasaccharide of formula **11-C** comprises the following step: Performing reductive removal of the acetal groups by treatment of the octasaccharide of formula **1-C** with a borane in presence of a catalytic amount of a Lewis acid to yield a saccharide of formula **11-C**, wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of octasaccharide of formula **11-C** comprises the following step: Performing reductive removal of the acetal groups by treatment of the octasaccharide of formula **1-C** with a borane in presence of a catalytic amount of a Lewis acid to yield a saccharide of formula **11-C**, wherein R¹ is selected from -Bz and -Ac, R² represents -Bn, P is -N(Bn)Cbz or N₃, R³ is -Ph, L represents -(CH₂)ₙ- and n is an integer selected from 2, 3, 4 and 5.

It is preferred that the reductive removal of acetal groups octasaccharide of formula **1-C** is performed in the presence of catalytic amounts of a Lewis acid and a borane, such as BH₃, BH₃•NMe₂, BH₃•THF. It is even more preferred that the reductive removal of acetal groups of octasaccharide of formula **1-C** is performed in the presence of catalytic amounts of silver trifluoromethanesulfonate and BH₃. According to the present invention the octasaccharide of formula **11-C** is preferably obtained in form of crystals by recrystallization or trituration.

Thus, it is especially preferred that the inventive method for preparation of octasaccharide of formula **11-C** comprises the following step: Performing reductive removal of the acetal groups by treatment of the octasaccharide of formula **1-C** with BH₃ in presence of a catalytic amount of silver trifluoromethanesulfonate to yield an octasaccharide of formula **11-C,** wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of octasaccharide diacid of formula 5-C

The preparation of octasaccharide diacid of formula **5-C** according to the present invention comprises the following step:
Performing oxidation of the octasaccharide of formula **4-C** to yield an octasaccharide diacid of formula **5-C,** 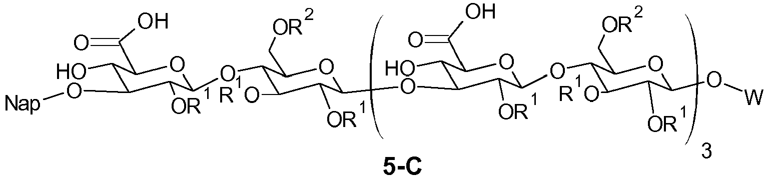 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of octasaccharide diacid of formula **5-C** comprises the following step: Performing oxidation of the octasaccharide of formula **4-C** to yield an octasaccharide diacid of formula **5-C,** wherein R¹ is selected from -Bz, -ClAc, -Ac, and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide diacid of formula **5-C** comprises the following step: Performing oxidation of the octasaccharide of formula **4-C** to yield an octasaccharide diacid of formula **5-C,** wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

More preferably the inventive method for preparation of octasaccharide diacid of formula **5-C** comprises the following step: Performing oxidation of the octasaccharide of formula **4-C** to yield an octasaccharide diacid of formula **5-C,** wherein R¹ is selected from -Bz, -ClAc, -Ac, and -Piv, R² represents -Bn, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

More preferably the inventive method for preparation of octasaccharide diacid of formula **5-C** comprises the following step: Performing oxidation of the octasaccharide of formula **4-C** to yield an octasaccharide diacid of formula **5-C,** wherein R¹ is selected from -Bz, -ClAc, -Ac, and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for preparation of octasaccharide diacid of formula **5-C** comprises the following step: Performing oxidation of the octasaccharide of formula **4-C** to yield an octasaccharide diacid of formula **5-C,** wherein R¹ is selected from -Bz, -ClAc, -Ac, and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of octasaccharide diacid of formula **5-C** comprises the following step: Performing oxidation of the octasaccharide of formula **4-C** to yield an octasaccharide diacid of formula **5-C,** wherein R¹ is selected from -Bz and -Ac, R² represents -Bn, W represents -L-P or -Bn, P is -N(Bn)Cbz or N₃, L represents -(CH₂)ₙ- and n is an integer selected from 2, 3, 4 and 5.

It is preferred that the oxidation of octasaccharide of formula **4-C** is performed in the presence of at least one oxidizing agent in a mixture of water and an aprotic apolar solvent at low temperatures between -10 °C and 10 °C. It is even more preferred that the oxidation of octasaccharide of formula **4-C** is performed in the presence of a catalytic amount of a first oxidizing agent and a second oxidizing agent in a mixture of water and an aprotic apolar solvent at low temperatures between -10 °C and 10 °C. It is even more preferred that the oxidation of octasaccharide of formula **4-C** is performed in the presence of a catalytic amount of TEMPO and a second oxidizing agent in a mixture of water and an aprotic apolar solvent at low temperatures between -10 °C and 10 °C. It is even more preferred that the oxidation of octasaccharide of formula **4-C** is performed in the presence of a catalytic amount of TEMPO and bis(acetoxy)iodobenzene (BAIB) in a mixture of water and an aprotic apolar solvent at low temperatures between -10 °C and 10 °C. It is even more preferred that the oxidation of octasaccharide of formula **4-C** is performed in the presence of a catalytic amount of TEMPO and bis(acetoxy)iodobenzene (BAIB) in a mixture of water and methylene chloride at low temperatures between -10 °C and 10 °C. It is especially preferred that the oxidation of octasaccharide of formula **4-C** is performed in the presence of a catalytic amount of TEMPO and bis(acetoxy)iodobenzene (BAIB) in a mixture of water and methylene chloride at 0 °C. According to the present invention the octasaccharide diacid of formula **5-C** is preferably obtained in form of crystals by recrystallization or trituration.

Thus, it is especially preferred that the inventive method for preparation of octasaccharide diacid of formula **5-C** comprises the following step: Performing oxidation of the octasaccharide of formula **4-C** with a catalytic amount of TEMPO and BAIB to yield a octasaccharide diacid of formula **5-C,** wherein R¹ is -Bz, R² is -Bn, W represents -L-P or -Bn, P is -N(Bn)CBz, L represents -(CH₂)ₙ-and n is an integer selected from 2, 3, 4 and 5.

### Preparation of octasaccharide diacid of formula 12-C

The preparation of octasaccharide diacid of formula **12-C** according to the present invention comprises the following step:
Performing oxidation of the octasaccharide of formula **11-C** to yield an octasaccharide diacid of formula **12-C,** 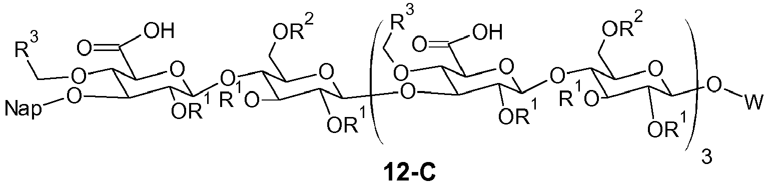 wherein
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

Preferably the inventive method for preparation of octasaccharide diacid of formula **12-C** comprises the following step: Performing oxidation of the octasaccharide of formula **11-C** to yield an octasaccharide diacid of formula **12-C,** wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -NR'R", R and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide diacid of formula **12-C** comprises the following step: Performing oxidation of the octasaccharide of formula **11-C** to yield an octasaccharide diacid of formula **12-C,** wherein R¹ represents -Bz, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide diacid of formula **12-C** comprises the following step: Performing oxidation of the octasaccharide of formula **11-C** to yield an octasaccharide diacid of formula **12-C,** wherein R¹ is selected from -Bz, -ClAc, -Ac, and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

More preferably the inventive method for preparation of octasaccharide diacid of formula **12-C** comprises the following step: Performing oxidation of the octasaccharide of formula **11-C** to yield an octasaccharide diacid of formula **12-C,** wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

Even more preferably the inventive method for octasaccharide diacid of formula **12-C** comprises the following step: Performing oxidation of the octasaccharide of formula **11-C** to yield an octasaccharide diacid of formula **12-C,** wherein R¹ is selected from -Bz, -ClAc, -Ac, and -Piv, R² is selected from -Bz, -Ac, -Piv, -Bn and -TBS, R³ is an aryl group, W represents -L-P or -Bn, P is -NR'R", R'and R" represent independently of each other -Bn, -Cbz, -Allyl or R' and R" form together phthaloyl, L represents -(CH₂)ₙ- and n is an integer selected from 2, 3, 4 and 5.

Even more preferably the inventive method for preparation of octasaccharide diacid of formula **12-C** comprises the following step: Performing oxidation of the octasaccharide of formula **11-C** to yield an octasaccharide diacid of formula **12-C,** wherein R¹ is selected from -Bz and -Ac, R² represents -Bn, R³ is -Ph, W represents -L-P or -Bn, P is -N(Bn)Cbz or N₃, L represents -(CH₂)ₙ- and n is an integer selected from 2, 3, 4 and 5.

It is preferred that the oxidation of octasaccharide of formula **11-C** is performed in the presence of at least one oxidizing agent in a mixture of water and an aprotic apolar solvent at low temperatures between -10 °C and 10 °C. It is even more preferred that the oxidation of octasaccharide of formula **11-C** is performed in the presence of a catalytic amount of a first oxidizing agent and a second oxidizing agent in a mixture of water and an aprotic apolar solvent at low temperatures between -10 °C and 10 °C. It is even more preferred that the oxidation of octasaccharide of formula **11-C** is performed in the presence of a catalytic amount of TEMPO and a second oxidizing agent in a mixture of water and an aprotic apolar solvent at low temperatures between -10 °C and 10 °C. It is even more preferred that the oxidation of octasaccharide of formula **11-C** is performed in the presence of a catalytic amount of TEMPO and bis(acetoxy)iodobenzene (BAIB) in a mixture of water and an aprotic apolar solvent at low temperatures between -10 °C and 10 °C. It is even more preferred that the oxidation of octasaccharide of formula **11-C** is performed in the presence of a catalytic amount of TEMPO and bis(acetoxy)iodobenzene (BAIB) in a mixture of water and methylene chloride at low temperatures between -10 °C and 10 °C. It is especially preferred that the oxidation of octasaccharide of formula **11-C** is performed in the presence of a catalytic amount of TEMPO and bis(acetoxy)iodobenzene (BAIB) in a mixture of water and methylene chloride at 0 °C. According to the present invention the octasaccharide diacid of formula **12-C** is preferably obtained in form of crystals by recrystallization or trituration.

Thus, it is especially preferred that the inventive method for preparation of octasaccharide diacid of formula **12-C** comprises the following step: Performing oxidation of the octasaccharide of formula **11-C** with a catalytic amount of TEMPO and BAIB to yield a octasaccharide diacid of formula **12-C,** wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of octasaccharide of formula 7-C

The preparation of octasaccharide of formula **7-C** according to the present invention comprises the following step: Performing removal of protecting groups R¹ of octasaccharide of formula **5-C** in presence of a base to yield octasaccharide of formula **7-C,** 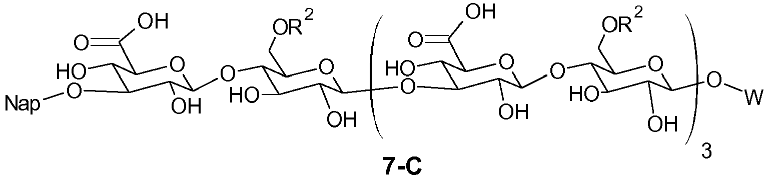 wherein
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

It is preferred that the inventive method for preparation of octasaccharide of formula **7-C** comprises the following step: Performing removal of protecting groups R¹ of octasaccharide of formula **5-C** in presence of a base to yield octasaccharide of formula **7-C,** wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of octasaccharide of formula **7-C** comprises the following step: Performing removal of protecting groups R¹ of octasaccharide of formula **5-C** in presence of a base to yield octasaccharide of formula **7-C,** wherein R¹ represents -Bz, R² is selected from -Bz, -Ac, -Piv, -Bn and -TBS, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation octasaccharide of formula **7-C** comprises the following step: Performing removal of protecting groups R¹ of octasaccharide of formula **5-C** in presence of a base to yield octasaccharide of formula **7-C,** wherein R¹ is selected from -Bz, -ClAc, -Ac and -Piv, R² represents -Bn, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of octasaccharide of formula **7-C** comprises the following step: Performing removal of protecting groups R¹ of octasaccharide of formula 5-C in presence of a base to yield octasaccharide of formula **7-C,** wherein R¹ is selected from -Bz, -ClAc, -Ac and -Piv, R² is selected from -Bz, -Ac, -Piv, -Bn and -TBS, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of octasaccharide of formula **7-C** comprises the following step: Performing removal of protecting groups R¹ of octasaccharide of formula **5-C** in presence of a base to yield octasaccharide of formula **7-C,** wherein R¹ is selected from -Bz, -ClAc, -Ac and -Piv, R² is selected from -Bz, -Ac, -Piv, -Bn and -TBS, W represents -L-P or -Bn, P is -NR'R", R' and R" represent independently of each other -Bn, -Cbz, - Allyl or R' and R" form together phthaloyl, L represents -(CH₂)ₙ- and n is an integer selected from 2, 3, 4 and 5.

It is even more preferred that the inventive method for preparation of octasaccharide of formula **7-C** comprises the following step: Performing removal of protecting groups R¹ of octasaccharide of formula **5-C** in presence of a base to yield octasaccharide of formula **7-C,** wherein R¹ is selected from -Bz and -Ac, R² represents -Bn, W represents -L-P or -Bn, P is -N(Bn)Cbz or N₃, L represents -(CH₂)ₙ- and n is an integer selected from 2, 3, 4 and 5.

Preferably, the removal of protecting groups R¹ of octasaccharide of formula **5-C** to yield octasaccharide of formula **7-C** is performed in presence of a base in a protic solvent at elevated temperatures between 25 °C and 60 °C. More preferably, the removal of protecting groups R¹ of octasaccharide of formula **5-C** to yield octasaccharide of formula **7-C** is performed in presence of sodium methoxide in a protic solvent at elevated temperatures between 25 °C and 60 °C. More preferably, the removal of protecting groups R¹ of octasaccharide of formula 5-C to yield octasaccharide of formula **7-C** is performed in presence of sodium methoxide in an alcohol at elevated temperatures between 30 °C and 50 °C. Even more preferably, the removal of protecting groups R¹ of octasaccharide of formula 5-C to yield octasaccharide of formula **7-C** is performed in presence of sodium methoxide in methanol at 40 °C. According to the present invention the octasaccharide of formula **7-C** is preferably obtained in form of crystals by recrystallization or trituration.

Thus, it is especially preferred that the inventive method for preparation of octasaccharide of formula **7-C** comprises the following step: Performing removal of protecting groups R¹ of octasaccharide of formula **5-C** in presence of sodium methoxide in methanol to yield octasaccharide of formula **7-C,** wherein R¹ is -Bz, R² is -Bn, W represents -L-P or -Bn, P is -N(Bn)CBz, L represents -(CH₂)ₙ-and n is an integer selected from 2, 3, 4 and 5.

### Preparation of octasaccharide of formula 13-C

The preparation of octasaccharide of formula **13-C** according to the present invention comprises the following step: Performing removal of protecting groups R¹ of octasaccharide of formula **12-C** in presence of a base to yield octasaccharide of formula **13-C,** 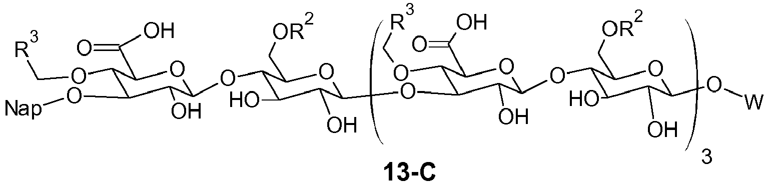 wherein
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
   R' and R" form together phthaloyl,
L represents -(CH₂)ₙ- or -C₂H₄-O-C₂H₄-,
n is an integer selected from 2, 3, 4, 5, 6, 7, 8, 9 and 10.

It is preferred that the inventive method for preparation of octasaccharide of formula **13-C** comprises the following step: Performing removal of protecting groups R¹ of octasaccharide of formula **12-C** in presence of a base to yield octasaccharide of formula **13-C,** wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents - C₂H₄-O-C₂H₄- or -(CH₂)ₙ- and n is an integer selected from 2, 3, 4, 5, 6, 7, 8, 9 and 10.

It is more preferred that the inventive method for preparation of octasaccharide of formula **13-C** comprises the following step: Performing removal of protecting groups R¹ of octasaccharide of formula **12-C** in presence of a base to yield octasaccharide of formula **13-C,** wherein R¹ represents -Bz, R² is selected from -Bz, -Ac, -Piv, -Bn and -TBS, R³ is an aryl group, W represents -L-P or -Bn, P is - NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is more preferred that the inventive method for preparation of octasaccharide of formula **13-C** comprises the following step: Performing removal of protecting groups R¹ of octasaccharide of formula **12-C** in presence of a base to yield octasaccharide of formula **13-C,** wherein R¹ is selected from -Bz, -ClAc, -Ac and -Piv, R² represents -Bn, R³ is an aryl group, W represents -L-P or -Bn, P is -NR'R", R' and R" represent independently of each other -Bn, -Cbz, -Allyl or R' and R" form together phthaloyl, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of octasaccharide of formula **13-C** comprises the following step: Performing removal of protecting groups R¹ of octasaccharide of formula **12-C** in presence of a base to yield octasaccharide of formula **13-C,** wherein R¹ is selected from -Bz, -ClAc, -Ac and -Piv, R² is selected from -Bz, -Ac, -Piv, -Bn and -TBS, R³ is an aryl group, W represents -L-P or -Bn, P is -N(Bn)Cbz, L represents -C₂H₄-O-C₂H₄- or -(CH₂)ₙ- and n is an integer selected from 2, 3, 4, 5, 6, 7, 8, 9 and 10.

It is even more preferred that the inventive method for preparation of octasaccharide of formula **13-C** comprises the following step: Performing removal of protecting groups R¹ of octasaccharide of formula **12-C** in presence of a base to yield octasaccharide of formula **13-C,** wherein R¹ is selected from -Bz, -ClAc, -Ac and -Piv, R² is selected from -Bz, -Ac, -Piv, -Bn and -TBS, R³ is an aryl group, W represents -L-P or -Bn, P is -NR'R", R' and R" represent independently of each other -Bn, -Cbz, -Allyl or R' and R" form together phthaloyl, L represents - (CH₂)ₙ- and n is an integer selected from 2, 3, 4 and 5.

It is even more preferred that the inventive method for preparation of octasaccharide of formula **13-C** comprises the following step: Performing removal of protecting groups R¹ of octasaccharide of formula **12-C** in presence of a base to yield octasaccharide of formula **13-C,** wherein R¹ is selected from -Bz and -Ac, R² represents -Bn, R³ is -Ph, W represents -L-P or -Bn, P is -N(Bn)Cbz or N₃, L represents -(CH₂)ₙ- and n is an integer selected from 2, 3, 4 and 5.

Preferably, the removal of protecting groups R¹ of octasaccharide of formula **12-C** to yield octasaccharide of formula **13-C** is performed in presence of a base in a protic solvent at elevated temperatures between 25 °C and 60 °C. More preferably, the removal of protecting groups R¹ octasaccharide of formula **12-C** to yield octasaccharide of formula **13-C** is performed in presence of sodium methoxide in a protic solvent at elevated temperatures between 25 °C and 60 °C. More preferably, the removal of protecting groups R¹ of octasaccharide of formula **12-C** to yield octasaccharide of formula **13-C** is performed in presence of sodium methoxide in an alcohol at elevated temperatures between 30 °C and 50 °C. Even more preferably, the removal of protecting groups R¹ of octasaccharide of formula **12-C** to yield octasaccharide of formula **13-C** is performed in presence of sodium methoxide in methanol at 40 °C. According to the present invention the octasaccharide of formula **13-C** is preferably obtained in form of crystals by recrystallization or trituration.

Thus, it is especially preferred that the inventive method for preparation of octasaccharide of formula **13-C** comprises the following step: Performing removal of protecting groups R¹ of octasaccharide of formula **12-C** in presence of sodium methoxide in methanol to yield octasaccharide of formula **13-C,** wherein R¹ is -Bz, R² is -Bn, R³ is -Ph, W represents -L-P or -Bn, P is -N(Bn)CBz, L represents -(CH₂)ₙ- and n is an integer selected from 2, 3, 4 and 5.

### Preparation of octasaccharide of formula 8-C

The preparation of octasaccharide of formula **8-C** or octasaccharide of formula **8'-C** according to the present invention comprises the following step: Hydrogenating the octasaccharide of formula **7-C** in presence of a catalyst to yield an octasaccharide of formula **8-C** or an octasaccharide of formula **8'-C,** 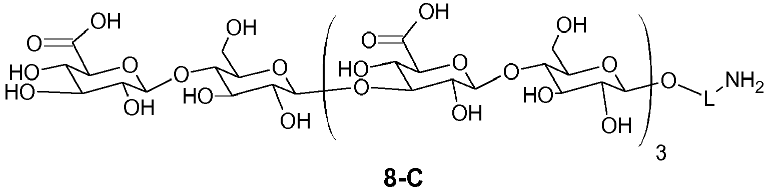 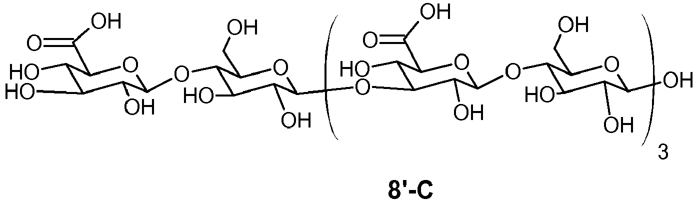
wherein L represents -(CH₂)ₙ- or -C₂H₄-O-C₂H₄- and
n is an integer selected from 2, 3, 4, 5, 6, 7, 8, 9 and 10.

Preferably, the inventive method for preparation of octasaccharide of formula **8-C** or octasaccharide of formula **8'-C** comprises the following step: Hydrogenating the octasaccharide of formula **7-C** in presence of a catalyst to yield an octasaccharide of formula **8-C** or an octasaccharide of formula **8'-C,** wherein L represents -C₂H₄-O-C₂H₄- or -(CH₂)ₙ- and n is an integer selected from 2, 3, 4, 5, 6, 7 and 8. More preferably, the inventive method for preparation of octasaccharide of formula **8-C** or octasaccharide of formula **8'-C** comprises the following step: Performing a hydrogenation reaction of the octasaccharide of formula **7-C** in presence of a catalyst to yield an octasaccharide of formula **8-C** or an octasaccharide of formula **8'-C,** wherein L represents -(CH₂)ₙ- and n is an integer selected from 2, 3, 4, and 5.

It is preferred that the hydrogenation reaction of the octasaccharide of formula **7-C** to yield an octasaccharide of formula **8-C** or an octasaccharide of formula **8'-C** is performed in the presence of a catalyst in a mixture of an alcohol and an aprotic apolar solvent. It is more preferred that the hydrogenation reaction of the octasaccharide of formula **7-C** to yield an octasaccharide of formula **8-C** or an octasaccharide of formula **8'-C** is performed in the presence of a palladium catalyst in a mixture of an alcohol and an aprotic apolar solvent It is even more preferred that the hydrogenation reaction of the octasaccharide of formula **7-C** to yield an octasaccharide of formula **8-C** or an octasaccharide of formula **8'-C** is performed in the presence of palladium on carbon (Pd/C) and/or Pd(OH)₂/C in a mixture of an alcohol and an aprotic apolar solvent. It is even more preferred that the hydrogenation reaction of the octasaccharide of formula **7-C** to yield an octasaccharide of formula **8-C** or an octasaccharide of formula **8'-C** is performed in the presence of palladium on carbon (Pd/C) and/or Pd(OH)₂/C in a mixture of tBuOH and methylene chloride.

Thus, it is especially preferred that the inventive method for preparation of octasaccharide of formula **8-C** or an octasaccharide of formula **8'-C** comprises the following step: Hydrogenating the octasaccharide of formula **7-C** in the presence of Pd/C and/or Pd(OH)₂/C to yield an octasaccharide of formula **8-C,** wherein L represents -(CH₂)ₙ- and n is an integer selected from 2, 3, 4, and 5 or an octasaccharide of formula **8'-C.**

In an alternative embodiment of the present invention the preparation of octasaccharide of formula **8-C** or an octasaccharide of formula **8'-C** comprises the following step:
Hydrogenating the octasaccharide of formula **13-C** in presence of a catalyst to yield an octasaccharide of formula **8-C** or an octasaccharide of formula **8'-C,** 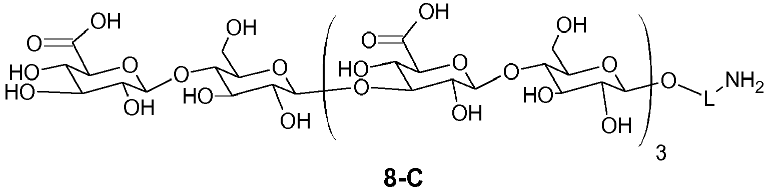 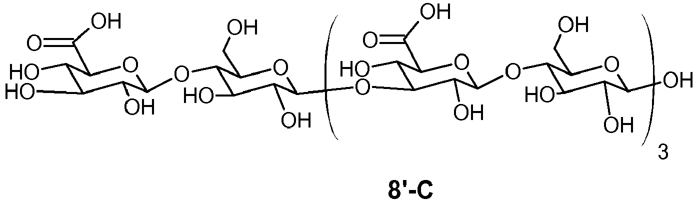
wherein L represents -(CH₂)ₙ- or -C₂H₄-O-C₂H₄- and
n is an integer selected from 2, 3, 4, 5, 6, 7, 8, 9 and 10.

Preferably, the inventive method for preparation of octasaccharide of formula **8-C** or octasaccharide of formula **8'-C** comprises the following step: Hydrogenating the octasaccharide of formula **13-C** in presence of a catalyst to yield an octasaccharide of formula **8-C** or an octasaccharide of formula **8'-C,** wherein L represents -C₂H₄-O-C₂H₄- or -(CH₂)ₙ- and n is an integer selected from 2, 3, 4, 5, 6, 7 and 8. More preferably, the inventive method for preparation of octasaccharide of formula **8-C** or octasaccharide of formula **8'-C** comprises the following step: Performing a hydrogenation reaction of the octasaccharide of formula **13-C** in presence of a catalyst to yield an octasaccharide of formula **8-C** or an octasaccharide of formula **8'-C,** wherein L represents -(CH₂)ₙ- and n is an integer selected from 2, 3, 4, and 5.

It is preferred that the hydrogenation reaction of the octasaccharide of formula **13-C** to yield an octasaccharide of formula **8-C** or an octasaccharide of formula **8'-C** is performed in the presence of a catalyst in a mixture of an alcohol and an aprotic apolar solvent. It is more preferred that the hydrogenation reaction of the octasaccharide of formula **13-C** to yield an octasaccharide of formula **8-C** or an octasaccharide of formula **8'-C** is performed in the presence of a palladium catalyst in a mixture of an alcohol and an aprotic apolar solvent It is even more preferred that the hydrogenation reaction of the octasaccharide of formula **13-C** to yield an octasaccharide of formula **8-C** or an octasaccharide of formula **8'-C** is performed in the presence of palladium on carbon (Pd/C) and/or Pd(OH)₂/C in a mixture of an alcohol and an aprotic apolar solvent. It is even more preferred that the hydrogenation reaction of the octasaccharide of formula **13-C** to yield an octasaccharide of formula **8-C** or an octasaccharide of formula **8'-C** is performed in the presence of palladium on carbon (Pd/C) and/or Pd(OH)₂/C in a mixture of tBuOH and methylene chloride.

Thus, it is especially preferred that the inventive method for preparation of octasaccharide of formula **8-C** or an octasaccharide of formula **8'-C** comprises the following step: Hydrogenating the octasaccharide of formula **13-C** in the presence of Pd/C and/or Pd(OH)₂/C to yield an octasaccharide of formula **8-C,** wherein L represents -(CH₂)ₙ- and n is an integer selected from 2, 3, 4, and 5 or an octasaccharide of formula **8'-C.**

The present invention provides very favorable and efficient methods for synthesizing the tetrasaccharide of formula **8-A** and formula **8'-A,** the hexasaccharide of formula **8-B** and formula **8'-B** and the octasaccharide of formula **8-C** and formula **8'-C** as well as their conjugates selectively and in high yields.

These methods involve the use of one or more of molecules of general formula 1', **1", 1 "', 1e, 1f, 1h, 2a, 4', 4", 4e, 4f, 5', 5", 5e, 5f, 7', 7", 7e, 7f, 9', 9", 9e, 9f** and **14a,** wherein n is an integer selected from 2, 3, 4, 5, 6, 7, 8, 9 and 10, and r is 1 or 2 or 3, as shown or defined below. Preferably n is an integer selected from 2, 3, 4 and 5. Especially preferred is, when n is selected from 2 and 5. 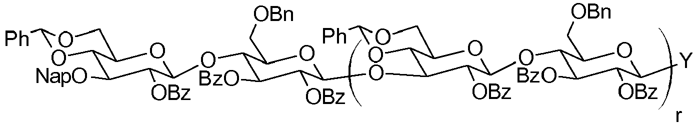
**1'** Y = -O-(CH₂)ₙ-N(Bn)Cbz
**1"** Y = -O-(CH₂)ₙ-N₃
**1'"** Y = -O-(CH₂)ₙ-NH₂
**1e** Y = -O-C₂H₄-O-C₂H₄-N₃
**1f** Y = -O-Bn
**1h** Y = -O-(CH₂)₂NH-CO-(CH₂)₂-S-Ac 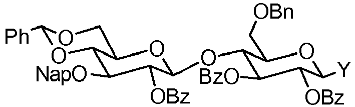
**2a** Y = -SEt
**9'** Y = -O-(CH₂)ₙ-N(Bn)Cbz
**9"** Y = -O-(CH₂)ₙ-N₃
**9e** Y = -O-C₂H₄-O-C₂H₄-N₃
**9f Y** = -O-Bn 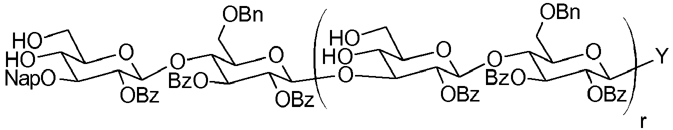
**4'** Y = -O-(CH₂)ₙ-N(Bn)Cbz
**4"** Y = -O-(CH₂)ₙ-N₃
**4e** Y = -O-C₂H₄-O-C₂H₄-N₃
**4f** Y = -O-Bn 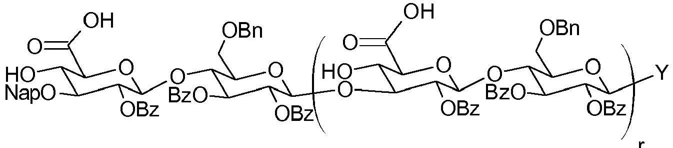
**5'** Y = -O-(CH₂)ₙ-N(Bn)Cbz
**5"** Y = -O-(CH₂)ₙ-N₃
**5e** Y = -O-C₂H₄-O-C₂H₄-N₃
**5f** Y = -O-Bn 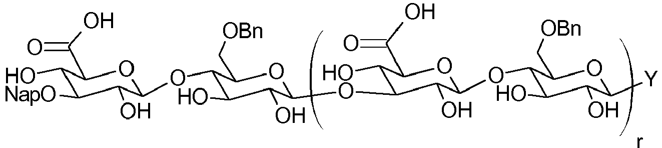
**7'** Y = -O-(CH₂)ₙ-N(Bn)Cbz
**7"** Y = -O-(CH₂)ₙ-N₃
**7e** Y = -O-C₂H₄-O-C₂H₄-N₃
**7f** Y = -O-Bn

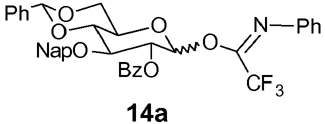

### ST3 saccharide conjugates

The method for preparing the ST3 tetrasaccharide conjugate, ST3 hexasaccharide conjugate or ST3 octasaccharide conjugate of the present invention typically comprises coupling the ST3 tetrasaccharide, the ST3 hexasaccharide or ST3 octasaccharide of the invention bearing a linker or spacer group, in particular wherein the linker is -(CH₂)ₙ-NH₂, with n being an integer from 2 to 10, preferably from 2 to 5, i.e. saccharide of formula **8**, with a carrier protein or a glycosphingolipid.

More specifically, the method for preparing the ST3 tetrasaccharide conjugate, ST3 hexasaccharide conjugate or ST3 octasaccharide conjugate of the present invention typically comprises the following two steps:
a4) reacting the saccharide of formula **8** wherein L is -(CH₂)ₙ-, with n being selected from 2, 3, 4, 5, 6, 7, 8, 9 and 10; with a linker J¹-L²-J² under addition of a base to yield saccharide of formula **28,** 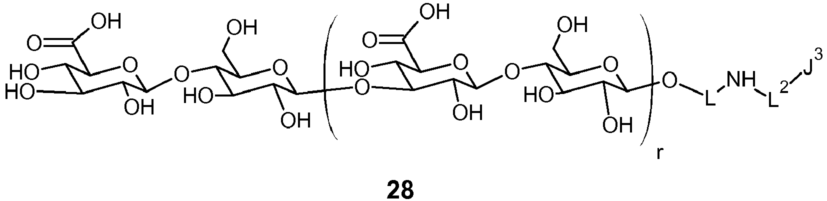
   wherein L represents -(CH₂)ₙ-,
   n is an integer selected from 2, 3, 4, 5, 6, 7, 8, 9 and 10,
   r is 1 or 2 or 3,
   J³ is J¹ or J²,
   L² is selected from:
      -C(O)-, -E-, -C(O)-NH-NH-C(O)-, 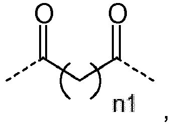 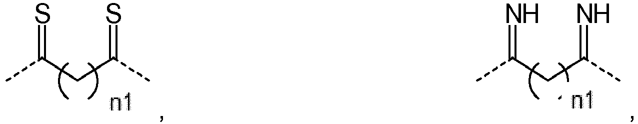 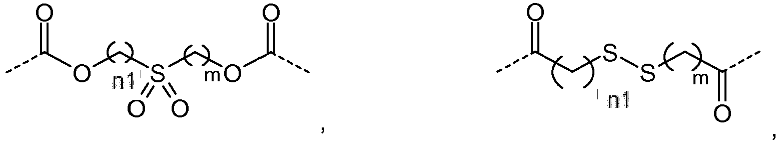 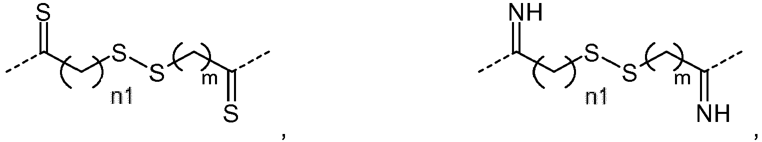 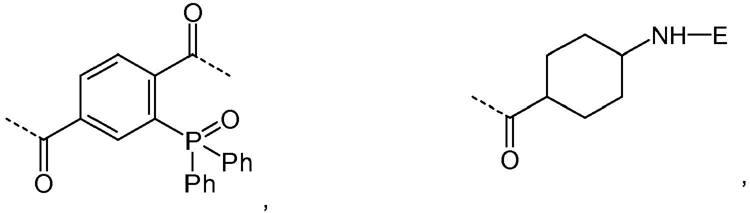 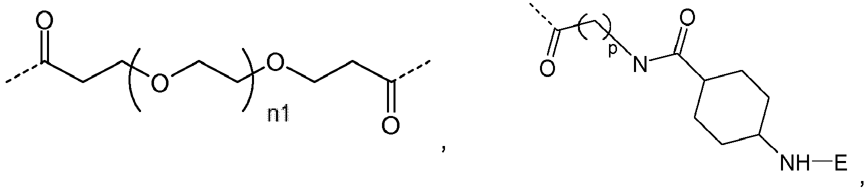 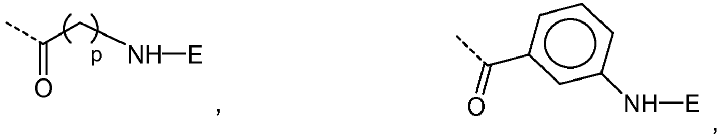 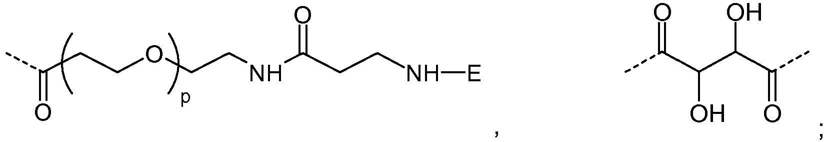
   E is selected from 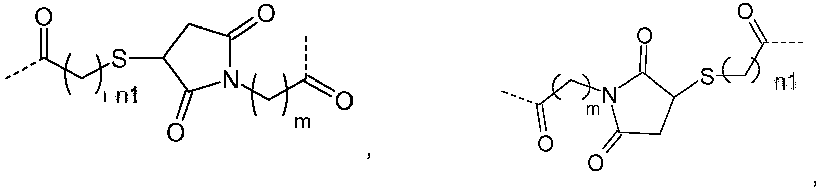 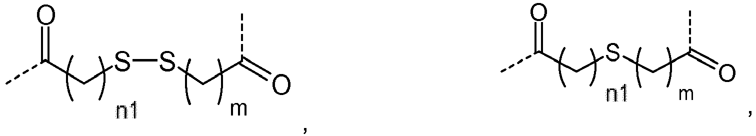 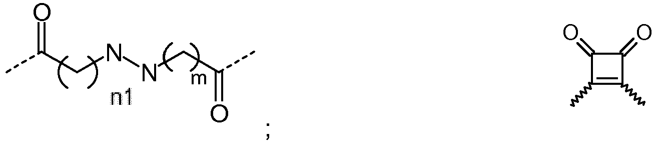
   m, n1, o and p represent independently of each other an integer from 1 to 10 and
   J¹ and J² are selected independently of each other from 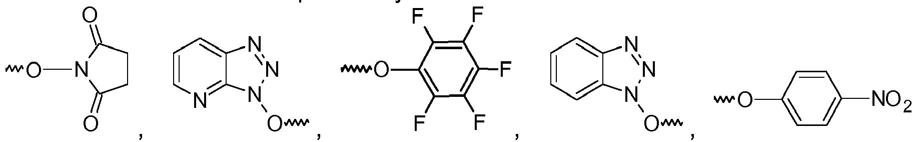
and b4) coupling the saccharide of formula **28** to a carrier protein or a glycosphingolipid.

More preferably, the method for preparing the ST3 tetrasaccharide conjugate of the present invention comprises a4) reacting the tetrasaccharide of formula **8-A** wherein L is -(CH₂)ₙ-, with n being selected from 2, 3, 4, and 5; with the linker di(N-succinimidyl) adipate under addition of a base to yield tetrasaccharide of formula **28-A,** and b4) coupling the tetrasaccharide of formula **28-A** to a carrier protein or a glycosphingolipid.

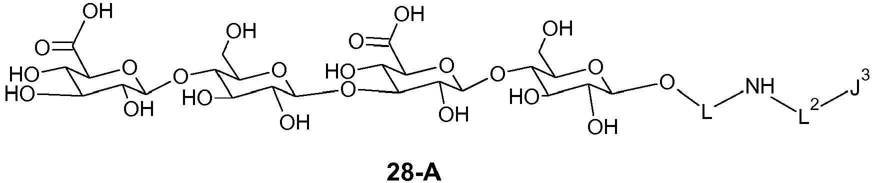

In one preferred embodiment according to the present invention said method for preparing the ST3 tetrasaccharide conjugate comprises a4) reacting the tetrasaccharide of formula **8-A** wherein L is -(CH₂)ₙ-, with n being selected from 2, 3, 4, and 5; with the linker di(N-succinimidyl) adipate under addition of a base to yield tetrasaccharide of formula **28-A,** and b4) coupling the tetrasaccharide of formula **28-A** to a carrier protein.

In an even more preferred embodiment according to the present invention said method for preparing the ST3 tetrasaccharide conjugate comprises a4) reacting the tetrasaccharide of formula **8-A** wherein L is -(CH₂)ₙ-, with n being selected from 2, 3, 4, and 5; with the linker di(N-succinimidyl) adipate under addition of triethylamine to yield tetrasaccharide of formula **28-A,** and b4) coupling the tetrasaccharide of formula **28-A** to a CRM₁₉₇ carrier protein.

More preferably, the method for preparing the ST3 hexasaccharide conjugate of the present invention comprises a4) reacting the hexasaccharide of formula **8-B** wherein L is -(CH₂)ₙ-, with n being selected from 2, 3, 4, and 5; with the linker di(N-succinimidyl) adipate under addition of a base to yield hexasaccharide of formula **28-B,** and b4) coupling the hexasaccharide of formula **28-B** to a carrier protein or a glycosphingolipid.

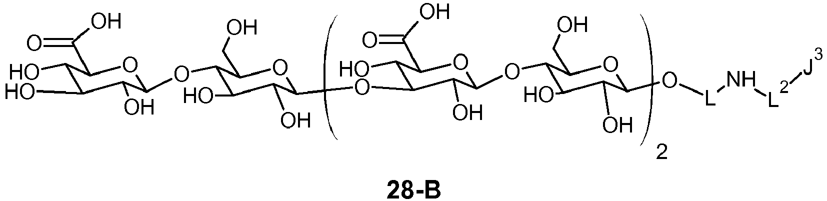

In one preferred embodiment according to the present invention said method for preparing the ST3 hexasaccharide conjugate comprises a4) reacting the hexasaccharide of formula **8-B** wherein L is -(CH₂)ₙ-, with n being selected from 2, 3, 4, and 5; with the linker di(N-succinimidyl) adipate under addition of a base to yield hexasaccharide of formula **28-B,** and b4) coupling the hexasaccharide of formula **28-B** to a carrier protein.

In an even more preferred embodiment according to the present invention said method for preparing the ST3 hexasaccharide conjugate comprises a4) reacting the hexasaccharide of formula **8-B** wherein L is -(CH₂)ₙ-, with n being selected from 2, 3, 4, and 5; with the linker di(N-succinimidyl) adipate under addition of triethylamine to yield hexasaccharide of formula **28-B,** and b4) coupling the hexasaccharide of formula **28-B** to a CRM₁₉₇ carrier protein.

More preferably, the method for preparing the ST3 octasaccharide conjugate of the present invention comprises a4) reacting the octasaccharide of formula **8-C** wherein L is -(CH₂)ₙ-, with n being selected from 2, 3, 4, and 5; with the linker di(N-succinimidyl) adipate under addition of a base to yield octasaccharide of formula **28-C,** and b4) coupling the octasaccharide of formula **28-C** to a carrier protein or a glycosphingolipid.

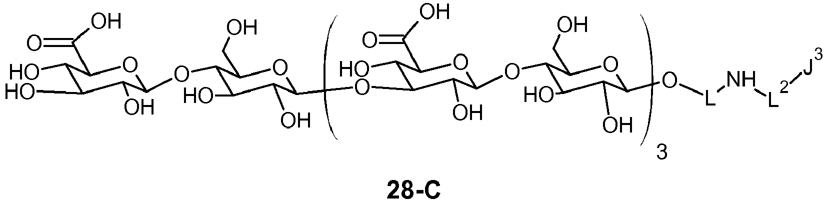

In one preferred embodiment according to the present invention said method for preparing the ST3 octasaccharide conjugate comprises a4) reacting the octasaccharide of formula **8-C** wherein L is -(CH₂)ₙ-, with n being selected from 2, 3, 4, and 5; with the linker di(N-succinimidyl) adipate under addition of a base to yield octasaccharide of formula **28-C,** and b4) coupling the octasaccharide of formula **28-C** to a carrier protein.

In an even more preferred embodiment according to the present invention said method for preparing the ST3 octasaccharide conjugate comprises a4) reacting the octasaccharide of formula **8-C** wherein L is -(CH₂)ₙ-, with n being selected from 2, 3, 4, and 5; with the linker di(N-succinimidyl) adipate under addition of triethylamine to yield octasaccharide of formula **28-C,** and b4) coupling the octasaccharide of formula **28-C** to a CRM₁₉₇ carrier protein.

### EXAMPLES

### Abbreviations

- NIS: N-iodosuccinimide;
- TfOH: triflic acid
- h: hour(s)
- d: day(s)
- DCM: dichloromethane
- TLC: thin layer chromatography
- MS: molecular sieves
- TEMPO: 2,2,6,6-tetramethyl-1-piperidinyloxy, free radical
- Cbz: carbonyloxybenzyl
- Nap: 2-naphthylmethyl
- BAIB: bis(acetoxy)iodobenzene
- PTSA: para-toluenesulfonic acid
- PMP: para-methoxyphenyl
- r.t. / RT: room temperature
- RM: reaction mixture

### General information

Commercial grade solvents were used unless stated otherwise. Dry solvents were obtained from a Waters Dry Solvent System. Solvents for chromatography were distilled prior to use. Sensitive reactions were carried out in heat-dried glassware and under an argon atmosphere. Analytical thin layer chromatography (TLC) was performed on silica gel 60 F254 glass plates precoated with a 0.25 mm thickness of silica gel. Spots were visualized by staining with vanillin solution (6% (w/v) vanillin and 10% (v/v) sulfuric acid in 95% EtOH) or Hanessian's stain (5% (w/v) ammonium molybdate, 1% (w/v) cerium(II) sulfate and 10% (v/v) sulfuric acid in water). Silica column chromatography was performed on Fluka silica gel 60 (230-400 mesh).

¹H, ¹³C and two-dimensional NMR spectra were measured with a Varian 400-MR, 600-MR and Bruker Avance 700 spectrometer at 296 K. Chemical shifts (δ) are reported in parts per million (ppm) relative to the respective residual solvent peaks (CDCl₃: δ 7.27 in ¹H and 77.16 in ¹³C NMR; CD₃OD: δ 3.31 in ¹H and 49.00 in ¹³C NMR; D₂O: δ 4.80 in ¹H NMR; acetone-d₆: δ 2.05 in ¹H and 29.92 in ¹³C NMR). The following abbreviations are used to indicate peak multiplicities: s singlet; d doublet; *dd* doublet of doublets; *t* triplet; *dt* doublet of triplets; *q* quartet; *m* multiplet. Coupling constants (*J*) are reported in Hertz (Hz). Optical rotation (OR) measurements were carried out with a Schmidt & Haensch UniPol L1000 polarimeter at λ = 589 nm and a concentration (c) expressed in g/100 mL in the solvent noted in parentheses. High resolution mass spectrometry (HRMS) was performed at the Free University Berlin, Mass Spectrometry Core Facility, with an Agilent 6210 TOF mass spectrometer. Infrared (IR) spectra were measured with a Perkin Elmer 100 FTIR spectrometer.

### A. Synthesis of monosaccharide building blocks

### Example A.1 Synthesis of the C3-NAP protected glucose 23

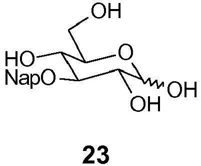

a) Diacetone glucose **21** (16.2 g, 62.2 mmol) was dissolved in anhydrous dimethylformamide (80 mL) at 0 °C. To this solution, sodium hydride (1.17 g, 74.7 mmol, 60% in mineral oil) was added and the solution was stirred until all gas evolution has ceased (2 h). To this solution, 2-(bromomethyl)naphthalene (40.8 g, 184 mmol) was added and the reaction was stirred for 16 h under argon. The reaction was quenched by slow addition of sat. aq. NH₄Cl solution. The mixture was extracted with diethyl ether and the organic layer was washed with brine. The organic layer was dried over MgSO₄ and concentrated *in vacuo* to give a viscous oil.
b) Water (0.5 L) and Amberlite® IR120 (50 g, H⁺) were added to the viscous oil and the reaction was stirred at 80 °C for 16 h. After cooling to room temperature, Amberlite® IR120 was filtered off and the aqueous layer was washed with a mixture of Et₂O-EtOAc 1:1. The aqueous layer was evaporated to yield a white paste, which was azeotropically dried using toluene. The pure compound 3-*O*-(2-naphthylmethyl)-D-glucopyranose **23** was obtained as a white solid (13.3 g, 67% over two steps). **¹H NMR** (400 MHz, CD₃OD): δ 7.89 - 7.77 (m, 4H), 7.55 (d, *J =* 8.8 Hz, 1H), 7.44 - 7.39 (m, 2H), 5.05-4.98 (m, 2H), 4.48 (d, *J* = 7.8 Hz, 1H), 3.83 (dd, *J* = 11.9, 1.9 Hz, 1H), 3.63 (dd, *J* = 11.8, 5.9 Hz, 1H), 3.41 (dt, *J* = 23.8, 8.8 Hz, 2H), 3.30-3.25 (m, 2H).

### Example A.2 Synthesis of the C3-NAP protected peracetylated glucose 24

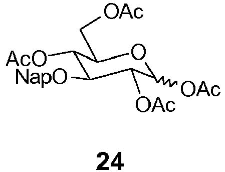

Monosaccharide **23** (16.3 g, 50.9 mmol) and sodium acetate (8.35 g, 102 mmol) were combined in stirred acetic anhydride (96 mL) and heated to 60 °C for 2 h. The reaction mixture was poured into water, forming a white precipitate. The white precipitate was dissolved in dichloromethane and washed with sat. aq. NaHCO₃ solution and brine. The organic layer was dried over MgSO₄ and the solution was concentrated *in vacuo* yielding 1,2,4,6-tetra-*O*-acetyl-3-*O-*(2-naphthylmethyl)-D-glucopyranose **24** as a pure white solid (24.1 g, 97%, α:β = 2:9). **¹H NMR** (400 MHz, CDCl₃): δ 7.80 (dd, *J* = 8.7, 3.2 Hz, 3H), 7.67 (s, 1H), 7.52 - 7.40 (m, 2H), 7.32 (d, *J* = 8.2 Hz, 1H), 5.63 (d, *J* = 8.2 Hz, 1H), 5.18 (td, *J =* 9.3, 5.0 Hz, 2H), 4.76 (s, 2H), 4.21 (dd, *J* = 12.5, 4.8 Hz, 1H), 4.07 (dd, *J* = 12.4, 2.1 Hz, 1H), 3.78 (t, *J* = 9.3 Hz, 1H), 3.74 - 3.69 (m, 1H), 2.09 (s, 3H), 2.07 (s, 3H), 1.92 - 1.90 (bs, 6H). **¹³C NMR** (100 MHz, CDCl₃): δ 170.7, 169.3, 169.2, 169.1, 134.9, 133.1, 133.0, 128.3, 127.9, 127.6, 126.5, 126.3, 126.1, 125.6, 91.9, 79.9, 74.3, 73.0, 71.5, 69.0, 61.7, 20.9, 20.8, 20.7, 20.7.

### Example A.3 Synthesis of monosaccharide 25a

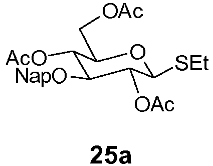

Monosaccharide **24** (5.45 g, 11.16 mmol), ethanethiol (1.45 g, 20.08 mmol) and dried acid-washed 4 A molecular sieve pellets were added to anhydrous dichloromethane (35 mL) and stirred under argon for 1 h. The solution was cooled to 0 °C, followed by drop-wise addition of boron trifluoride diethyl etherate (2.83 mL, 22.31 mmol) over 40 min. The reaction mixture was stirred for 2 h, followed by dilution with dichloromethane. The organic layer was washed with sat. aq. NaHCO₃ solution, water and brine. The organic layer was dried over MgSO₄. Concentration *in vacuo* revealed the product ethyl 2,4,6-tri-*O*-acetyl-3-*O-*(2-naphthylmethyl)-1-thio-β-D-glucopyranoside **25a** as a white foam (5.37 g, 98%). **¹H NMR** (400 MHz, CDCl₃): δ 7.84 - 7.76 (m, 3H), 7.67 (s, 1H), 7.51 - 7.43 (m, 2H), 7.32 (d, *J* = 7.9 Hz, 1H), 5.11 (ABq, *J* = 9.8 Hz, 2H), 4.78 - 4.72 (m, 2H), 4.38 (d, *J* = 10 Hz, 1H), 4.19 - 4.08 (m, 2H), 3.74 (t, *J* = 9.3 Hz, 1H), 3.62-3.55 (m, 1H), 2.75 - 2.61 (m, 2H), 2.06 (s, 3H), 1.96 (s, 3H), 1.91 (s, 3H), 1.24 (t, *J* = 7.4 Hz, 3H).

### Example A.4 Synthesis of monosaccharide 18a

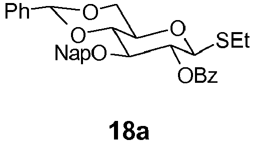

a) Monosaccharide **25a** (5.37 g, 10.90 mmol) and sodium methoxide (1.77 g, 32.7 mmol) were dissolved in anhydrous methanol (30 mL) and stirred under argon for 16 h. The resulting solution was filtered through Amberlite® IR120 (3 g, H⁺). Concentration *in vacuo* revealed the product ethyl 3-O-(2-naphthylmethyl)-1-thio-β-D-glucopyranoside **26a** as a pure white solid.
b) This white solid was added to a stirred solution of benzaldehyde (11 mL). The addition of trifluoroacetic acid (0.52 mL, 7.03 mmol) resulted in the formation of a white precipitate after 10 min. After 1 h, the reaction was quenched by addition of triethylamine (1.5 mL). Filtration, followed by trituration with cold diethyl ether gave the product ethyl 4,6-*O*-benzylidene-3-*O*-(2-naphthylmethyl)-1-thio-(β-D-glucopyranoside **27a** as a white solid.
c) Subsequently, the white solid **27a** was transferred to a stirred solution of pyridine (2 mL) in dichloromethane (20 mL) under argon. Benzoyl chloride (0.91 mL, 7.79 mmol) was added and the reaction mixture was stirred for 1 h. The reaction was quenched with methanol (3 mL), followed by dilution with dichloromethane. The organic layer was washed with sat. aq. NaHCO₃ solution, water and brine. The organic layer was dried over MgSO₄. The solution was concentrated *in vacuo* yielding title compound ethyl 2-*O*-benzoyl-4,6-*O-*benzylidene-3-*O*-(2-naphthylmethyl)-1-thio-β-D-glucopyranoside **18a** as a pure white solid (2.54 g, 44% over 3 steps). **¹H NMR** (400 MHz, CDCl₃): δ 7.93 (d, *J =* 7.2 Hz, 2H), 7.68 (d, *J* = 7.6 Hz, 1H), 7.69 - 7.21 (m, 14H), 5.64 (s, 1H), 5.36 (dd, *J* = 8.5, 1.5 Hz, 1H), 4.97 (d, *J =* 12.2 Hz, 1H), 4.85 (d, *J =* 12.2 Hz, 1H), 4.59 (d, *J* = 10.0 Hz, 1H), 4.41 (dd, *J* = 10.5, 4.9 Hz, 1H), 3.96 - 3.81 (m, 3H), 3.61-3.51 (m, 1H), 2.75 - 2.66 (m, 2H), 1.21 (t, *J* = 7.4 Hz, 2H). **¹³C NMR** (100 MHz, CDCl₃): δ 165.2, 137.2, 135.3, 133.2, 133.1, 132.9, 129.9, 129.7, 129.1, 128.4, 128.1, 127.9, 127.6, 127.0, 126.2, 126.1, 125.9, 125.8, 101.4, 84.3, 81.8, 79.0, 74.2, 71.8, 70.7, 68.7, 24.1, 14.8. **HRMS** (ESI⁺) calculated for C₃₃H₃₂O₆Na [M + Na]+ 579.1817, found 579.1788.

### Example A.5 Synthesis of monosaccharide 14a

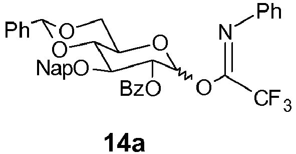

a) Monosaccharide **18a** (1.5 g, 2.63 mmol) and NIS (591 mg, 2.63 mmol) were added to a stirred solution of DCM (30 mL) and H₂O (6 mL) under nitrogen. To this solution was added trifluoromethanesulfonic acid (26 µL, 0.26 mmol) at 0 °C. After 3 h, the reaction was quenched by addition of sat. NaHCO₃ solution and the product was extracted with DCM and washed with sat. Na₂S₂O₃ solution. After phase separation, the organic phase was dried over MgSO₄ and concentrated in vacuum. Purification was achieved by silica gel flash chromatography using ethyl acetate in hexanes (2:5). This gave the product 1-*O*-benzoyl-4,6-*O*-benzylidene-3-*O*-(2-naphthylmethyl)-α-D-glucopyranose **17a** as an off-white solid.
b) Compound **17a** was dissolved in DCM (40 mL), triethylamine (7.0 mL) was added and the reaction mixture was stirred under nitrogen atmosphere overnight. The reaction mixture was concentrated in vacuum and then purified by silica gel flash chromatography using ethyl acetate in hexanes (2:5) to yield 2-*O*-benzoyl-4,6-*O*-benzylidene-3-*O*-(2-naphthylmethyl)-D-glucopyranose **16a** as a pure white solid (1.21 g, 89%).
c) To a stirred solution of compound **16a** (1.2 g, 2.34 mmol) in DCM (30 ml), cesium carbonate (1.52 g, 4.68 mmol) and 2,2,2-trifluoro-N-phenylacetimidoyl chloride (0.76 mL, 4.68 mmol) were added. The reaction mixture was stirred overnight under nitrogen. The reaction mixture was filtered through Celite® and the filtrate was concentrated in vacuum. The crude product was purified by trituration using diethyl ether to yield title compound 2-*O*-benzoyl-4,6-*O-*benzylidene-3-*O*-(2-naphthylmethyl)-D-glucopyranosyl 2,2,2-trifluoro-*N*-phenyl-acetimidate **14a** as a pure white solid (1.1 g, 69%). **¹H NMR** (400 MHz, CDCl₃) δ 8.03 - 7.00 (m, 22H), 6.67 (d, *J* = 7.7 Hz, 2H), 5.65 (s, 1H), 5.55 (t, *J* = 7.7 Hz, 1H), 5.08 - 4.81 (m, 2H), 4.42 (dd, *J* = 10.3, 4.9 Hz, 1H), 3.99 (d, *J* = 7.8 Hz, 2H), 3.87 (t, *J* = 10.2 Hz, 1H). **¹³C NMR** (101 MHz, CDCl₃) δ 164.9, 143.1, 137.2, 135.2, 133.5, 133.2, 133.1, 130.0, 129.4, 129.3, 128.9, 128.6, 128.5, 128.3, 128.0, 127.8, 127.1, 126.2, 126.1, 126.0, 124.6, 119.4, 101.7, 81.3, 77.7, 74.1, 72.5, 68.7, 67.1.

### Synthesis of monosaccharide 14a on a larger scale:

a) Monosaccharide **18a** (10 g, 18 mmol) and NIS (3.94 g, 17.5 mmol) were added to a stirred solution of DCM (80 mL) and H₂O (4 mL) under nitrogen. To this solution was added trifluoromethanesulfonic acid (310 µL, 3.50 mmol) at 0 °C. After 5 h, the reaction was quenched by addition of sat. NaHCO₃ solution. The product was extracted with DCM and washed with sat. Na₂S₂O₃ solution. After phase separation, the organic phase was dried over MgSO₄ and concentrated in vacuum. Purification was achieved by silica gel flash chromatography using ethyl acetate in hexanes (2:5). This gave the product 1-*O*-benzoyl-4,6-*O*-benzylidene-3-*O*-(2-naphthylmethyl)-α-D-glucopyranose **17a** as an off-white solid.
b) Compound **17a** was dissolved in DCM (40 mL), triethylamine (24 mL) was added and the reaction mixture was stirred under nitrogen atmosphere overnight. The reaction mixture was concentrated in vacuum and then purified by silica gel flash chromatography using ethyl acetate in hexanes (2:5) to yield 2-*O*-benzoyl-4,6-*O*-benzylidene-3-*O*-(2-naphthylmethyl)-D-glucopyranose **16a** as a pure white solid (6.89 g, 76%).
c) To a stirred solution of compound **16a** (3.5 g, 6.83 mmol) in DCM (25 ml), cesium carbonate (4.45 g, 13.66 mmol) and 2,2,2-trifluoro-*N*-phenylacetimidoyl chloride (3.54 g, 17.07 mmol) were added. The reaction mixture was stirred overnight under nitrogen. The reaction mixture was filtered through Celite® and the filtrate was concentrated in vacuum. The crude product was purified by trituration using diethyl ether to yield title compound 2-*O*-benzoyl-4,6-*O-*benzylidene-3-*O*-(2-naphthylmethyl)-D-glucopyranosyl 2,2,2-trifluoro-*N*-phenyl-acetimidate **14a** as a pure white solid (3.9 g, 84%).

The synthesis of monosaccharide **16a** (step a) and step b)) as well as the synthesis of monosaccharide **14a** were further carried out on different scales:
step a) + step b)

| **18a [g]** | **16a [g]** | **yield** |
|---|---|---|
| 0.4 g | 0.31 g | 84% |
| 0.55 g | 0.45 g | 89% |
| 1.5 g | 1.21 g | 89% |
| 5g | 3.75 g | 83% |
| 15 g | 10.4 g | 76% |
| 80 g | 54.6 g | 74% |

step c)

| **16a [g]** | **14a [g]** | **yield** |
|---|---|---|
| 0.45 g | 0.6 g | >95%^{a} |
| 1.2 g | 1.13 g | 69% |
| 2.0 g | 2.25 g | 80% |
| 2.76 g | 3.25 g | 83% |
| 3.0 g | 3.0 g | 75% |
| 3.5 g | 3.9 g | 84% |
| 3.75 g | 4.3 g | 88% |
| 12.5 g | 7.21 g | 43% |
| 44.7 g | 49.2 g | 82% |

| | | |
|---|---|---|
| ^{a}crude product, only filtration | | |

### Example A.6 Synthesis of monosaccharide

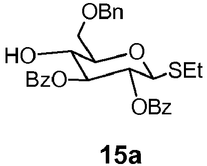

Ethyl 2,3-di-*O*-benzoyl-4,6-*O*-benzylidene-1-thiO-β-D-glucopyranoside **29a** (4 g, 7.7 mmol) was dissolved in DCM (50 mL) at 0 °C. 4Å molecular sieves were added and the resulting mixture was stirred for 10 min. Then triethylsilane (8.4 mL, 52.4 mmol) and trifluoroacetic acid (3.8 mL, 52.4 mmol) were added and the reaction mixture was stirred at room temperature for 16 h. The reaction was quenched by addition of triethylamine at 0 °C, filtered and concentrated in vacuum. The residue was purified by silica gel chromatography using ethyl acetate in n-hexane (1:3 to 1:1) affording the title compound ethyl 2,3-di-*O*-benzoyl-6-*O-*benzyl-1-thio-β-D-glucopyranoside **15a** as foamy gum (3.53 g, 88%). **¹H NMR** (400 MHz, CDCl₃) δ 8.10 - 7.79 (m, 4H), 7.55 - 7.15 (m, 11H), 5.56 - 5.31 (m, 2H), 4.76 - 4.68 (m, 1H), 4.68 - 4.57 (m, 2H), 3.98 (td, *J* = 9.3, 3.3 Hz, 1H), 3.93 - 3.80 (m, 2H), 3.72 (ddd, *J* = 9.3, 4.9, 4.2 Hz, 1H), 3.23 (d, *J* = 3.4 Hz, 1H), 2.83 - 2.64 (m, 2H), 1.27 (t, *J* = 7.5 Hz, 3H). **¹³C NMR** (101 MHz, CDCl₃) δ 167.2, 165.5, 137.8, 133.5, 133.3, 130.1 (2C), 130.0 (2C), 129.5, 129.2, 128.6, 128.5 (2C), 128.4, 128.0, 127.9, 83.1, 78.9 (2C), 73.9, 71.2, 70.4, 70.3, 24.3, 15.1.

### Synthesis of monosaccharide 15a on a larger scale:

Ethyl 2,3-di-*O*-benzoyl-4,6-*O*-benzylidene-1-thio-β-D-glucopyranoside **29a** (15 g, 28.9 mmol) was dissolved in DCM (120 mL) at 0 °C. 4Å molecular sieves were added and the resulting mixture was stirred for 10 min. Then triethylsilane (32 mL, 202 mmol) and trifluoroacetic acid (15 mL, 202 mmol) were added and the reaction mixture was stirred at room temperature for 12 h. The reaction was quenched by addition of sat. aq. NaHCO₃ solution (100 mL) and extracted with dichloromethane (3 x 50 mL). The organic layer was washed with brine (150 mL), dried over. MgSO₄ and concentrated *in vacuo.* The residue was purified by silica gel chromatography using ethyl acetate in n-hexanes (1:10 to 1:1) to afford the title compound ethyl 2,3-di-*O*-benzoyl-6-*O*-benzyl-1-thio-β-D-glucopyranoside **11a** as foamy gum (11.16 g, 74%).

The synthesis of monosaccharide **15a** was further carried out on different scales:

| **29a [g]** | **15a [g]** | **yield** |
|---|---|---|
| 1.65 g | 1.40 g | 85% |
| 4.0 g | 3.53 g | 89% |
| 5.0 g | 3.6 g | 72% |
| 10 g | 8.66 g | 86% |
| 15 g | 11.14 g | 74% |

### B. Synthesis of ST3 tetrasaccharide bearing an aminoethyl linker

### Example B.1 Synthesis of disaccharide 2a

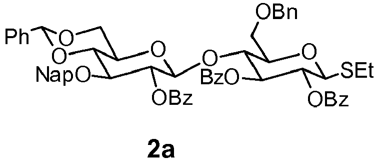

Monosaccharide **14a** (0.85 g, 1.24 mmol) and monosaccharide **15a** (0.78 g, 1.49 mmol) were dissolved in DCM (12 mL) before activated 4Å acid-washed molecular sieves were added. The resulting suspension was stirred for 30 min before cooling to -10 °C. TMSOTf (34 µL, 0.186 mmol) was added and the suspension was stirred for 1 h at -10 °C. The solution was allowed to warm to 0 °C for 30 min before the reaction was quenched by addition of Et₃N (0.5 mL). The resulting mixture was filtered and concentrated *in vacuo.* Precipitation of the pure product was achieved by addition of cold diethyl ether. Additional trituration with diethyl ether gave the title compound ethyl 2,3-di-*O*-benzoyl-4-*O*-(2-*O-*benzoyl-4,6-*O*-benzylidene-3-*O*-(2-naphthylmethyl)-β-D-gIucopyranosyl)-6-*O-*benzyl-1-thio-β-D-glucopyranoside **2a** as a pure white solid (950 mg, 75%). **¹H NMR** (400 MHz, CDCl₃) δ 7.98 (d, *J* = 7.1 Hz, 2H), 7.93 - 7.90 (m, 2H), 7.81 (d, *J* = 7.1 Hz, 2H), 7.68 (d, *J* = 7.8 Hz, 1H), 7.58 - 7.31 (m, 20H), 7.22 - 7.09 (m, 5H), 5.61 (t, *J* = 9.3 Hz, 1H), 5.38 (t, *J* = 9.7 Hz, 1H), 5.29 (s, 1H), 5.17 (dd, *J* = 9.0, 8.1 Hz, 1H), 4.88 (d, *J* = 12.4 Hz, 1H), 4.75 (d, *J* = 12.4 Hz, 1H), 4.57 - 4.50 (m, 3H), 4.25 (d, *J* = 12.2 Hz, 1H), 4.13 (t, *J* = 9.3 Hz, 1H), 3.65 (dt, *J* = 8.0, 7.1 Hz, 2H), 3.58 - 3.50 (m, 2H), 3.45 (d, *J* = 9.8 Hz, 2H), 3.17 - 3.05 (m, 1H), 2.75 - 2.60 (m, 3H), 1.20 (t, *J* = 7.5 Hz, 2H). **¹³C NMR** (101 MHz, CDCl₃) δ 165.3, 165.1, 164.6, 138.0, 137.2, 135.3, 133.2, 133.1, 133.0, 132.9, 130.2, 129.8, 129.7, 129.5, 129.3, 129.0, 128.4, 128.3 (2C), 127.9, 127.8 (2C), 127.6, 126.9, 126.1, 126.0, 125.9, 125.7, 101.2, 101.1, 83.4, 81.4, 78.7, 77.5, 75.6, 74.6, 73.7, 73.4 (2C), 70.6, 67.9, 67.4, 65.8, 24.1, 14.8. **HRMS** (ESI⁺) calculated for [M + H]⁺ 1017.3520, found 1017.3514.

### Synthesis of monosaccharide 2a on a larger scale:

Monosaccharide **14a** (1.96 g, 2.87 mmol) and monosaccharide **15a** (1.0 g, 1.91 mmol) were dissolved in DCM (12 mL) before activated 4Å acid-washed molecular sieves were added. The resulting suspension was stirred for 30 min before cooling to -30 °C. TMSOTf (69 µL, 0.383 mmol) was added and the solution was stirred for 1 h at -20 °C. The mixture was allowed to warm to 0 °C for 30 min, after which the reaction was quenched by addition of Et₃N (0.5 mL). The resulting solution was filtered and concentrated in vacuum. Purification was achieved by flash chromatography using ethyl acetate in hexanes (1:4 to 1:1), which gave the title compound ethyl 2,3-di-*O*-benzoyl-4-*O*-(2-*O*-benzoyl-4,6-*O-*benzylidene-3-*O*-(2-naphthylmethyl)-β-D-glucopyranosyl)-6-*O*-benzyl-1-thio-β-D-glucopyranoside **2a** as a white solid (570 mg, 31%).

The synthesis of disaccharide **2a** was further carried out on different scales and different conditions:

| **14a [g]** | **2a [g]** | **yield** | **activator/ catalyst** | **solvent** | **purification** |
|---|---|---|---|---|---|
| 0.60 g | 0.54 g | 61% | TMSOTf | DCM | trituration |
| 0.80 g | 0.785 g | 66% | TMSOTf | DCM | trituration |
| 0.85 g | 0.95 g | 75% | TMSOTf | DCM | trituration |
| 0.47 g | 0.512 g | 73% | TMSOTf | DCM | trituration and cc^{a} |
| 1.0 g | 0.57 g | 31% | TMSOTf | DCM | cc |
| 0.05 g | 0.041 g | 45% | TMSOTf | DCM/toluene | cc |
| 0.05 g | 0.047 g | 52% | TMSOTf | toluene | trituration |
| 0.25 g | 0.29 g | 64% | TfOH | DCM | trituration |
| 0.25 g | 0.32 g | 71% | TMSOTf | DCM | trituration |
| 2.8 g | 4.5 g | 82% | TMSOTf | toluene | trituration |
| 23.8 g | 37.07 g | 80% | TMSOTf | toluene | trituration |

| | | | | | |
|---|---|---|---|---|---|
| ^{a}cc: column chromatography | | | | | |

### Example B.2 Synthesis of disaccharide 9a

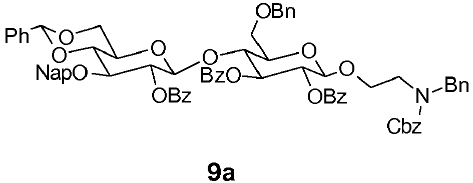

Disaccharide **2a** (150 mg, 0.147 mmol) and 2-(benzyl(benzyloxycarbonyl)amino)-ethanol (50.5 mg, 0.177 mmol) were dried azeotropically using toluene. DCM (5 mL) was added, followed by addition of activated 4Å acid-washed molecular sieves and the solution was stirred at room temperature for 30 min before cooling to -10 °C. NIS (40 mg, 0.177 mmol) and TfOH (1.3 µL, 0.015 mmol) were added and the reaction mixture was stirred between -10 °C to 0 °C for 1.5 h. The reaction mixture was quenched by addition of Et₃N (50 µL), followed by addition of 10% aq. Na₂S₂O₃ solution (10 mL). The product was extracted with DCM (3 x 10 mL) and the combined organic layers were washed with brine (10 mL). The resulting organic layer was dried over MgSO₄ and concentrated *in vacuo.* Purification was achieved by flash chromatography using ethyl acetate in hexanes (1:4 to 1:2), which gave the product 2-(benzyl(benzyloxycarbonyl)amino)-ethyl 2,3-di-*O*-benzoyl-4-*O*-(2-*O*-benzoyl-3-*O*-(2-naphthylmethyl)-4,6-*O*-benzylidene-β-D-glucopyranosyl)-6-*O*-benzyl-*β*-D-glucopyranoside **9a** as a white solid (163 mg, 89%). **[a]_{D}²⁰** = 16.3° (c = 2, CHCl₃). **IR** (thin film, cm⁻¹): vₘₐₓ: 3033, 2875, 1733, 1700, 1454, 1267, 1094, 1072. **¹H NMR** (400 MHz, CDCl₃) δ 7.86 (dd, *J* = 7.6, 5.0 Hz, 2H), 7.76 (dd, *J* = 7.8, 1.6 Hz, 1H), 7.66 - 7.39 (m, 5H), 7.53 - 7.38 (m, 12H), 7.37 - 7.21 (m, 20H), 7.14 - 7.12 (m, 2H), 5.62 (s, 1H), 5.31 (dd, *J* = 9.1, 8.0 Hz, 1H), 5.17 (d, *J* = 8.0 Hz, 2H), 5.00 (dd, *J* = 11.6, 5.0 Hz, 2H), 4.90 - 4.80 (m, 3H), 4.77 - 4.51 (m, 5H), 4.30 - 4.14 (m, 3H), 4.01 - 3.96 (m, 1H), 3.80 (dt, *J* = 36.0, 9.2 Hz, 3H), 3.64 - 3.27 (m, 9H), 3.08 (dd, *J* = 49.9, 9.8 Hz, 1H) **¹³C NMR** (101 MHz, CDCl₃) δ 164.9, 156.6, 156.3, 139.2, 138.6, 138.5, 138.1, 138.0, 137.9, 137.4, 136.7, 136.6, 135.5, 133.4, 133.2, 133.0, 129.9, 129.6, 129.2, 128.6, 128.5 (2C), 128.4, 128.3, 128.1 (2C), 128.0 (2C), 127.9 (2C), 127.7 (2C), 127.5, 127.3, 127.0, 126.2, 126.0, 125.9, 103.7, 101.4, 100.8, 82.8, 82.1, 81.8, 77.7, 76.6, 75.5, 75.0, 74.9, 74.4, 74.3, 74.0, 73.6, 68.8, 68.0, 67.6, 67.4, 67.3, 66.1, 51.6, 47.2, 46.1. **HRMS** (ESI⁺) calculated for C₇₅H₇₃NO₁₄Na [M + Na]+ 1234.4929, found 1234.4957.

The synthesis of disaccharide **9a** was also performed on a larger scale. When 11.3 g of disaccharide **2a** were employed according to the procedure described above, 9.75 g of disaccharide **9a** were obtained (93% yield).

### Example B.3 Synthesis of disaccharide 3a

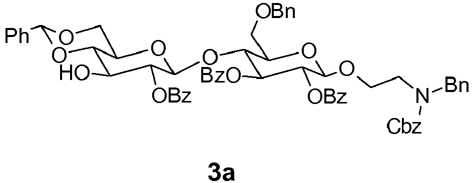

Disaccharide **9a** (155 mg, 0.13 mmol) was transferred to a stirred mixture of dichloromethane (7 mL) and water (135 µL), and subsequently cooled (5 to 10 °C) for 5 min. DDQ (128 mg, 0.56 mmol) was added slowly over a period of 2.5 h, until all of the starting disaccharide **9a** had been consumed (observed by TLC analysis). The reaction was quenched by addition of sat. aq. NaHCO₃ (5 mL) and extracted with dichloromethane (2 x 50 mL). The organic layer was dried over MgSO₄ and concentrated *in vacuo.* Purification was achieved by flash chromatography using ethyl acetate in hexanes (1:4 to 1:1), which gave the product 2-(benzyl(benzyloxycarbonyl)amino)-ethyl 2,3-di-*O*-benzoyl-4-*O*-(2-*O-*benzoyl-4,6-*O*-benzylidene-β-D-glucopyranosyl)-6-*O*-benzyl-β-D-glucopyranoside **3a** as a white solid (115 mg, 84%). **[a]_{D}²⁰** = -6.7° (c = 1, CHCl₃). **IR** (thin film, cm⁻¹): vₘₐₓ : 3424, 3033, 2881, 1732, 1700, 1455, 1269, 1094, 1056, 1029. **¹H NMR** (400 MHz, CDCl₃) δ 7.99 (t, *J* = 7.3 Hz, 2H), 7.61 (t, *J* = 7.5 Hz, 1H), 7.52 - 7.44 (m, 4H), 7.43 - 7.17 (m, 27H), 7.11 - 7.07 (m, 1H), 5.48 (s, 1H), 5.18 - 5.12 (m, 3H), 4.93 (d, *J =* 10.8 Hz, 1H), 4.84 - 4.75 (m, 3H), 4.72 - 4.63 (m, 2H), 4.59 - 4.48 (m, 2H), 4.32 (d, *J =* 12.1 Hz, 1H), 4.27 - 4.11 (m, 2H), 4.03 - 3.78 (m, 3H), 3.73 - 3.32 (m, 9H), 3.27 (td, *J* = 9.7, 5.0 Hz, 1H), 3.10 (dd, *J* = 47.0, 9.8 Hz, 1H), 2.58 (br s, 1H). **¹³C NMR** (101 MHz, CDCl₃) δ 165.6, 156.6, 156.3, 139.2, 138.6, 138.5, 138.2, 138.0, 137.9, 137.0, 136.7, 136.6, 133.6, 130.0, 129.5, 128.7, 128.6, 128.5, 128.4, 128.3, 128.1 (2C), 128.0, 127.9, 127.6 (2C), 127.5, 127.3, 126.4, 103.7, 102.0, 100.7, 82.8, 81.8, 81.1, 76.8, 75.5, 75.1, 75.0 (2C), 74.5, 74.4, 73.6, 72.6, 68.7, 68.0, 67.9, 67.5, 67.4, 66.2, 51.6, 47.2, 46.1. **HRMS** (ESI⁺) calculated for C₆₄H₆₅NO₁₄Na [M + Na]+ 1094.4303, found 1094.4311.

### Synthesis of disaccharide 3a on a larger scale:

Disaccharide **9a** (3.0 g, 2.42 mmol) was transferred to a stirred mixture of dichloromethane (100 mL) and water (2 mL), and subsequently cooled (5 to 10 °C) for 5 min. DDQ (2.47 g, 10.88 mmol) was added slowly over a period of 2.5 h, until all of the starting disaccharide **9a** had been consumed (observed by TLC analysis). The reaction was quenched by addition of sat. aq. NaHCO₃ (40 mL) and extracted with dichloromethane (2 x 25 mL). The organic layer was dried over Na₂SO₄ and concentrated *in vacuo.* Purification was achieved by flash chromatography using ethyl acetate in hexanes (1:4 to 1:1), which gave the product 2-(benzyl(benzyloxycarbonyl)amino)-ethyl 2,3-di-*O*-benzoyl-4-*O*-(2-*O-*benzoyl-4,6-*O*-benzylidene-β-D-glucopyranosyl)-6-*O*-benzyl-β-D-glucopyranoside **3a** as a white solid (2.1 g, 79%).

The synthesis of disaccharide **3a** was further carried out on different scales:

| **9a [g]** | **3a [g]** | **yield** |
|---|---|---|
| 0.155 g | 0.155 g | 84% |
| 0.161 g | 0.103 g | 72% |
| 0.25 g | 0.12 g | 55% |
| 3.0 g | 2.1 g | 79% |
| 9.35 g | 6.4 g | 77% |

### Example B.4 Synthesis of tetrasaccharide 1a

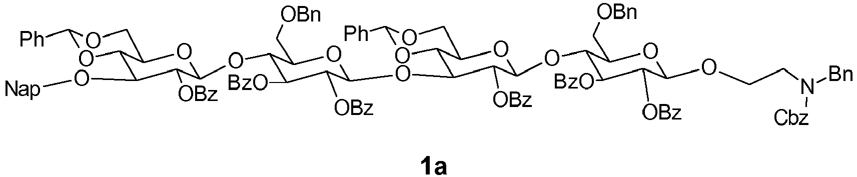

Disaccharide **3a** (110 mg, 0.10 mmol) and disaccharide **2a** (132 mg, 0.13 mmol) were dried azeotropically using toluene. DCM (4 mL) and 4Å molecular sieves were added. The solution was stirred at room temperature for 30 min and subsequently cooled to -10 °C. NIS (30 mg, 0.135 mmol) and TfOH (0.9 µL, 0.01 mmol) were added and the reaction mixture was stirred between -10 °C to 0 °C for 1.5 h. The reaction was quenched by addition of sat. aq. NaHCO₃ solution (1 mL) and filtered. To the filtrate was added 10% aq. Na₂S₂O₃ solution (20 mL) and the resulting solution was diluted with DCM, followed by extraction with DCM (3 x 15 mL). The combined organic phases were washed with sat. aq. NaHCO₃ solution (15 mL) and brine (10 mL). The organic layer was dried over MgSO₄ and concentrated *in vacuo.* Purification was achieved by flash chromatography using ethyl acetate in hexanes (1:4 to 1:1), which gave the product 2-(benzyl(benzyloxycarbonyl)amino)ethyl 2-*O*-benzoyl-4,6-*O*-benzylidene-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-4,6-*O*-benzylidene-β-D-glucopyranosyl-(1→4)-2,3-di-*O*-benzoyl-6-*O*-benzyl-β-D-glucopyranoside **1a** as a white solid (139 mg, 68%). **¹H NMR** (400 MHz, CDCl₃) δ 8.00 (d, *J* = 7.7 Hz, 1H), 7.97 - 7.82 (m, 3H), 7.70 (d, *J* = 7.7 Hz, 1H), 7.67 - 7.59 (m, 1H), 7.58 - 6.84 (m, 26H), 5.59 - 5.42 (m, 1H), 5.41 - 5.31 (m, 1H), 5.26 (s, 1H), 5.19 (d, *J* = 1.4 Hz, 1H), 5.12 (td, *J =* 14.9, 8.0 Hz, 1H), 5.02 (d, *J = 7.0* Hz, 1H), 4.95 (s, 1H), 4.87 (d, *J =* 12.4 Hz, 1H), 4.74 (d, *J* = 12.4 Hz, 1H), 4.60 (d, *J* = 8.0 Hz, 1H), 4.55 (d, *J =* 11.4 Hz, 1H), 4.47 (dd, *J* = 15.7, 7.9 Hz, 1H), 4.42 - 4.32 (m, 1H), 4.32 - 4.27 (m, 1H), 4.24 (d, *J* = 12.1 Hz, 1H), 4.15 - 4.02 (m, 1H), 3.94 (t, *J* = 9.3 Hz, 1H), 3.80 (t, *J* = 8.4 Hz, 1H), 3.71 - 3.58 (m, 1H), 3.56 - 3.36 (m, 2H), 3.33 - 3.18 (m, 2H), 3.18 - 3.02 (m, 2H), 2.69 - 2.57 (m, 1H). **¹³C NMR** (101 MHz, CDCl₃) δ 165.3, 165.0, 164.7, 164.5, 138.1, 137.2, 136.8, 135.2, 133.2, 133.0, 132.9, 132.5, 130.1, 129.9, 129.8, 129.8, 129.6, 129.2, 129.1, 128.5, 128.4, 128.3, 128.3, 128.1, 128.0, 127.8, 127.8, 127.7, 127.6, 127.2, 126.9, 126.2, 126.0, 126.0, 125.9, 125.7, 101.3, 101.1, 101.0, 101.0, 100.8, 100.1, 81.4, 79.3, 77.4, 77.2, 77.0, 76.7, 75.8, 75.4, 74.1, 73.7, 73.6, 73.3, 73.1, 72.1, 68.8, 67.7, 67.0, 66.1, 65.7, 51.5. **MALDI MS** (TOF): [M + K]⁺ calculated: 2092.7, found: 2093.1.

### Synthesis of tetrasaccharide 1a on a larger scale:

Disaccharide **3a** (1.0 g, 0.91 mmol) and disaccharide **2a** (1.12 g, 1.18 mmol) were dried azeotropically using toluene. DCM (25 mL) and 4Å molecular sieves (2.0 g) were added. The solution was stirred at room temperature for 30 min and subsequently cooled to -10 °C. NIS (0.27 g, 1.18 mmol) and TfOH (16 µL, 0.18 mmol) were added and the reaction mixture was stirred between -10 °C to 0 °C for 1.5 h. The reaction was quenched by addition of sat. aq. NaHCO₃ solution (1 mL) and filtered. To the filtrate was added 10% aq. Na₂S₂O₃ solution (20 mL) and the resulting solution was diluted with DCM, followed by extraction with DCM (3 x 20 mL). The combined organic phases were washed with sat. aq. NaHCO₃ solution (10 mL) and brine (10 mL). The organic layer was dried over Na₂SO₄ and concentrated *in vacuo.* Purification was achieved by flash chromatography using ethyl acetate in hexanes (1:9 to 1:4), which gave the product 2-(benzyl(benzyloxycarbonyl)amino)ethyl 2-*O*-benzoyl-4,6-*O*-benzylidene-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-4,6-*O*-benzylidene-β-D-glucopyranosyl-(1→4)-2,3-di-*O*-benzoyl-6-*O*-benzyl-β-D-glucopyranoside **1a** as a white solid (1.2 g, 64%).

The synthesis of tetrasaccharide **1a** was also performed with 6.5 g of disaccharide **3a.** 11.0 g of tetrasaccharide **1a** were then obtained (91% yield).

### Example B.5 Synthesis of tetrasaccharide 4a

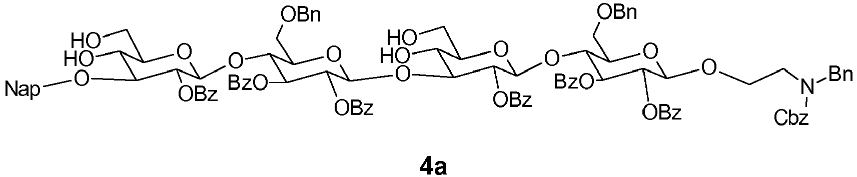

Compound **1a** (139 mg, 0.068 mmol), PTSA (1.9 mg, 0.012 mmol) and EtSH (73 µL, 1.02 mmol) were added to DCM (5 mL) and the resulting solution was stirred at room temperature for 4 h. The reaction mixture was quenched with Et₃N (50 µL) and concentrated *in vacuo.* Purification was achieved by flash chromatography using ethyl acetate in hexanes (1:5 to 100% ethyl acetate) obtaining 2-(benzyl(benzyloxycarbonyl)amino)ethyl 2-*O*-benzoyl-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-β-D-glucopyranosyl-(1→4)-2,3-di-*O*-benzoyl-6-*O-*benzyl-β-D-glucopyranoside **4a** as white foam (120 mg, 94%). **¹H NMR** (400 MHz, CDCl₃) δ 7.97 (d, *J* = 7.5 Hz, 1H), 7.93 - 7.81 (m, 3H), 7.77 - 7.70 (m, 1H), 7.70 - 7.64 (m, 1H), 7.57 (ddd, *J* = 14.0, 12.4, 7.7 Hz, 2H), 7.52 - 7.12 (m, 22H), 5.52 (t, *J* = 9.2 Hz, 1H), 5.47 - 5.32 (m, 1H), 5.27 (dd, *J* = 9.6, 7.8 Hz, 1H), 5.14 (t, 1H), 5.06 (dd, *J* = 10.5, 6.6 Hz, 1H), 5.00 (dd, *J* = 13.5, 8.1 Hz, 1H), 4.81 (d, *J* = 11.9 Hz, 1H), 4.75 (d, *J* = 7.5 Hz, 1H), 4.61 (t, *J* = 11.9 Hz, 1H), 4.51 (d, *J =* 7.8 Hz, 1H), 4.46 - 4.31 (m, 2H), 4.27 (dd, *J* = 12.8, 8.6 Hz, 1H), 4.18 (d, *J =* 11.8 Hz, 1H), 4.15 (d, *J* = 7.2 Hz, 1H), 4.10 - 4.03 (m, 1H), 4.00 (t, *J* = 9.3 Hz, 1H), 3.93 - 3.85 (m, 1H), 3.64 - 3.49 (m, 3H), 3.45 (d, *J* = 8.1 Hz, 1H), 3.36 (dd, *J* = 14.7, 6.8 Hz, 2H), 3.29 - 3.16 (m, 2H), 3.07 - 2.96 (m, 1H). **MALDI MS** (TOF): [M + K]⁺ calculated: 1916.6, found: 1917.0.

### Synthesis of tetrasaccharide 4a on a larger scale:

Compound **1a** (400 mg, 0.20 mmol), PTSA (18.7 mg, 0.09 mmol) and EtSH (210 µL, 2.92 mmol) were added to DCM (5 mL) and the resulting solution was stirred at room temperature for 4 h. The reaction mixture was quenched with Et₃N (500 µL) and concentrated *in vacuo.* Purification was achieved by flash chromatography using ethyl acetate in hexanes (1:5 to 100% ethyl acetate) obtaining 2-(benzyl(benzyloxycarbonyl)amino)ethyl 2-*O*-benzoyl-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-β-D-glucopyranosyl-(1→4)-2,3-di-*O*-benzoyl-6-*O-*benzyl-β-D-glucopyranoside **4a** as white foam (250 mg, 68%).

The synthesis of tetrasaccharide **4a** was further carried out on different scales:

| **1a[g]** | **4a[g]** | **yield** |
|---|---|---|
| 0.139 g | 0.120 g | 94% |
| 0.185 g | 0.127 g | 75% |
| 0.30 g | 0.23 g | 84% |
| 0.40 g^{a} | 0.25 g | 68% |

| | | |
|---|---|---|
| ^{a}crude | | |

### Example B.6 Synthesis of tetrasaccharide 11a

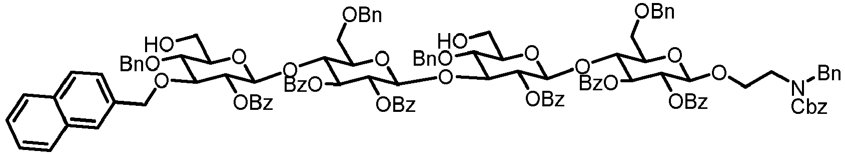

Compound **1a** (8.79 g, 4.28 mmol) in anhydrous DCM (85 mL) was stirred under nitrogen atmosphere and BH₃.THF (1M in THF, 43 mL, 43.0 mmol) was added followed by AgOTf (315 mg, 1.22 mmol) and stirred the reaction mixture at RT for 4.5 h till the reaction completion (60% EtOAc/toluene). The reaction was then quenched with methanol (17 mL) (careful: effervescence - hydrogen gas releases). Reaction mixture was stirred for additional 2 h at rt before basified with triethylamine (6 mL) and stirred for 30 min more. RM was then evaporated to minimum volume and diluted with DCM (100 mL) and water (50 mL). After layer separation the aqueous layer was extracted with DCM (25 mL X 3). Combined organic phases were then washed with dil. HCI (50 mL), sat. NaHCO₃ (50 mL X 2), brine solution (50 mL), dried over anhyd. Na₂SO₄ (∼25 g), filtered, evaporated to obtain colorless gummy liquid. Crude product was purified by silica column chromatography using acetone in toluene. Title compound **11a** was obtained as colorless solid (6.85 g, 78%). **¹H NMR** (400 MHz, CDCl₃) δ 8.13 - 6.66 (m, 67H), 5.54 - 5.24 (m, 3.5H), 5.17 - 4.95 (m, 5H), 4.88 - 4.46 (m, 7.5H), 4.46 - 4.23 (m, 6H), 4.17 - 4.01 (m, 3H), 4.00 - 3.74 (m, 2H), 3.72 - 2.96 (m, 19H). **¹³C NMR** (101 MHz, CDCl₃) δ 165.5, 165.3, 165.1, 165.0, 164.8, 163.8, 138.6, 138.1, 137.9, 135.4, 133.6, 133.2, 133.0, 132.8, 130.0, 129.8, 129.76, 129.7, 129.5, 129.4, 129.1, 128.8, 128.7, 128.6, 128.5, 128.47, 128.3, 128.2, 128.1, 128.0, 127.9, 127.7, 127.6, 127.3, 126.7, 126.0, 125.99, 125.9, 101.1, 100.7, 100.66, 100.3, 82.3, 80.2, 77.5, 76.0, 75.6, 75.5, 75.0, 74.7, 74.6, 73.7, 73.6, 73.5, 72.3, 68.8, 67.9, 67.2, 61.9, 61.3, 51.7.

### Example B.7 Synthesis of tetrasaccharide 5a

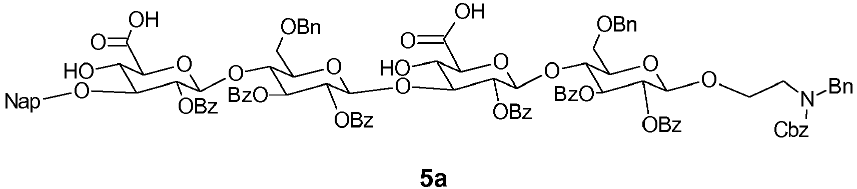

Tetrasaccharide **4a** (34 mg, 0.018 mmol) was taken in a mixture of DCM-water (0.7 mL/0.14 mL) and cooled to 0 °C. TEMPO (0.57 mg, 0.004 mmol) and BAIB (35 mg, 0.109 mmol) were added. The mixture was stirred at 0 °C for 20 min and slowly warmed to room temperature, followed by additional stirring for 4 h. The reaction mixture was then diluted with DCM (10 mL) and water (5 mL) and the aqueous layer was extracted with DCM (4 x 10 mL). The combined organic extracts were dried over MgSO₄ and concentrated *in vacuo.* The crude product was then purified by flash chromatography using DCM (100%), DCM-acetone (10:1) and finally DCM-acetone-AcOH (100:10:1) to obtain the title compound 2-(benzyl(benzyloxycarbonyl)amino)ethyl 2-*O*-benzoyl-3-*O*-(2-naphthylmethyl)-β-D-glucopyranuronosyl-(1→4)-2,3-di-*O*-benzoyl-6-*O*-benzyl-β-D-glucopyranosyl-(1→3)-2-*O*-benzoyl-β-D-glucopyranuronosyl-(1→4)-2,3-di-*O*-benzoyl-6-*O*-benzyl-β-D-glucopyranoside **5a** as an off-white solid. This compound was transferred to the next reaction without additional purification. **¹H NMR** (400 MHz, CD₃OD) δ 8.01 - 6.74 (m, 57H), 5.50 -5.36 (m, 2H), 5.18 - 5.03 (m, 2H), 5.02 - 4.83 (m, 5H), 4.71 - 4.59 (m, 3H), 4.49 - 4.45 (m, 3H), 4.41 - 4.02 (m, 7H), 3.93 - 3.35 (m, 13H), 3.26 - 3.06 (m, 3H). **MALDI MS** (TOF): [M + Na]+ calculated: 1929.946, found: 1929.967.

### Synthesis of tetrasaccharide 5a on a larger scale:

Tetrasaccharide **4a** (100 mg, 0.05 mmol) was taken in a mixture of DCM-water (3 mL/0.6 mL) and cooled to 0 °C. TEMPO (2 mg, 0.01 mmol) and BAIB (103 mg, 0.32 mmol) were added and the solution was stirred at 0 °C for 20 min and slowly warmed to room temperature, followed by additional stirring for 4 - 6 h. The reaction mixture was then diluted with DCM (5 mL) and water (5 mL) and the aqueous layer was extracted with DCM (3 x 15 mL). The combined organic phases were dried over Na₂SO₄ and concentrated in vacuum. The crude product was then purified by flash chromatography using DCM (100%), DCM-acetone-AcOH (9:1:0 to 9:1:0.2) to obtain the title compound 2-(benzyl(benzyloxycarbonyl)amino)ethyl 2-*O*-benzoyl-3-*O*-(2-naphthylmethyl)-β-D-glucopyranuronosyl-(1→4)-2,3-di-*O*-benzoyl-6-*O*-benzyl-β-D-glucopyranosyl-(1→3)-2-*O*-benzoyl-β-D-glucopyranuronosyl-(1→4)-2,3-di-*O*-benzoyl-6-*O*-benzyl-β-D-glucopyranoside **5a** as an off-white solid (63 mg, 62%).

The synthesis of tetrasaccharide **5a** was also performed with 145 mg of tetrasaccharide **4a.** 39 mg of tetrasaccharide **5a** were then obtained (62% yield).

### Example B.8 Synthesis of tetrasaccharide 12a

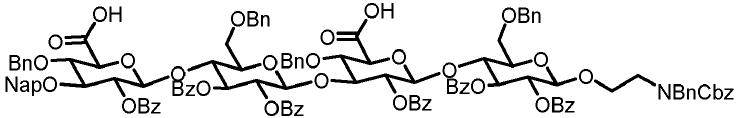

To tetrasaccharide **11a** (6.35 g, 3.08 mmol) in a mixture of DCM (125 mL) - water (25 mL) were added TEMPO (0.12 g, 0.77 mmol) and BAIB (5.96 g, 18.50 mmol) were added and the solution was stirred at 0 °C for 20 min and then at 10 °C for 2 h under nitrogen atmosphere. The reaction mixture was slowly warmed to room temperature, followed by additional stirring for 2 h. Added TEMPO (50 mg) more and continued the stirring at rt for 2 h more. Reaction was monitored by TLC (30% acetone/DCM + 2% AcOH). Reaction showed the presence of intermediate and product, so more TEMPO (70 mg) was added and continued the stirring at rt for 20 h. Reaction mixture was then diluted with DCM (50 mL) and water (50 mL). Separate the layers, and the aqueous layer was extracted with DCM (50 mL X 3). Combined organic extracts were dried over Na₂SO₄ (∼25 g), filtered, and the filtrate was concentrated under vacuum at 30-35 °C bath temperature of rotary evaporator to obtain the crude product. Purification was done on silica gel column chromatography using DCM- 10% acetone/DCM + 1% AcOH. Title Compound **12a** was obtained as an off-white solid (5.48 g, 85%). **¹H NMR** (400 MHz, CDCl₃) δ 8.08 - 6.87 (m, 67H), 5.64 - 5.56 (m, 1H), 5.50 - 5.35 (m, 1H), 5.31 - 4.86 (m, 9H), 4.81 - 4.73 (m, 3H), 4.63 - 4.09 (m, 12H), 4.02 - 3.18 (m, 17H), 3.03 - 2.81 (m, 1H).

### Example B.9 Synthesis of tetrasaccharide 7a

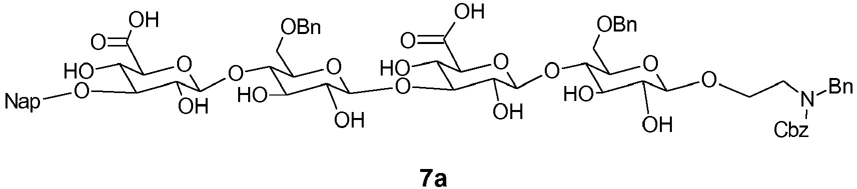

Crude compound **5a** (34.5 mg, 0.018 mmol) from the previous step and sodium methoxide (20 mg, 0.36 mmol) were dissolved in methanol (3 mL). The resulting solution was stirred for 4 d at 40 °C. The resulting solution was neutralized by addition of Amberlite® IR120 H⁺ resin, followed by stirring for 10 min. The clear suspension was filtered and washed thoroughly with methanol. The filtrate was concentrated *in vacuo* and subsequently triturated with ethyl acetate in hexanes (1:10). The title compound 2-(benzyl(benzyloxycarbonyl)amino)ethyl 3-*O*-(2-naphthylmethyl)-β-D-glucopyranuronosyl-(1→4)-6-*O*-benzyl-β-D-glucopyranosyl-(1→3)-β-D-glucopyranuronosyl-(1→4)-6-*O*-benzyl-β-D-glucopyranoside **7a** was obtained as an off-white solid (20.2 mg, 87% over two steps), which was transferred to the next step without any additional purification. **¹H NMR** (400 MHz, CD₃OD) δ 8.16 - 6.99 (m, 27H), 5.11 (d, *J* = 9.2 Hz, 2H), 5.05 (s, 2H), 4.67 - 4.32 (m, 9H), 4.20 (dd, *J* = 31.8, 8.0 Hz, 1H), 4.00 - 3.15 (m, 24H). **MALDI MS** (TOF): [M-H+Na]⁺ calculated: 1304.2898, found: 1304.764.

### Synthesis of tetrasaccharide 7a on a larger scale:

Compound **5a** (62 mg, 0.032 mmol) was taken in THF (4 mL) and 0.5 M methanolic solution of sodium methoxide (2.1 mL, 1.07 mmol) was added to it. The resulting solution was stirred at 40 °C for 16 h. The reaction mixture was evaporated in vacuum and then diluted with methanol (5 mL) and neutralized by addition of Amberlite® 120 H⁺ resin. The mixture was filtered and washed thoroughly with methanol. The filtrate was concentrated in vacuum to a pale yellow solid. The crude product was subsequently triturated with diethyl ether (3 x 2 mL). The title compound 2-(benzyl(benzyloxycarbonyl)amino)ethyl 3-*O*-(2-naphthylmethyl)-β-D-glucopyranuronosyl-(1→4)-6-*O*-benzyl-β-D-glucopyranosyl-(1→3)-β-D-glucopyranuronosyl-(1→4)-6-*O*-benzyl-β-D-glucopyranoside **7a** was obtained as an off-white solid (35 mg, 84%), which was transferred to the next step without any additional purification.

The synthesis of tetrasaccharide **7a** was also performed with 90 mg of tetrasaccharide **5a.** 55 mg of tetrasaccharide **7a** were then obtained (91% yield).

### Example B.10 Synthesis of tetrasaccharide 13a

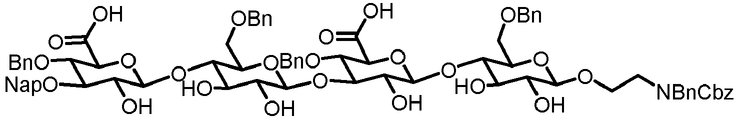

Diacid **12a** (5.15 g, 2.47 mmol) was taken in THF (100 mL) in a 500 mL RBF under nitrogen atmosphere equipped with a stir bar and stirring of 250 rpm at RT. 0.5 M methanolic solution of sodium methoxide (247 mL, 123.37 mmol) was added. The resulting solution was stirred at 50 °C for 25 h. Reaction was monitored by NMR and the TLC [(30% MeOH/acetone+∼2 drops of AcOH and 30% acetone/DCM+∼1-2% AcOH). An aliquot of the reaction mixture was worked up and analyzed by NMR]. The reaction mixture was evaporated in vacuum at 30-35 °C bath temperature of rotary evaporator for 15 min to minimum volume and then diluted with ethyl acetate (∼150 mL) and water (50 mL). The reaction mixture was acidified using 50% aq. AcOH solution (50 mL) and separated the layers. The aqueous layer was then extracted with ethyl acetate (50 mL X 3). Combined organic layer was washed with water (50 mL), brine (50 mL), dried (Na₂SO₄), filtered, and concentrated in vacuum at 30-35 °C bath temperature of rotary evaporator in a 100 mL RBF under vacuum to get yellow solid. Then -2% ethylacetate/hexanes (∼25 mL) was added to the solid in the RBF, warmed the RBF to 45 °C in the water bath and triturated, cooled to rt and filtered. The solid was washed with warm -2% ethylacetate/hexanes four more times and dried in high vacuum to afford pale yellow solid as the desired product **13a** (3.0 g, 83%). **¹H NMR** (400 MHz, Methanol-d4) δ 8.08 - 6.88 (m, 37H), 5.10 - 4.93 (m, 5H), 4.76 (d, J = 7.8 Hz, 1H), 4.75 - 4.63 (m, 2H), 4.61 - 4.54 (m, 4H), 4.51 - 4.37 (m, 5H), 4.27- 4.17 (m, 1H), 3.94 - 3.36 (m, 23H), 3.27 - 3.21 (m, 1H).

### Example B.11 Synthesis of tetrasaccharide 8a

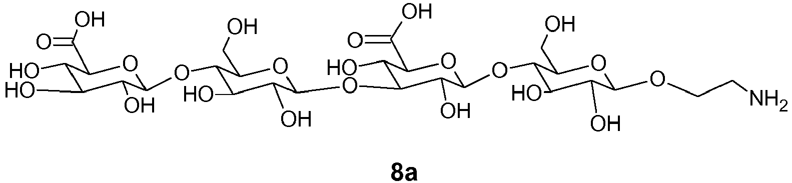

Tetrasaccharide **7a** (20 mg) was taken in a mixture of H₂O-*^{t}*BuOH-DCM (1 mL/2 mL/0.2 mL), and 20% Pd(OH)₂/C (22 mg) was added. The solution was degassed and subsequently stirred over a hydrogenous atmosphere for 24 h. The reaction mixture was then filtered through a PTFE hydrophobic filter and the filter washed thoroughly with methanol (5 x 3 mL), water-methanol (5 x 3 mL), and finally with NH₄OH in methanol (3 mL in 15 mL). After evaporation of the filtrate and drying under vacuum, the target compound 2-aminoethyl β-D-glucopyranuronosyl-(1→4)-β-D-glucopyranosyl-(1→3)-β-D-glucopyranuronosyl-(1→4)-β-D-glucopyranoside **8a** (9.2 mg, 79%) was obtained as a white material.

**¹H NMR** (400 MHz, D₂O) δ 4.84 (d, *J* = 8.0 Hz, 1H), 4.56 (d, *J* = 8.0 Hz, 2H), 4.53 (d, *J* = 7.9 Hz, 1H), 4.14 (dt, *J* = 11.5, 4.9 Hz, 1H), 4.04 - 3.92 (m, 3H), 3.89 - 3.75 (m, 5H), 3.72 - 3.49 (m, 10H), 3.43 - 3.34 (m, 3H), 3.29 (t, *J* = 5.1 Hz, 2H) **¹³C NMR** (101 MHz, D₂O) δ 175.4, 175.2, 102.3, 102.2, 102.0, 101.8, 82.7, 78.9, 78.6, 75.7, 75.65, 75.2, 74.8, 74.7, 74.1 (2C), 73.1, 72.9, 72.87, 72.7, 71.6, 70.1, 65.7, 60.0, 59.84, 39.3. **HRMS** (ESI⁺): calculated for C₂₆H₄₃NNaO₂₃ [M + Na]⁺, 760.2124, found 760.2134.

### Synthesis of tetrasaccharide 8a on a larger scale:

Tetrasaccharide **7a** (35 mg) was taken in a mixture of H₂O₋*^{t}*BuOH-DCM (0.2 mL/1 mL/0.5 mL), and a suspension of 10% Pd/C (10 mg) in *^{t}*BuOH (1 mL) was added. The reaction mixture was purged under hydrogen gas and subsequently stirred over a hydrogenous atmosphere for 24 h at rt. The reaction mixture was then filtered through a PTFE hydrophobic filter and the filter was washed thoroughly with methanol (5 x 3 mL), water-methanol (6:4, 5 x 3 mL). The filtrate was evaporated to dryness under vacuum to get an off-white solid (17 mg, 84.5% crude yield). The crude product was then purified by C₁₈ Sepak column chromatography using water and water-methanol to get the desired major product in water fractions. Lyophilization of the water fractions yielded the desired product 2-aminoethyl β-D-glucopyranuronosyl-(1→4)-β-D-glucopyranosyl-(1→)-β-D-glucopyranuronosyl-(1→4)-β-D-glucopyranoside **8a** as a white puffy solid (13 mg, 65%).

The synthesis of tetrasaccharide **8a** was also performed with 55 mg of tetrasaccharide **7a.** 19 mg of pure tetrasaccharide **8a** were then obtained (60% yield; 73% yield of the crude product).

The synthesis of tetrasaccharide **8a** was also performed with 800 mg of tetrasaccharide **13a.** 360 mg of pure tetrasaccharide **8a** were then obtained (89% yield).

### C. Synthesis of ST3 tetrasaccharide bearing an aminopentyl linker

### Example C.1 Synthesis of disaccharide 9b

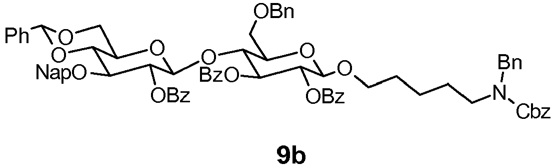

Ethyl 2,3-di-*O*-benzoyl-4-*O*-(2-*O*-benzoyl-4,6-*O*-benzylidene-3-*O*-(2-naphthylmethyl)-β-D-glucopyranosyl)-6-*O*-benzyl-1-thio-β-D-glucopyranoside **2a** (150 mg, 0.147 mmol) and protected amino alcohol HO-(CH₂)₅-N(Bn)Cbz (62.8 mg, 0.192 mmol) were dried azeotropically using toluene. Dichloromethane (5 mL) was added, followed by the addition of activated acid-washed 4Å MS and the solution was stirred at room temperature for 30 min before cooling to -10 °C. NIS (40 mg, 0.177 mmol) and TfOH (1.3 µL, 0.015 mmol) were added and the reaction mixture was stirred between -10 °C to 0 °C for 1.5 h. The reaction mixture was quenched with Et₃N (50 µL), followed by addition of 10% aq. Na₂S₂O₃ solution (10 mL). The product was extracted with DCM (3 x 10 mL) and the combined organic phases were washed with brine (10 mL). The resulting organic layer was dried over MgSO₄ and concentrated *in vacuo.* Purification was achieved by flash chromatography using ethyl acetate in hexanes (1:4 to 1:2), which gave the product 5-(benzyl(benzyloxycarbonyl)amino)-pentyl 2,3-di-*O*-benzoyl-4-*O*-(2-*O-*benzoyl-3-*O*-(2-naphthylmethyl)-4,6-*O*-benzylidene-β-D-glucopyranosyl)-6-*O-*benzyl-β-D-glucopyranoside **9b** as a white solid (189 mg, 85%). **¹H NMR** (400 MHz, CDCl₃) δ 7.98 (d, 2H), 7.86 (d, *J* = 7.6 Hz, 2H), 7.81 (d, *J* = 7.3 Hz, 2H), 7.67 (d, *J* = 7.7 Hz, 1H), 7.58 - 7.03 (m, 35H), 5.57 (t, *J* = 9.4 Hz, 1H), 5.34 - 5.24 (m, 2H), 5.16 (t, 1H), 5.11 (s, 2H), 4.87 (d, *J* = 12.4 Hz, 1H), 4.75 (d, *J* = 12.4 Hz, 1H), 4.58 - 4.40 (m, 3H), 4.34 (d, *J =* 10.4 Hz, 2H), 4.23 (d, *J =* 12.3 Hz, 1H), 4.08 (t, *J* = 9.4 Hz, 1H), 3.72 (s, 1H), 3.68 - 3.59 (m, 2H), 3.58 - 3.47 (m, 2H), 3.41 (d, *J* = 10.5 Hz, 2H), 3.36 - 3.23 (m, 1H), 3.17 - 3.05 (m, 1H), 3.05 - 2.84 (m, 2H), 2.70 (t, *J* = 10.4 Hz, 1H), 1.45 - 1.25 (m, 4H), 1.18 - 0.95 (m, 2H). **¹³C NMR** (101 MHz, CDCl₃) δ 165.1, 164.6, 137.2, 135.3, 133.2, 133.0, 132.9, 129.8, 129.8, 129.6, 129.5, 129.0, 128.4, 128.4, 128.2, 127.9, 127.8, 127.8, 127.6, 126.9, 126.1, 126.0, 125.9, 125.7, 101.2, 101.0, 100.9, 81.4, 75.8, 74.5, 73.7, 73.4, 67.0, 65.8, 50.3, 47.0, 46.1, 29.0, 28.9, 22.9. **HRMS** (ESI⁺) calculated for C₇₈H₇₆NO₁₆Na [M + Na]+ 1305.5062, found 1305.5056.

### Example C.2 Synthesis of disaccharide 3b

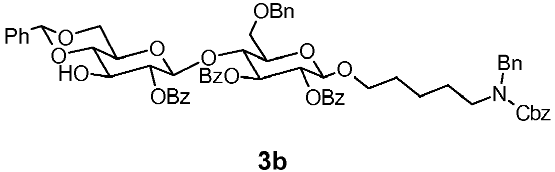

Disaccharide **9b** (145 mg, 0.113 mmol) was transferred to a stirred solution of dichloromethane (7 mL) and water (122 µL), and subsequently cooled (5 to 10 °C) for 5 min. DDQ (115 mg, 0.51 mmol) was added slowly over a period of 2.5 h, until all of the starting disaccharide **9b** had been consumed (observed by TLC analysis). The reaction was quenched with sat. aq. NaHCO₃ solution (5 mL) and extracted with dichloromethane (2 x 50 mL). The organic layer was dried over anhydrous MgSO₄ and concentrated *in vacuo.* Purification was achieved by flash chromatography using ethyl acetate in hexanes (1:4 to 1:1), which gave the product 5-(benzyl(benzyloxycarbonyl)amino)-pentyl 2,3-di-*O*-benzoyl-4-*O*-(2-*O-*benzoyl-4,6-*O*-benzylidene-β-D-glucopyranosyl)-6-*O*-benzyl-β-D-glucopyranoside **3b** as a white foam (112 mg, 87%). **¹H NMR** (400 MHz, CDCl₃) δ 7.98 (d, *J* = 8.1 Hz, 4H), 7.86 (d, *J* = 7.6 Hz, 2H), 7.65 - 7.14 (m, 29H), 5.58 (t, *J* = 9.4 Hz, 1H), 5.31 (t, 1H), 5.21 (s, 1H), 5.14 - 5.01 (m, 3H), 4.66 (d, *J* = 7.9 Hz, 1H), 4.62 (d, *J* = 12.3 Hz, 1H), 4.54 - 4.39 (m, 1H), 4.36 (d, *J* = 12.0 Hz, 3H), 4.14 (t, *J* = 9.3 Hz, 1H), 3.84 - 3.71 (m, 2H), 3.68 (dd, J = 11.1, 3.1 Hz, 1H), 3.61 (dd, *J* = 10.6, 5.0 Hz, 1H), 3.52 (d, *J* = 10.8 Hz, 1H), 3.47 - 3.39 (m, 1H), 3.31 (t, *J =* 9.3 Hz, 2H), 3.12 (td, *J* = 9.7, 5.1 Hz, 1H), 3.03 - 2.83 (m, 2H), 2.66 (t, *J =* 10.3 Hz, 1H), 1.39 - 1.24 (m, 4H), 1.13 - 0.98 (m, 2H). **¹³C NMR** (101 MHz, CDCl₃) δ 165.4, 165.1, 136.7, 133.4, 133.0, 129.9, 129.7, 129.6, 128.5, 128.5, 128.4, 128.3, 128.2, 127.9, 127.9, 127.8, 126.2, 101.6, 101.0, 100.9, 80.5, 75.9, 74.7, 74.6, 73.5, 71.8, 67.1, 65.8, 50.0, 45.3, 28.8, 22.8. **HRMS** (ESI⁺) calculated for C₆₇H₆₇NO₁₆Na [M + Na]+ 1164.4358, found 1164.4353.

### Example C.3 Synthesis of tetrasaccharide 4b

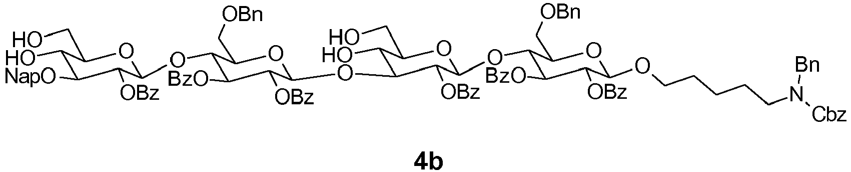

a) Disaccharide **3b** (129 mg, 0.113 mmol) and donor disaccharide **2b** (149 mg, 0.147 mmol) were dried azeotropically using toluene. Dichloromethane (3 mL) and 4Å MS were added and the mixture was stirred at room temperature for 30 min and subsequently cooled to -10 °C. NIS (34 mg, 0.152 mmol) and TfOH (0.9 µL, 0.01 mmol) were added and the reaction mixture was stirred between -10 °C to 0 °C for 1.5 h. The reaction was quenched by addition of sat. aq. NaHCO₃ solution (1 mL) and filtered. To the filtrate was added 10% aq. Na₂S₂O₃ solution (20 mL) and the resulting solution was diluted with dichloromethane, followed by extraction with dichloromethane (3 x 15 mL). The combined organic phases were washed with sat. aq. NaHCO₃ solution (15 mL) and brine (10 mL). The organic layer was dried over MgSO₄ and concentrated *in vacuo.*
b) The subsequent crude solid (containing **1b)** was transferred to a stirred solution of PTSA (3.7 mg, 0.02 mmol) and EtSH (141 µL, 1.95 mmol) in dichloromethane (3.5 mL) and the resulting solution was stirred at room temperature for 3 h. The reaction mixture was quenched with Et₃N (50 µL) and concentrated *in vacuo.* Purification was achieved by flash chromatography using ethyl acetate in hexanes (1:5 to 100% ethyl acetate) obtaining the tetrasaccharide product 5-(benzyl(benzyloxycarbonyl)amino)-pentyl 2-*O*-benzoyl-3-*O*-(2-naphthylmethyl)-β-D-glucopyranosyl-(1→4)-2,3-di-*O*-benzoyl-6-*O*-benzyl-β-D-glucopyranosyl-(1→3)-2-*O*-benzoyl-β-D-glucopyranosyl-(1→4)-2,3-di-*O*-benzoyl-6-*O*-benzyl-β-D-glucopyranoside **4b** as white foam (161 mg, 64% over two steps). **¹H NMR** (400 MHz, CDCl₃) δ 7.95 (d, *J* = 7.2 Hz, 2H), 7.85 (dd, *J* = 13.4, 7.3 Hz, 6H), 7.73 - 7.17 (m, 49H), 5.54 - 5.39 (m, 2H), 5.33 - 5.21 (m, 2H), 5.14 - 5.05 (m, 3H), 4.96 (t, *J =* 8.5 Hz, 1H), 4.80 - 4.69 (m, 2H), 4.58 (d, *J =* 12.3 Hz, 1H), 4.49 (dd, *J* = 7.6, 5.9 Hz, 2H), 4.40 - 4.29 (m, 5H), 4.28 - 4.20 (m, 1H), 4.16 (d, *J* = 11.7 Hz, 1H), 4.01 (dt, *J* = 27.9, 9.2 Hz, 3H), 3.93 - 3.83 (m, 1H), 3.76 - 3.61 (m, 1H), 3.60 - 3.51 (m, 2H), 3.48 (t, *J* = 7.9 Hz, 2H), 3.46 - 3.37 (m, 3H), 3.34 (dd, *J* = 15.4, 6.8 Hz, 3H), 3.28 - 3.12 (m, 4H), 3.07 - 2.94 (m, 4H), 2.94 - 2.87 (m, 2H), 1.37 - 1.21 (m, 4H), 1.14 - 0.98 (m, 2H). **¹³C NMR** (101 MHz, CDCl₃) δ 165.1 (2C), 165.0, 164.9, 164.6, 163.7, 137.93, 137.2, 135.2, 133.4, 133.0, 133.0, 132.9, 132.8, 132.5, 130.0, 129.6, 129.59, 129.5, 129.4, 129.3, 128.8, 128.7, 128.5, 128.4, 128.3, 128.2, 128.2, 128.1, 128.0, 127.9, 127.9, 127.8, 127.6, 127.2, 126.6, 126.1, 125.9, 125.6, 100.9 (2C), 100.1, 84.9, 82.0, 77.2, 75.7, 75.5, 75.3, 74.8, 74.50, 74.5, 74.1, 73.6, 73.4, 73.2, 72.0, 71.7, 71.5, 70.2, 69.2, 67.0, 62.3, 61.6, 50.5, 47.0, 46.0, 28.9, 28.8, 22.9. **HRMS** (ESI⁺) calculated for C₁₁₁H₁₀₉NO₂₉Na [M + Na]+ 1942.6983, found 1942.7086.

### Example C.4 Synthesis of tetrasaccharide

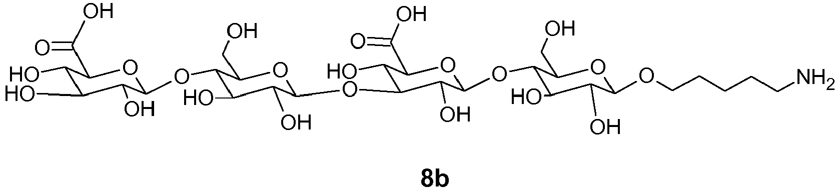

a) Tetrasaccharide **4b** (160 mg, 0.083 mmol) was taken in a mixture of dichloromethane (2.5 mL) and water (0.5 mL) and cooled to 0 °C. TEMPO (2.6 mg, 0.017 mmol) and BAIB (188 mg, 0.583 mmol) were added and the mixture was stirred at 0 °C for 20 min and slowly warmed to room temperature, followed by additional stirring for 5 h. The reaction mixture was then diluted with dichloromethane (10 mL) and water (5 mL) and the aqueous layer was extracted with dichloromethane (4 x 10 mL). The combined organic extracts were dried over Mg₂SO₄ and concentrated *in vacuo.* The crude product was then purified by flash chromatography using dichloromethane (100%), dichloromethane/acetone (10:1) and finally dichloromethane/acetone/AcOH (100:10:1) to obtain 5-(benzyl(benzyloxycarbonyl)amino)pentyl 2-*O*-benzoyl-3-*O*-(2-naphthylmethyl)-β-D-glucopyranuronosyl-(1→4)-2,3-di-*O*-benzoyl-6-*O*-benzyl-β-D-glucopyranosyl-(1→3)-2-*O*-benzoyl-β-D-glucopyranuronosyl-(1→4)-2,3-di-*O*-benzoyl-6-*O*-benzyl-β-D-glucopyranoside **5b** as an off-white solid. This diacid **5b** was transferred to the next reaction without additional purification, assuming total conversion to the desired product (100% yield).
b) Tetrasaccharide **5b** (143 mg, 0.073 mmol) was taken in 0.5 M methanolic solution of sodium methoxide (20 mL, 1.07 mmol) and dichloromethane (1-4 mL) and the resulting solution was stirred at 50 °C for 4 days. The reaction mixture was evaporated in vacuum and then diluted with methanol (5 mL) and neutralized by addition of Amberlite® IR120 H⁺ resin. The clear suspension was filtered and washed thoroughly with methanol. The filtrate was concentrated in vacuum. The remaining colorless solid 5-(benzyl(benzyloxycarbonyl)amino)-pentyl 3-*O*-(2-naphthylmethyl)-β-D-glucopyranuronosyl-(1→4)-6-*O*-benzyl-β-D-glucopyranosyl-(1→3)-β-D-glucopyranuronosyl-(1→4)-6-*O*-benzyl-β-D-glucopyranoside **7b** was transferred to the next step without additional purification.
c) Tetrasaccharide **7b** (97 mg, 0.073 mmol) was taken in a mixture of H₂O (2 mL), *^{t}*BuOH (4 mL) and dichloromethane (0.3 mL), and added to 20% Pd/C (97 mg). The solution was degassed under reduced pressure and subsequently stirred over a hydrogenous atmosphere for 24 h. The reaction mixture was then filtered through a PTFE hydrophobic filter and the filter washed thoroughly with water and methanol. After evaporation of the filtrate and drying under vacuum, target compound 5-aminopentyl β-D-glucopyranuronosyl-(1→4)-β-D-glucopyranosyl-(1→3)-β-D-glucopyranuronosyl-(1→4)-β-D-glucopyranoside **8b** was obtained as an off-white solid (35 mg, 61% over three steps). **¹H NMR** (600 MHz, D₂O) δ 4.49 - 4.30 (m, 3H), 3.98 (dd, *J* = 12.5, 9.9 Hz, 1H), 3.88 - 3.76 (m, 5H), 3.73 - 3.64 (m, 6H), 3.63 - 3.33 (m, 16H), 3.29 - 3.21 (m, 3H), 3.16 (t, *J* = 7.4 Hz, 1H), 2.88 (t, *J =* 7.5 Hz, 2H), 1.61 - 1.50 (m, 4H), 1.38 - 1.29 (m, 2H). **HRMS** (ESI⁻) calculated for C₂₉H₄₈NO₂₉ [M - H]⁻ 778.2617, found 778.2672.

### D. Synthesis of ST3 tetrasaccharide bearing an azidoethyl linker

### Example D.1 Synthesis of disaccharide 9c

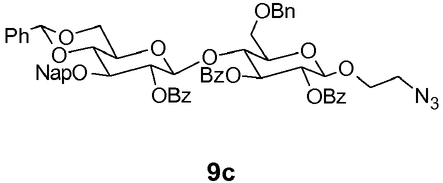

Ethyl 2-*O*-benzoyl-4,6-*O*-benzylidene-3-*O*-(2-naphthylmethyl)-1-thio-β-D-glucopyranoside **18a** (2.6g, 4.67 mmol) and 1-azidoethyl 2,3-di-*O*-benzoyl-4,6-*O-*benzylidene-1-β-D-glucopyranoside (3.07g, 5.60 mmol) were dissolved in DCM (60mL) at room temperature for 5 min, and then cooled to 0 °C (ice-water mixture). NIS (1.26 g, 5.60 mmol) and Triflic acid (0.1141 mL, 5.60 mmol) were added to the reaction mixture at 0 °C. After 30 min the reaction was completed. TLC analysis (n-Hex/EtOAc=1:1) showed the disappearance of the donor spot and the presence of a new spot. The reaction was quenched by addition of sat. NaHCO₃ solution (50 mL) and sat. Na₂S₂O₃ solution (100 mL). The crude compound was extracted with DCM (20 mL X 2) and washed with sat. Na₂S₂O₃ solution (100 mL X 2), sat. NaHCO₃ solution (100 mL X 2), and brine (100 mL X 2). After separation, organic layer was dried over anhydrous MgSO₄ (∼20 g), filtered, and concentrated under reduced pressure to obtain the crude. The crude organic product was purified through a short silica gel chromatography (column ø 40 X 450 mm) using n-Hex/EtOAc gradient system. The title compound **9c** was obtained (5.58g, 66 %) as a white brownish solid. **HRMS** (ESI⁺) calculated for C₆₀H₅₅O₁₄N₃Na⁺ [M + Na]⁺ 1064.3582, found 1064.3695.

### Alternative synthesis of disaccharide 9c

Disaccharide **2a** (0.9g, 0.88 mmol) and chloroethanol (0.14g mg, 01.77 mmol) were dissolved in DCM (4.5 mL) in a 50 mL RBF at room temperature for 5 min, and then cooled to 0 °C (ice-water mixture in isotherm). NIS (0.24 mg, 1.06 mmol) and Triflic acid (22 uL, 0.18 mmol) were added to the reaction mixture at 0 °C. After 30 min the reaction was completed. The reaction was quenched by the addition of sat. NaHCO₃ solution (20 mL) and sat. Na₂S₂O₃ solution (50 mL). The crude compound was extracted into DCM (20 mL X 2) and washed with sat. Na₂S₂O₃ solution (40 mL X 2), sat. NaHCO₃ solution (40 mL X 2), and brine (50 mL X 2). After separation, organic layer was dried over anhydrous MgSO₄ (∼20 g), filtered, and concentration at 30-35 °C bath temperature of rotary evaporator in a 250 mL RBF for 30-45 min to obtain the crude. The crude organic product was purified by through a short silica gel chromatography using n-Hex/EtOAc Gradient system. The fraction containing product was concentrated in vacuum and then was dried for 16 h to afford 0.8 g (87%) chlorinated intermediate as a white solid. HRMS (ESI⁺) Calcd for C₆₀H₅₅ClO₁₄Na⁺ [M+Na]+ 1057.3178, found 1057.3191.

The chlorinated intermediate 0.76 g (0.24 mmol) was dissolved in anhyd. DMF (8 mL), sodium azide (0.10 g, 1.48 mmol) and 18-Crown-6 (0.2130g, 0.81 mmol) were added. The reaction mixture was heated to 60 °C for overnight. After completing of the reaction it was cooled to room temperature and diluted with water (30 mL). The mixture was extracted with DCM, washed with brine and dried over MgSO₄. The organic layer was concentrated in vacuum and then the residue was purified by silica gel column chromatography (Hex/EtOAc: Gradient). The product was dried under high vacuum for 16 h to afford **9c** (0.61 g, 80%) as a white solid.

### Example D.2 Synthesis of disaccharide 3c

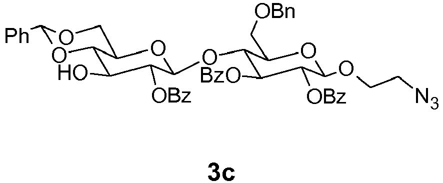

Disaccharide **9c** (0.5 g, 0.48 mmol) was transferred to a stirring solution of DCM (17 mL) and water (0.3 mL), and subsequently cooled (5 to 10 °C) for 5 min under nitrogen atmosphere. DDQ (0.49 g, 2.15 mmol) was added slowly over a period of 2.5 h, TLC analysis (40% EtOAc/hexanes) showed the absence of the starting material and presence of product after 4 h. The reaction was quenched by the addition of sat. aq. NaHCO₃ (40 mL) and extracted into DCM (25 mL X 3). The combined organic layers were washed with sat. NaHCO₃ solution (10 mL), brine (50 mL), dried over anhyd. MgSO₄ (∼50 g), filtered and concentrated under reduced pressure to obtain the crude as a pale yellow oil. Purification was done on silica gel column flash chromatography (12g column) using ethyl acetate in hexanes. The title compound **3c** was obtained as a fluffy white solid (0.32g, 75%). **HRMS** (ESI⁺) calculated for C₄₉H₄₇O₁₄N₃Na⁺ [M + Na]⁺ 924.2956, found 924.2997.

### Example D.3 Synthesis of tetrasaccharide 1c

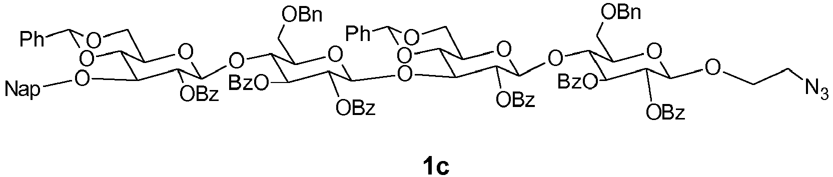

Disaccharide **2a** (0.21 g, 0.20 mmol) and disaccharide **3c** (0.14 g, 0.16 mmol) were taken in 50 mL anhydrous toluene and evaporated under vacuum for 30 min to dryness and repeated this azeotropic drying process two times more. The material was dried under high vacuum for 12h. Then anhyd. DCM (6mL) was added under Argon atmosphere and stirred at 300 rpm. About 400mg dried 4 A molecular sieves (MS) were added and stirred at room temperature for 45 min and then cooled to -5°C (ice-acetone mixture). NIS (0.045g, 0.20 mmol) and Triflic acid (3 µmL, 0.03 mmol) were added to the reaction mixture and stirred for 15 min. Then it was slowly warmed to 10 °C for 0.5 h and then stirred at RT for 1 h. TLC analysis (40% ethyl acetate in n-hexane)) showed the disappearance of the disaccharide **2a** and the presence of a new spot. The reaction was quenched at 5 °C by the addition of sat. NaHCO₃ solution (10 mL) and sat. Na₂S₂O₃ solution (20 mL). The crude compound was extracted with DCM (25 mL X 2) and washed with sat. Na₂S₂O₃ solution (50 mL X 2), sat. NaHCO₃ solution (50 mL X 2), and brine (50 mL X 2). After separation, organic layer was dried over anhydrous MgSO₄ (∼5 g), filtered, and concentrated under reduced pressure to obtain the crude. The crude organic product was purified by flash chromatography (column 12g column) using n-Hex/EtOAc gradient system. The title compound **1c** was obtained (0.1447g, 50 %) as a white solid. **HRMS** (ESI⁺) calculated for C₁₀₇H₉₇O₂₇N₃Na⁺ [M + Na]⁺ 1878.6207 found 1878.6294.

### Example D.4 Synthesis of tetrasaccharide 1g

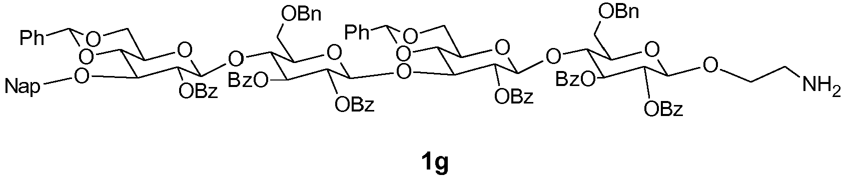

Tetrasaccharide **1c** (0.1 g, 0.05 mmol) was dissolved in anhydrous DCM (3mL). Acetic acid (150µl) and zinc (106mg, 1.62mmol) were added. The mixture was stirred at room temperature for 1 hour. The reaction was filtered through a celite pad (1cm). The pad was washed with DCM (30mL) and the filtrate was concentrated under reduced pressure.
Purification was done by flash silica gel column chromatography (DCM/MeOH). Fraction containing pure product was collected, concentrated under vacuum and dried under high vacuum to afford **1g** (98mg, 100%).
HRMS (ESI⁺) Calcd for C₁₀₇H₉₉O₂₇NH⁺ [M+H]⁺ 1830.6483, found 1830.6445.

### Example D.4 Synthesis of tetrasaccharide 1h

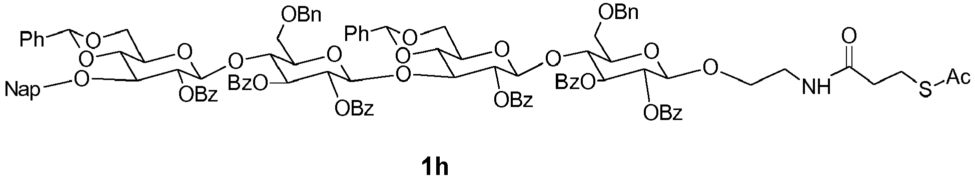

Tetrasaccharide **1g** (0.04 g, 0.02 mmol) and N-succinimidyl 3-(acetylthio)-propionate (0.107 g, 0.44 mmol) were dissolved in anhydrous DCM (3mL) and triethylamine (0.1 mL) was added. The mixture was stirred at room temperature for 3 hour. Purification was done by flash silica gel column chromatography (DCM/MeOH). The fraction containing the desired product was collected, concentrated under reduced pressure and dried under high vacuum to afford **1h** (29 mg, 65%).
HRMS (ESI⁺) Calcd for C₁₁₂H₁₀₅O₂₉NSNa⁺ [M+Na]⁺ 1982.6391, found 1982.6362.

### E. Synthesis of ST3 tetrasaccharide bearing an azidodecyl linker

### Example E.1 Synthesis of disaccharide 9d

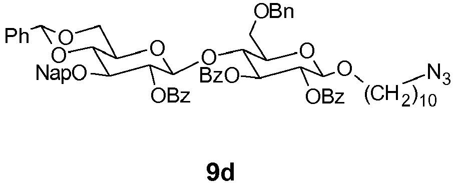

Ethyl 2-*O*-benzoyl-4,6-*O*-benzylidene-3-*O*-(2-naphthylmethyl)-1-thio-β-D-glucopyranoside **18a** (0.25 g, 0.25 mmol) and 10-azido-1-decanole (0.098 g, 0.50 mmol) were dissolved in DCM (0.5 mL) in at room temperature for 5 min, and then cooled to 0 °C (ice-water mixture). NIS (67 mg, 0.29 mmol) and triflic acid (6 µL, 0.05 mmol) were added to the reaction mixture at 0 °C. After 30 min the reaction was completed. TLC analysis (n-Hex/EtOAc=2:1) showed the disappearance of the donor spot and the presence of a new spot. The reaction was quenched by the addition of sat. NaHCO₃ solution (20 mL) and sat. Na₂S₂O₃ solution (50 mL). The crude compound was extracted with DCM (20 mL X 2) and washed with sat. Na₂S₂O₃ solution (20 mL X 2), sat. NaHCO₃ solution (20 mL X 2), and brine (30 mL X 2). After separation, organic layer was dried over anhydrous MgSO₄ (∼5 g), filtered, and concentrated under reduced pressure to obtain the crude. The crude organic product was purified by column chromatography on silica using n-Hex/EtOAc Gradient system. The title compound **9d** was obtained (0.2064 g, 73%) as a white solid. **HRMS** (ESI⁺) calculated for C₆₈H₇₁O₁₄N₃Na⁺ [M + Na]⁺ 1176.4834, found 1176.4812.

### Example E.2 Synthesis of disaccharide 3d

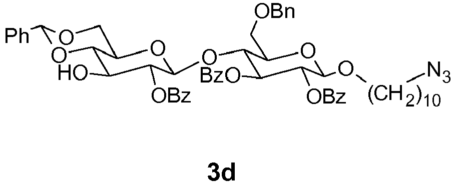

Disaccharide **9d** (0.2g, 0.19 mmol) was transferred to a stirring solution of DCM (6 mL) and water (0.12 mL), and subsequently cooled (5 to 10 °C) for 5 min under nitrogen atmosphere and stirred at 400 rpm. DDQ (0.2 g, 0.86 mmol) was added slowly over a period of 2.5 h, TLC analysis (40%EtOAC/Hexanes) showed the presence of a new spot slightly polar to the major amount of starting material even after 2 h, so stirred the reaction mixture for additional 2 h at RT. TLC analysis (40% EtOAc/hexanes) showed the absence of the starting material and presence of product after 4 h. The reaction was quenched by the addition of sat. aq. NaHCO₃ (40 mL) and extracted into DCM (25 mL X 3). The combined organic layers were washed with sat. NaHCO₃ solution (10 mL), brine (50 mL), dried over anhyd. MgSO₄ (∼5 g), filtered and concentrated under vacuum to obtain the crude product as a pale yellow oil. Purification was done by flash chromatography using ethyl acetate in hexanes. The title compound **3d** was obtained as a fluffy white solid (0.11g, 57%). **HRMS** (ESI⁺) calculated for C₅₇H₆₃O₁₄N₃Na⁺ [M + Na]⁺ 1036.4208, found 1036.4194.

### Example E.3 Synthesis of tetrasaccharide 1d

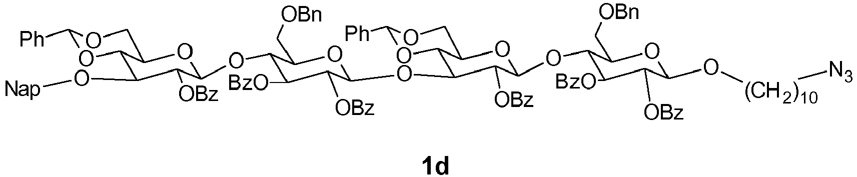

Disaccharide **2a** (0.130 g, 0.13 mmol) and disaccharide **3d** (0.1g, 0.099 mmol) were taken in anhydrous toluene (10mL) and evaporated under vacuum for 30 min to dryness and repeated this azeotropic drying process two times more. The material was dried under high vacuum for 12h. Then anhyd. DCM (2.5mL) was added under Argon atmosphere and stirred at 300 rpm. About 1 g dried 4 A molecular sieves (MS) were added and stirred at room temperature for 45 min and then cooled to -5°C (ice-acetone mixture). NIS (0.029 g, 0.13 mmol) and triflic acid (2 µL, 0.02 mmol) were added to the reaction mixture and stirred for 30 min. Then it was slowly warmed to 0°C for 0.5 h, 10°C for 30 min and then stirred at RT for 1h. TLC analysis (40% ethyl acetate in n-Hexane)) showed the disappearance of the donor spot and the presence of a new spot. The reaction was quenched at 5 °C by the addition of sat. NaHCO₃ solution (10 mL) and sat. Na₂S₂O₃ solution (20 mL). The crude compound was extracted with DCM (20 mL X 2) and washed with sat. Na₂S₂O₃ solution (20 mL X 2), sat. NaHCO₃ solution (20 mL X 2), and brine (20 mL X 2). After separation, organic layer was dried over anhydrous MgSO₄ (∼5 g), filtered, and concentrated under reduced pressure to obtain the crude product. The crude organic product was purified by flash chromatography (12g silica) using n-Hex/EtOAc (0.1104 mg, 59%). **HRMS** (ESI⁺) calculated for C₁₁₅H₁₁₃O₂₇N₃Na⁺ [M + Na]⁺ 1990.7459, found 1990.7459. 1H NMR (400 MHz, Chloroform-d) δ 8.06 - 6.83 (m, 57H), 5.51 (dd, J = 9.8, 9.0 Hz, 1H), 5.37 - 5.21 (m, 3H), 5.19 - 5.01 (m, 3H), 4.93 (s, 1H), 4.88 - 4.66 (m, 2H), 4.64 - 4.40 (m, 5H), 4.23 (dd, J = 12.2, 9.0 Hz, 2H), 4.14 - 3.99 (m, 2H), 3.92 (t, J = 9.4 Hz, 1H), 3.86 - 3.71 (m, 2H), 3.69 - 3.39 (m, 5H), 3.40 - 3.18 (m, 5H), 3.17 - 2.97 (m, 4H), 2.73 - 2.50 (m, 2H), 1.54 (s, 6H), 1.47 - 0.85 (m, 12H).

### F. Synthesis of ST3 tetrasaccharide bearing an 2-(2-azidoethoxy)ethyl linker

### Example F.1 Synthesis of disaccharide 9e

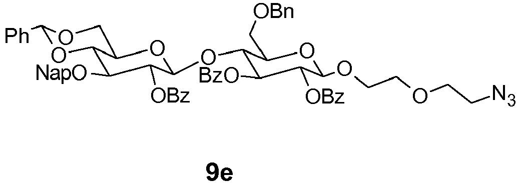

Ethyl 2-*O*-benzoyl-4,6-*O*-benzylidene-3-*O*-(2-naphthylmethyl)-1-thio-β-D-glucopyranoside **18a** (0.25g, 0.25 mmol) and 2-(2-azidoethoxy)ethanol (0.07 g, 0.50 mmol) were dissolved in DCM (0.5 mL) at room temperature for 5 min, and then cooled to 0 °C (ice-water mixture). NIS (67 mg, 0.29 mmol) and triflic acid (6 µL, 0.05 mmol) were added to the reaction mixture at 0 °C. After 30 min the reaction was completed. TLC analysis (n-Hex/EtOAc=2:1) showed the disappearance of the donor spot and the presence of a new spot. The reaction was quenched by the addition of sat. NaHCO₃ solution (20 mL) and sat. Na₂S₂O₃ solution (50 mL). The crude compound was extracted with DCM (20 mL X 2) and washed with sat. Na₂S₂O₃ solution (20 mL X 2), sat. NaHCO₃ solution (20 mL X 2), and brine (30 mL X 2). After separation, organic layer was dried over anhydrous MgSO₄ (∼5 g), filtered, and concentrated under reduced pressure to obtain the crude product. The crude organic product was purified by column chromatography (12g column) using n-Hex/EtOAc gradient system. The title compound **9e** was obtained (0.1925 g, 72%) as a white solid. **HRMS** (ESI⁺) calculated for C₆₂H₅₉O₁₅N₃Na⁺ [M + Na]⁺ 1108.3844, found 1108.3839.

### Example F.2 Synthesis of disaccharide 3e

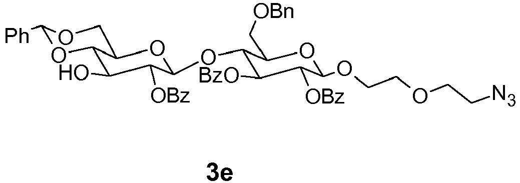

Disaccharide **9e** (0.18 g, 0.17 mmol) was transferred to a stirring solution of DCM (6 mL) and water (0.12 mL), and subsequently cooled (5 to 10 °C) for 5 min under nitrogen atmosphere and stirred at 400 rpm. DDQ (0.17 g, 0.75 mmol) was added slowly over a period of 2.5 h. TLC analysis (40% EtOAc/hexanes) showed the absence of the starting material and presence of product after 4 h. The reaction was quenched by the addition of sat. aq. NaHCO₃ (40 mL) and extracted with DCM (25 mL X 3). The combined organic layers were washed with sat. NaHCO₃ solution (10 mL), brine (50 mL), dried over anhyd. MgSO₄ (∼50 g), filtered and concentrated under vacuum to obtain the crude as a pale yellow oil. Purification was done by flash chromatography using ethyl acetate in n-hexanes. The title compound **3e** was obtained as a fluffy white solid (0.102g, 65%). **HRMS** (ESI⁺) calculated for C₅₁H₅₁O₁₅N₃Na⁺ [M + Na]⁺ 968.3218, found 968.3218.

### Example F.3 Synthesis of tetrasaccharide 1e

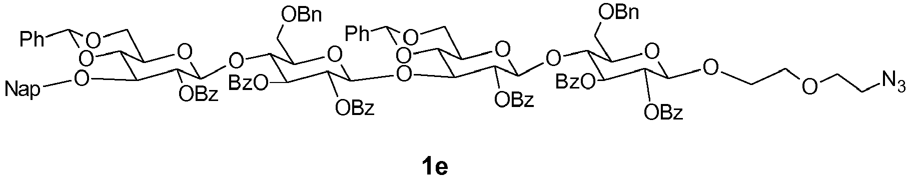

Disaccharide **2a** (0.126g, 0.12 mmol) and disaccharide **3e** (0.09g, 0.095mmol) were taken in anhydrous toluene (10mL) and evaporated under for 30 min to dryness and repeated this azeotropic drying process two times more. The material was dried under high vacuum for 12h. Then anhyd. DCM (2 mL) was under Argon atmosphere and stirred at 300 rpm. About 1 g dried 4 A molecular sieves (MS) were added and stirred at room temperature for 45 min and then cooled to -5°C (ice-acetone mixture). NIS (0.028g, 0.12 mmol) and triflic acid (2 µL, 0.02 mmol) were added to the reaction mixture and stirred for 30 min. Then it was slowly warmed to 0°C for 0.5 h, 10°C for 30 min and then stirred at RT for 1h. TLC analysis (40% ethyl acetate in n-hexane) showed the disappearance of the donor spot and the presence of a new spot. The reaction was quenched at 5°C by the addition of sat. NaHCO₃ solution (10 mL) and sat. Na₂S₂O₃ solution (20 mL). The crude compound was extracted with DCM (20 mL X 2) and washed with sat. Na₂S₂O₃ solution (20 mL X 2), sat. NaHCO₃ solution (20 mL X 2), and brine (20 mL X 2). After separation, organic layer was dried over anhydrous MgSO₄ (∼5 g), filtered, and concentrated under reduced to obtain the crude product. The crude organic product was purified by flash chromatography (column 12g column) using n-Hex/EtOAc gradient system (64.2mg, 36%). **HRMS** (ESI⁺) calculated for C₁₀₉H₁₀₁O₂₈N₃Na⁺ [M + Na]⁺ 1922.6469, found 1922.6470. 1H NMR (400 MHz, Chloroform-d) δ 8.07 - 6.86 (m, 57H), 5.52 (t, J = 9.4 Hz, 1H), 5.37 - 5.26 (m, 2H), 5.23 (s, 1H), 5.20 - 5.00 (m, 3H), 4.93 (s, 1H), 4.88 - 4.66 (m, 2H), 4.63 - 4.42 (m, 5H), 4.22 (dd, J = 12.1, 7.8 Hz, 2H), 4.15 - 4.01 (m, 2H), 3.97 - 3.73 (m, 3H), 3.71 - 3.19 (m, 13H), 3.18 - 2.93 (m, 7H), 2.63 (dt, J = 20.5, 10.4 Hz, 2H).

### G. Synthesis of ST3 tetrasaccharide

### Example G.1 Synthesis of disaccharide 9f'

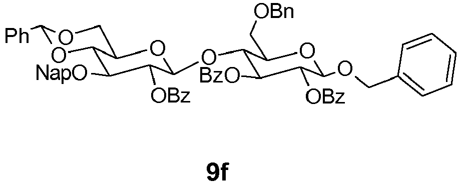

Ethyl 2-*O*-benzoyl-4,6-*O*-benzylidene-3-*O*-(2-naphthylmethyl)-1-thio-β-D-glucopyranoside **18a** (1g, 9.83 mmol) and anhydr. benzyl alcohol (0.20 mL, 1.2 mmol) were dissolved in DCM (5 mL) at room temperature for 5 min, and then cooled to 0 °C (ice-water mixture). NIS (0.27 mg, 1.18 mmol) and triflic acid (24 µL, 0.20 mmol) were added to the reaction mixture at 0 °C. After 30 min the reaction was completed. TLC analysis (n-Hex/EtOAc=2:1) showed the disappearance of the donor spot and the presence of a new spot. The reaction was quenched by the addition of sat. NaHCO₃ solution (20 mL) and sat. Na₂S₂O₃ solution (50 mL). The crude compound was extracted with DCM (20 mL X 2) and washed with sat. Na₂S₂O₃ solution (40 mL X 2), sat. NaHCO₃ solution (40 mL X 2), and brine (50 mL X 2). After separation, organic layer was dried over anhydrous MgSO₄ (∼20 g), filtered, and concentrated under reduced pressure to obtain the crude product . The crude organic product was purified twice by Büchi Combiflash system using n-Hex/EtOAc Gradient system. The title compound **9f** was obtained (0.8414 g, 80%) as a white solid. **HRMS** (ESI⁺) calculated for C₆₅H₅₈O₁₄Na⁺ [M + Na]⁺ 1085.3724 found 1085.3747.

### Example G.2 Synthesis of disaccharide 3f

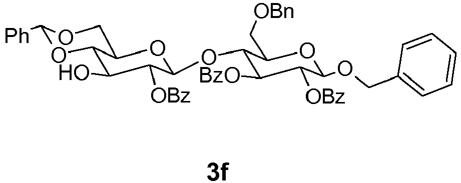

Disaccharide **3f** (0.245 g, 0.23 mmol) was transferred to a stirring solution of DCM (9 mL) and water (0.15 mL), and subsequently cooled (5 to 10 °C) for 5 min under nitrogen atmosphere and stirred at 400 rpm. DDQ (0.24 g, 1.04 mmol) was added slowly over a period of 2.5 h. TLC analysis (40% EtOAc/hexanes) showed the absence of the starting material and presence of product after 4 h. The reaction was quenched by the addition of sat. aq. NaHCO₃ (40 mL) and extracted with DCM (25 mL X 3). The combined organic layers were washed with sat. NaHCO₃ solution (10 mL), brine (50 mL), dried over anhyd. MgSO₄ (∼5g), filtered and concentrated under vacuum to obtain the crude as a pale yellow oil. Purification was done by silica gel column flash chromatography (12g column) using ethyl acetate in hexanes. The title compound **3f** was obtained as a fluffy white solid (0.1765 g, 84%). **HRMS** (ESI⁺) calculated for C₅₄H₅₀O₁₄Na⁺ [M + Na]⁺ 945.3098, found 945.3137.

### Example G.3 Synthesis of tetrasaccharide 1f

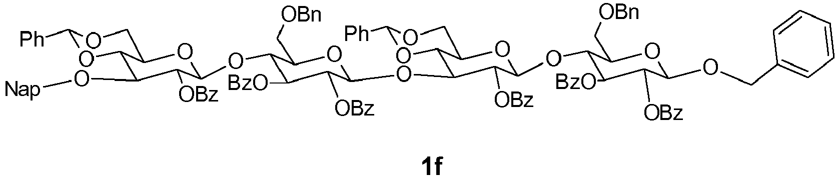

Disaccharide **2a** (0.4 g, 0.43 mmol) and disaccharide **3f** (0.57g, 0.56 mmol) were taken in anhydrous toluene (10mL) and evaporated under vacuum for 30 min to dryness and repeated this azeotropic drying process two times more. The material was dried under high vacuum for 12h. Then anhyd. DCM (10mL) was added to it under Argon atmosphere and stirred at 300 rpm. About 1 g dried 4 A molecular sieves (MS) were added and stirred at room temperature for 45 min and then cooled to -5°C (ice-acetone mixture). NIS (0.127g, 0.56 mmol) and triflic acid (8 µL, 0.09 mmol) were added to the reaction mixture and stirred for 15 min. Then it was slowly warmed to 10 °C for 0.5 h and then stirred at RT for 1h. TLC analysis (40% ethyl acetate in n-hexane) showed the disappearance of the donor spot and the presence of a new spot. The reaction was quenched at 5 °C by the addition of sat. NaHCO₃ solution (50 mL) and sat. Na₂S₂O₃ solution (100 mL). The crude compound was extracted with DCM (50 mL X 2) and washed with sat. Na₂S₂O₃ solution (50 mL X 2), sat. NaHCO₃ solution (250 mL X 2), and brine (50 mL X 2). After separation, organic layer was dried over anhydrous MgSO₄ (∼5 g), filtered, and concentrated under reduced pressure to obtain the crude compound. The crude organic product was purified by flash chromatography (column 24g column) using n-Hex/EtOAc gradient system and the title compound **1f** (0.575 g, 70 %) was obtained as a white solid. **HRMS** (ESI⁺) calculated for C₁₁₂H₁₀₀O₂₇Na⁺ [M + Na]⁺ 1899.6350, found 1899.6342.

### Example G.4 Synthesis of tetrasaccharide 11f

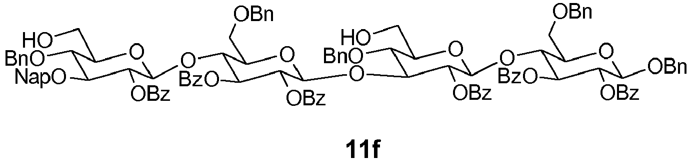

Tetrasaccharide **1f** (0.45 g, 0.24 mmol) in anhydrous DCM (5 mL) was stirred under nitrogen atmosphere at 350-400 rpm. Added BH₃•THF (1M in THF, 2.4 mL, 2.4 mmol) followed by AgOTf (18.5 mg, 0.07 mmol) and covered the reaction mixture with aluminum foil to avoid light and stirred the reaction mixture at RT for 2 h. TLC analysis (60%EtOAc/toluene and 10-15% acetone/toluene) showed the presence of a new spot slightly polar to the major amount of starting material even after 2 h, so stirred the reaction mixture for additional 2.5 h at RT. The reaction was then carefully quenched with methanol (18 mL). Reaction mixture was stirred for additional 2 h at rt before basified with triethylamine (9 mL) and stirred for 30 min more. The reaction mixture was then evaporated to minimum volume and diluted with DCM (20 mL) and water (20 mL). The layers were separated. Aqueous layer was extracted with DCM (10 mL X 3). Combined organic phases were then washed with dil. HCI (20 mL), sat. NaHCO₃ (20 mL X 2), brine solution (20 mL), dried over anhyd. MgSO₄ (∼5 g), filtered, evaporated under vacuum to obtain colorless gummy liquid. Crude product was purified by silica column chromatography using acetone in toluene. The title compound **11f** was obtained as a colorless fluffy (0.302 g, 68%). **HRMS** (ESI⁺) calculated for C₁₁₂H₁₀₄O₂₇Na⁺ [M + Na]⁺ 1903.6663, found 1903.6657.

### Example G.5 Synthesis of tetrasaccharide 12f

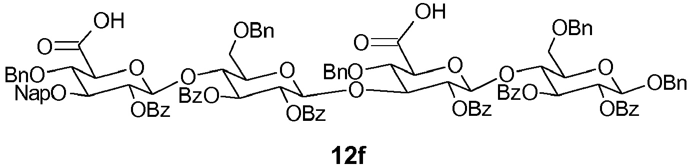

Tetrasaccharide **11f** (0.15 g, 3.08 mmol) in a mixture of DCM (3 mL) - water (0.6 mL) was stirred under nitrogen atmosphere at 400-450 rpm and cooled to 0 °C (ice -water). TEMPO (6 mg, 0.04 mmol) and BAIB (0.15 g, 0.48 mmol) were added and the solution was stirred at 0 °C for 20 min and then at 10 °C for 2 h. The reaction mixture was slowly warmed to room temperature, followed by additional stirring for 2 h. Added TEMPO (50 mg) more and continued stirring at rt for 2 h more. Reaction was monitored by TLC (25% acetone/DCM and 30% acetone/DCM + 2% AcOH). Reaction showed the presence of intermediate and product; so more TEMPO (70 mg) was added and continued the stirring at rt for 20 min. Reaction mixture was then diluted with DCM (20 mL) and water (20 mL). The layers were separated, and the aqueous layer was extracted with DCM (10 mL X 3). Combined organic extracts were dried over MgSO₄ (∼5 g), filtered, and the filtrate was concentrated under vacuum to obtain the crude product. Purification was done by silica gel column chromatography (column ø 20 X 300 mm) using DCM- 10% acetone/DCM+1% AcOH. The title compound **12f** was obtained as an off-white solid (0.1220 g, 80%). **HRMS** (ESI⁺) calculated for C₁₁₂H₁₀₀O₂₉Na⁺ [M + Na]⁺ 1931.6248, found 1931.6268.

### Example G.6 Synthesis of tetrasaccharide 13f

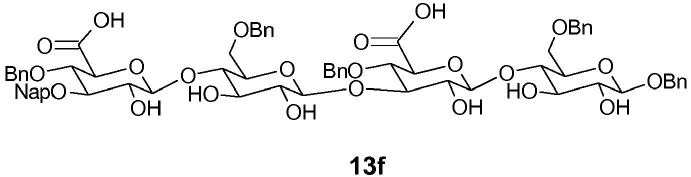

Diacid **12f** (0.117 g, 0.06 mmol) was taken in THF (2.5 mL) and stirred under nitrogen atmosphere equipped at 250 rpm at RT. 0.5 M methanolic solution of sodium methoxide (6.2 mL, 3.076 mmol) was added. The resulting solution was stirred at 50 °C for 25 h. Reaction was monitored by NMR and TLC [(30% MeOH/acetone+∼2 drops of AcOH and 30% acetone/DCM+∼1-2% AcOH). The reaction mixture was evaporated under and then diluted with ethyl acetate (∼25 mL) and water (50 mL).The mixture was acidified using 50% aq. AcOH solution (50 mL) and the layers were separated. The aqueous layer was then extracted with ethyl acetate (50 mL X 3). Combined organic layers were washed with water (25 mL), brine (25 mL), dried (MgSO₄), filtered, and concentrated under vacuum to get a yellow solid. Then ∼2% ethyl acetate/hexanes (∼25 mL) was added to the solid, warmed to 45 °C and triturated, cooled to rt and filtered. The solid was washed with warm ∼2% ethyl acetate/hexanes six times more and dried in high vacuum to afford pale yellow solid as the title compound **13f** (0.05 g, 59). **HRMS** (ESI⁺) calculated for C₇₀H₇₆O₂₃₋ [M - H]⁻ 1283.4705, found 1283.4688.

### Example G.7 Synthesis of ST3 tetrasaccharide 8'

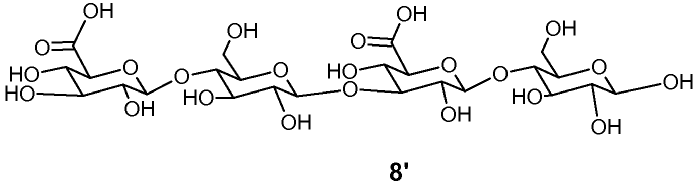

Diacid **13f** (15 mg, 0.012 mmol) was taken in a solvent mixture of *t*BuOH (1.625 mL), DCM (0.375 mL), and H₂O (0.25 mL) and stirred under nitrogen at 250 rpm at RT. A suspension of 10% Pd/C (5 mg) in *t*BuOH (1 mL) was added to it. The reaction mixture was purged under hydrogen gas and subsequently stirred under a pressure of 3 bar for 24 h at RT. The reaction mixture was then filtered through a PTFE hydrophobic filter (0.45 µm) and the filter was washed thoroughly with methanol (3 mL X 5) and water-methanol (6:4, 3 mL X 5). The filtrate was evaporated to dryness under vacuum to obtain the off-white solid as crude product. 1H NMR of the crude product showed the completion of the reaction and absence of any diacid **13f** and intermediates presence. The crude product was then purified by chromatography using C18 Sepak column using water and methanol as eluents to get desired pure product. The title compound **8'** was obtained as a white foamy solid (4.8 mg, 60%). **HRMS** (ESI⁺) calculated for C₂₄H₃₈O₂₃Na⁺ [M + Na]⁺ 717.1702, found 717.1680.**¹H NMR** (400 MHz, Deuterium Oxide, mixture of anomers) δ 5.28 (d, J = 3.6 Hz, 0.5H), 4.86 (d, J = 7.8 Hz, 1H), 4.71 (d, J = 8.1 Hz, 0.5H), 4.68 - 4.57 (m, 2H), 4.13 - 3.95 (m, 4H), 3.95 - 3.81 (m, 4H), 3.79 - 3.60 (m, 10H), 3.44 - 3.31 (m, 2H).

### H. Synthesis of ST3 hexasaccharide

### Example H.1 Synthesis of ST3 tetrasaccharide 3a-B

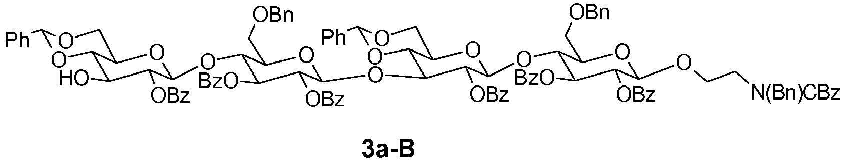

Tetrasaccharide **1a** (0.4 g, 0.195 mmol, impure) was transferred to a stirring solution of DCM (10 mL) and water (0.2 mL) and subsequently cooled (5 to 10 °C) for 5 min under nitrogen atmosphere equipped with a stir bar and stirring of 400 - 450 rpm. DDQ (0.2 g, 0.876 mmol) was added slowly over a period of 2 h in portions and stirred the reaction mixture at RT for additional 2.5 h till the completion of the reaction, monitored by TLC analysis (20% EtOAc/toluene). The reaction was quenched by the addition of sat. aq. NaHCO₃ (25 mL) and extracted into DCM (15 mL X 3). The combined organic layer was washed with sat. NaHCO₃ solution (5 mL), brine (10 mL), dried over anhyd. Na₂SO₄ (∼5 g), filtered and concentrated under vacuum at 30-35 °C bath temperature of rotary evaporator in a 100 mL RBF for 1 h to obtain the crude as a dark brownish fluffy solid. Purification was done on silica gel column chromatography using ethyl acetate in toluene to afford white solid (225 mg, 60%). MALDI analysis: calculated for C₁₁₁H₁₀₃NNaO₂₉ [M+Na]+ 1938.01, found 1938.86.

### Example H.2 Synthesis of ST3 hexasaccharide 1a-B

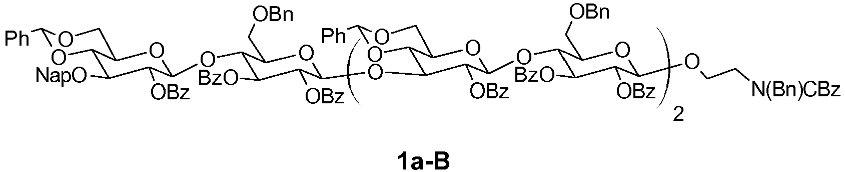

Thioglycoside donor **2a** (118 mg, 0.062 mmol) and tetrasaccharide acceptor **3a-B** (188 mg, 0.185 mmol) were taken in a 25 mL RBF, added anhydrous toluene (10 mL) and evaporated under vacuum for 30 min to dryness and repeated this azeotropic drying process. The material was dried under high vacuum for 2-3 h. Then DCM (2 mL) was added to it under argon atmosphere equipped with a stir bar and stirring of 600 rpm. 0.1 g of microwave dried 4 Å molecular sieves (MS) were added and stirred at room temperature for 30 min. NIS (34.7 mg, 0.154 mmol) and TfOH (∼1 µL, 0.012 mmol) were added to the reaction mixture and stirred at rt for 2 h and monitored using TLC analysis (20% EtOAc/toluene and 15% acetone/toluene). The reaction was then quenched with sat. aq. NaHCO₃ (5 mL) and the reaction mixture was stirred for 10 min at RT. RM was filtered through Celite® bed and washed with DCM (5 mL X 2) and separated the layers. Then the aqueous was extracted with DCM (15 mL X 2). Combined organic layer was washed with 10% aq. Na₂S₂O₃ solution (5 mL X 2), sat. NaHCO₃ solution (5 mL), brine (5 mL), dried over anhyd. Na₂SO₄, filtered and concentrated under vacuum at 30-35 °C bath temperature of rotary evaporator in a 100 mL RBF for 1 h to obtain the crude as a pale yellow solid. Purification was done on silica gel column chromatography using ethyl acetate in toluene to yield off-white solid as the desired product (125 mg, 70.7%). MALDI analysis: calculated for C₁₆₉H₁₅₃NKO₄₂ [M+K]⁺ 2909.15, found 2909.92.

### J. Synthesis of ST3 octasaccharide

### Example J.1 Synthesis of ST3 hexasaccharide 3a-C

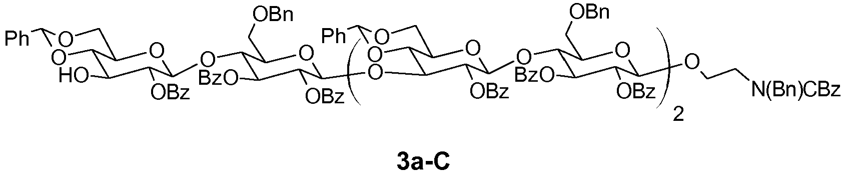

Hexasaccharide **1a-B** (80 mg, 0.03 mmol) was transferred to a stirring solution of DCM (3 mL) and water (0.1 mL) in a 10 mL RBF, and subsequently cooled (5 to 10 °C) for 5 min under nitrogen atmosphere equipped with a stir bar and stirring of 400 - 450 rpm. DDQ (29 mg, 0.13 mmol) was added slowly over a period of 2 h in portions and stirred the reaction mixture at RT for additional 2.5 h and reaction was monitored using TLC analysis (20%EtOAc/Toluene) till the completion of the reaction. The reaction was quenched by the addition of sat. aq. NaHCO₃ (10 mL) and extracted into DCM (15 mL X 3). The combined organic layer was washed with sat. NaHCO₃ solution (5 mL), brine (5 mL), dried over anhyd. Na₂SO₄, filtered and concentrated under vacuum at 30-35 °C bath temperature of rotary evaporator in a 50 mL RBF for 1 h to obtain the crude as a dark brownish fluffy solid. Purification was done on silica gel column chromatography using ethyl acetate in toluene to afford white solid (45 mg, 59%). MALDI analysis: calculated for C₁₅₈H₁₄₅NKO₄₂ [M+K]⁺ 2768.96, found 2768.44.

### Example J.2 Synthesis of ST3 octasaccharide 1a-C

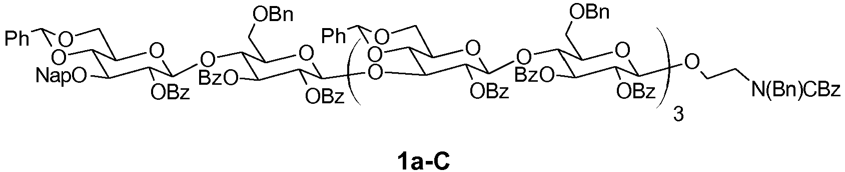

Thioglycoside donor **2a** (39.1 mg, 0.039 mmol) and hexasaccharide acceptor **3a-C** (35 mg, 0.013 mmol) were taken in a 25 mL RBF, added anhydrous toluene (5 mL) and evaporated under vacuum for 30 min to dryness and repeated this azeotropic drying process. The material was dried under high vacuum for 2-3 h. Then DCM (2 mL) was added to it under argon atmosphere equipped with a stir bar and stirring of 400 rpm. -0.05 g of microwave dried 4 Å molecular sieves (MS) were added and stirred at room temperature for 30 min. NIS (7.2 mg, 0.032 mmol) and TfOH (∼1 µL, 0.003 mmol) were added to the reaction mixture and stirred at rt for 2 h and monitored the reaction using TLC analysis (15% EtOAc + 5% acetone)/toluene) till the completion of the reaction. The reaction was then quenched with sat. aq. NaHCO₃ (3 mL) and the reaction mixture was stirred for 10 min at RT. RM was filtered through Celite® bed and washed with DCM (5 mL X 2) and separated the layers. Then the aqueous was extracted with DCM (5 mL X 2). Combined organic layer was washed with 10% aq. Na₂S₂O₃ solution (5 mL), sat. NaHCO₃ solution (3 mL), brine (3 mL), dried over anhyd. Na₂SO₄, filtered and concentrated under vacuum at 30-35 °C bath temperature of rotary evaporator in a 100 mL RBF for 1 h to obtain the crude as a pale yellow solid. Purification was done on silica gel column chromatography using ethyl acetate in toluene and (EtOAc + 5% acetone) in toluene to get off-white gummy solid (15 mg, 31.8%). MALDI analysis: calculated for C₂₁₆H₁₉₅KNO₅₅ [M+K]⁺ 3723.99, found 3725.94.

## Claims

1. A synthetic method comprising the following steps:
a) providing a disaccharide of formula **2** 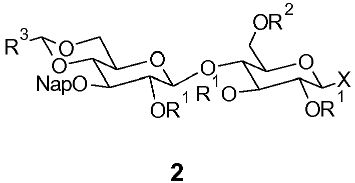 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl or alkyl group, and
X represents -OAc or -SR⁵, with R⁵ being selected from -Me, -Et, -Pr, -Bu, -Ph and -Tol;
and a saccharide of formula **3** 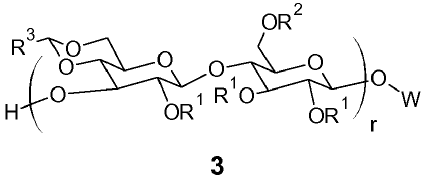 wherein
R¹ represents an orthogonal oxidation-stable and acid-stable protecting group,
R² represents an orthogonal oxidation-stable and acid-stable protecting group,
R³ is an aryl or alkyl group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10,
r is 1, 2 or 3;
b) coupling of the disaccharide of formula **2** with the saccharide of formula **3** in presence of a catalyst to obtain a saccharide of formula **1** 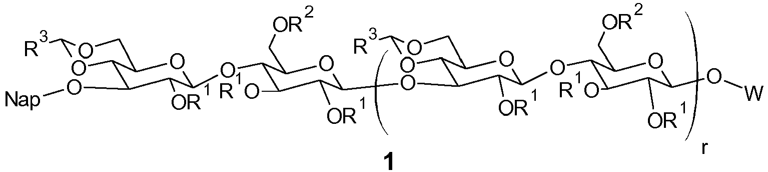 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl or alkyl group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3.

2. The method according to claim 1, further comprising performing selective removal of the acetal groups by treatment of the saccharide of formula **1** according to claim 1 with a nucleophile in presence of a catalytic amount of an acid to yield a saccharide of formula **4,** 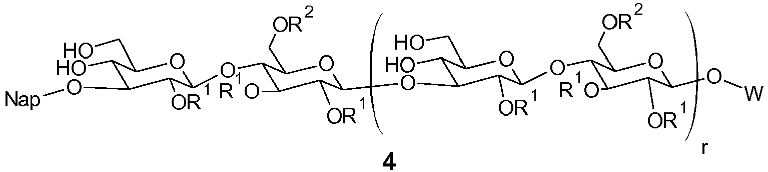 wherein
Nap represents 2-naphthylmethyl group,
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3;
or
performing reductive removal of the acetal groups by treatment of the saccharide of formula **1** according to claim 1 with a borane in presence of a catalytic amount of a Lewis acid to yield a saccharide of formula **11**, 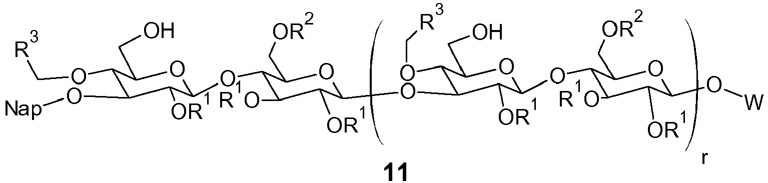 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3.

3. The method according to claim 2, further comprising performing oxidation of the saccharide of formula **4** according to claim 2 to yield a saccharide of formula **5**, 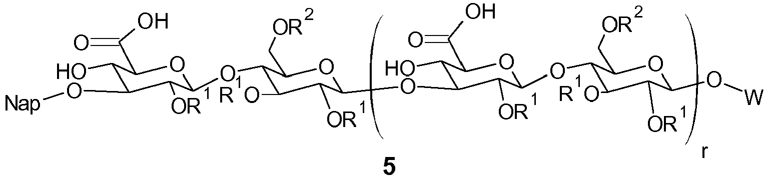 wherein
Nap represents 2-naphthylmethyl group,
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3;
or
performing oxidation of the saccharide of formula **11** according to claim 2 to yield a saccharide of formula **12**, 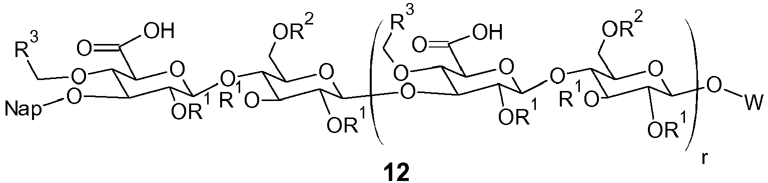 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3.

4. The method according to claim 3, further comprising performing removal of protecting groups R¹ of saccharide of formula **5** according to claim 3 in presence of a base to yield a saccharide of formula **7**, 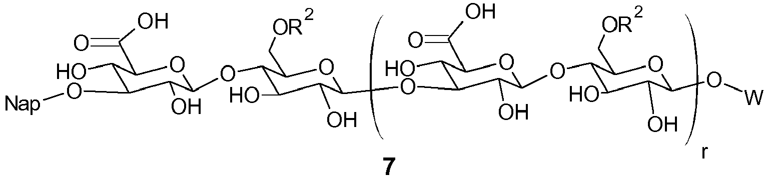 wherein
Nap represents 2-naphthylmethyl group,
R¹ represents an orthogonal oxidation-stable and acid-stable protecting group,
R² represents an orthogonal oxidation-stable and acid-stable protecting group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3;
or
performing removal of protecting groups R¹ of saccharide of formula **12** according to claim 3 in presence of a base to yield a saccharide of formula **13**, 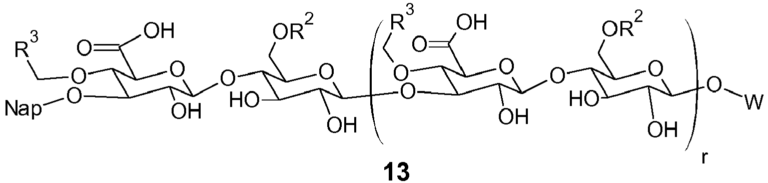 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10, and
r is 1, 2 or 3.

5. The method according to claim 4, further comprising hydrogenating the saccharide of formula **7** or formula **13** according to claim 4 in presence of a catalyst to yield a saccharide of formula **8** or a saccharide of formula **8'**, 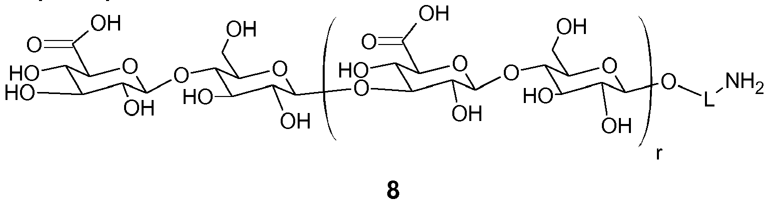 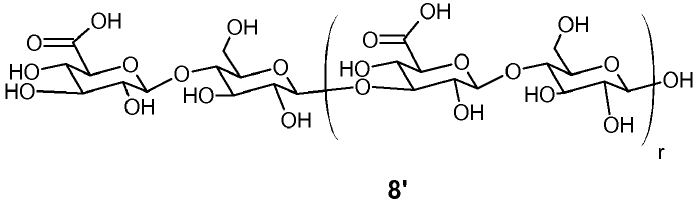
wherein L represents -(CH₂)ₙ- or -C₂H₄-O-C₂H₄-,
n is an integer selected from 2, 3, 4, 5, 6, 7, 8, 9 and 10, and
r is 1, 2 or 3.

6. The method according to any one of claims 1 - 5 further comprising
a6) reacting the disaccharide of formula **2** according to claim 1 with an alcohol HO-W in presence of a catalyst to yield a disaccharide of formula **9** 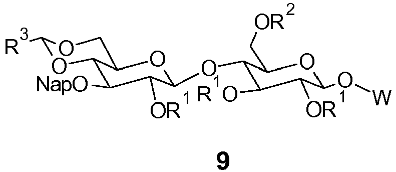 wherein
Nap represents 2-naphthylmethyl group,
R¹ represents an orthogonal oxidation-stable and acid-stable protecting group,
R² represents an orthogonal oxidation-stable and acid-stable protecting group,
R³ is an aryl or alkyl group,
W represents -L-P or -Bn,
P is -NR'R" or -N₃,
R' and R" represent independently of each other -Bn, -Cbz, -Allyl or
R' and R" form together phthaloyl,
L represents -(CH₂)ₙ- or -C₂H₄-O-C₂H₄-,
n is an integer selected from 2, 3, 4, 5, 6, 7, 8, 9 and 10; and
b6) converting the disaccharide of formula **9** according to claim 6 to the saccharide of formula **3-A** according to claim 1 by treatment with an oxidizing agent

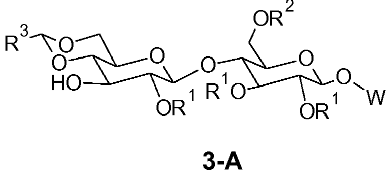

7. The method according to any one of claims 1 - 6 further comprising b6) converting the saccharide of formula **1** according to claim 1 to the saccharide of formula **3** by treatment with an oxidizing agent.

8. The method according to any one of claims 1 - 7 further comprising:
a2) providing a monosaccharide building block of formula **14** 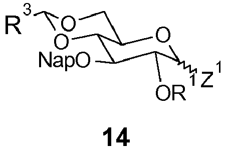 wherein
Nap represents 2-naphthylmethyl group,
R¹ represents an orthogonal oxidation-stable and acid-stable protecting group,
R³ is an aryl or alkyl group,
and Z¹ is selected from -O-C(=NH)-CCl₃, -O-C(=NPh)-CF₃, -OAc and -SR⁵, with R⁵ being selected from -Me, -Et, -Pr, -Bu, -Ph and -Tol,
and a monosaccharide building block **15** 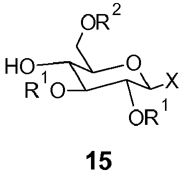 wherein
R¹ represents an orthogonal oxidation-stable and acid-stable protecting group,
R² represents an orthogonal oxidation-stable and acid-stable protecting group, and
X represents -SR⁵, with R⁵ being selected from -Me, -Et, -Pr, -Bu, -Ph and -Tol;
and
b2) coupling of the monosaccharide building block of formula **14** with the monosaccharide building block of formula **15** in presence of a catalyst to yield the disaccharide of formula **2** according to claim 1.

9. The method according to claim 8, further comprising:
a3) providing a monosaccharide of formula **18** 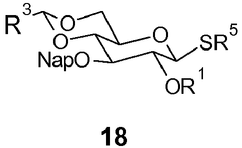 wherein
Nap represents 2-naphthylmethyl group,
R¹ is an orthogonal oxidation-stable and acid-stable protecting group,
R³ is an aryl or alkyl group, and
R⁵ is selected from -Me, -Et, -Pr, -Bu, -Ph, and -Tol;
and
b3) converting the monosaccharide of formula **18** to a monosaccharide of formula **17** 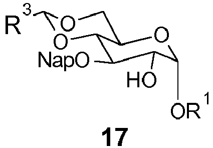 wherein
Nap represents 2-naphthylmethyl group,
R¹ is an orthogonal oxidation-stable and acid-stable protecting group,
R³ is an aryl or alkyl group;
and
c3) treatment of the monosaccharide of formula **17** with a base to obtain a monosaccharide of formula **16** 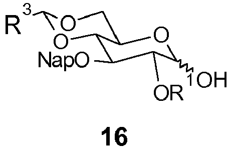 wherein
Nap represents 2-naphthylmethyl group,
R¹ is an orthogonal oxidation-stable and acid-stable protecting group,
R³ is an aryl or alkyl group;
and
d3) converting the monosaccharide of formula **16** to the monosaccharide of formula **14** according to claim 8.

10. The method according to any one of claims 1 - 9, wherein
R¹ is selected from -Bz, -ClAc, -Ac and -Piv,
R² represents -Bn
R³ is selected from -Ph, -PMP and -Me,
P is -NR'R",
R' and R" represent independently of each other -Bn, -Cbz, -Allyl, and
n is an integer selected from 2, 3, 4 and 5.

11. Intermediates of the following general formula: 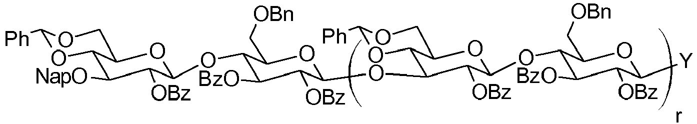
**1'** Y = -O-(CH₂)ₙ-N(Bn)Cbz
**1"** Y = -O-(CH₂)ₙ-N₃
**1'"** Y = -O-(CH₂)ₙ-NH₂
**1e** Y = -O-C₂H₄-O-C₂H₄-N₃
**1f** Y = -O-Bn
**1h** Y = -O-(CH₂)₂NH-CO-(CH₂)₂-S-Ac 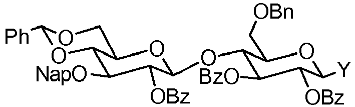
**2a** Y = -SEt
**9'** Y = -O-(CH₂)ₙ-N(Bn)Cbz
**9"** Y = -O-(CH₂)ₙ-N₃
**9e** Y = -O-C₂H₄-O-C₂H₄-N₃
**9f** Y = -O-Bn 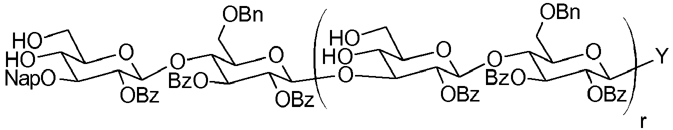
**4'** Y = -O-(CH₂)ₙ-N(Bn)Cbz
**4"** Y = -O-(CH₂)ₙ-N₃
**4e** Y = -O-C₂H₄-O-C₂H₄-N₃
**4f** Y = -O-Bn 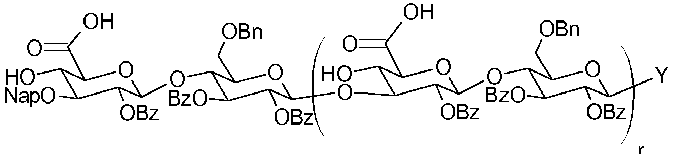
**5'** Y = -O-(CH₂)ₙ-N(Bn)Cbz
**5"** Y = -O-(CH₂)ₙ-N₃
**5e** Y = -O-C₂H₄-O-C₂H₄-N₃
**5f** Y = -O-Bn 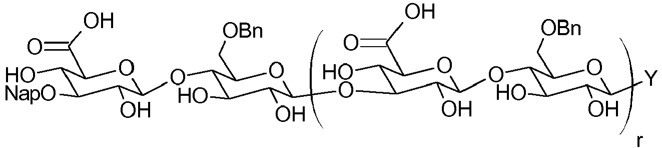
**7'** Y = -O-(CH₂)ₙ-N(Bn)Cbz
**7"** Y = -O-(CH₂)ₙ-N₃
**7e** Y = -O-C₂H₄-O-C₂H₄-N₃
**7f** Y = -O-Bn 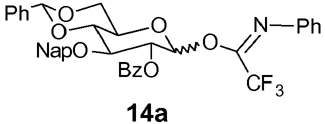
wherein Nap represents 2-naphthylmethyl group,
n is an integer selected from 2, 3, 4, 5, 6, 7, 8, 9 and 10, and
r is 1 or 2 or 3.

12. Intermediates according to claim 11, wherein intermediates **1'**, **1"**, **1"'**, **1e**, **1f**, **1h**, **2a**, **4'**, **4"**, **4e**, **4f**, **5'**, **5"**, **5e**, **5f**, **7'**, **7"**, **7e**, **7f**, **9'**, **9"**, **9e**, **9f** and **14a** are crystalline.

13. The method according to claim 5 further comprising:
a4) reacting the saccharide of formula **8** with a linker J¹-L²-J² under addition of a base to yield saccharide of formula **28**, 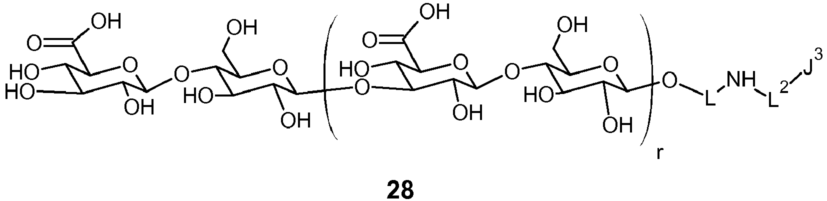
wherein L represents -(CH₂)ₙ-,
n is an integer selected from 2, 3, 4, 5, 6, 7, 8, 9 and 10,
r is 1, 2 or 3,
J³ is J¹ or J²,
L² is selected from:
-C(O)-, -E-, -C(O)-NH-NH-C(O)-, 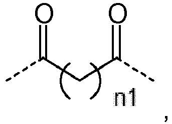 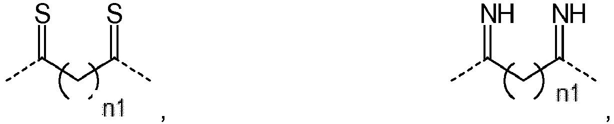 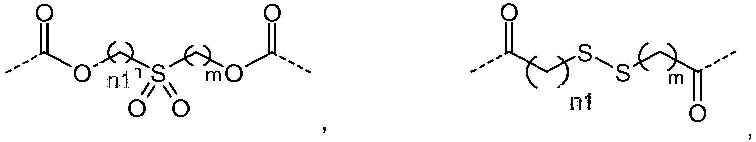 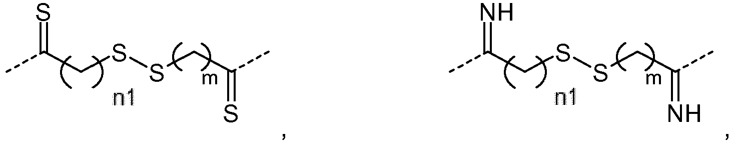 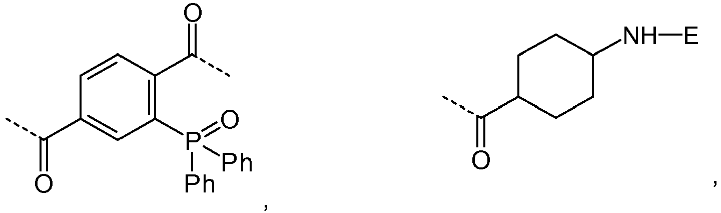 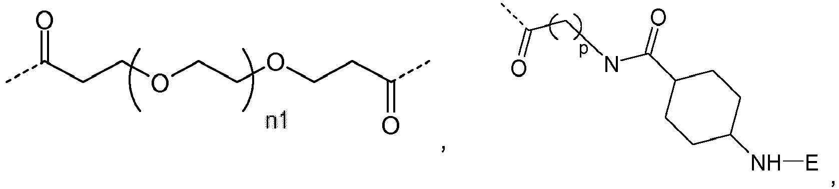 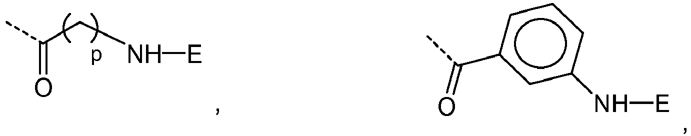 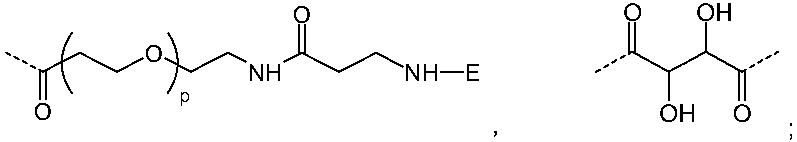
E is selected from 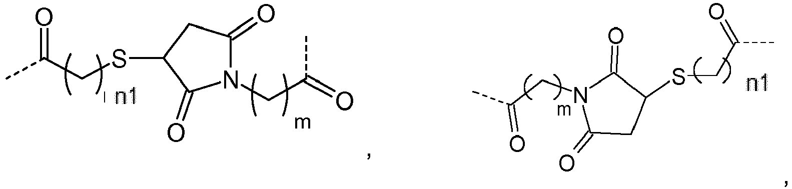 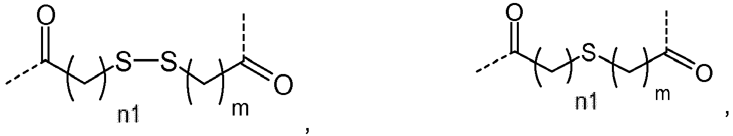 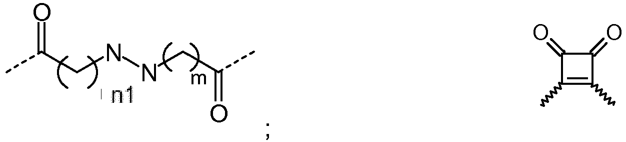
m, n1, o and p represent independently of each other an integer from 1 to 10 and
J¹ and J² are selected independently of each other from: 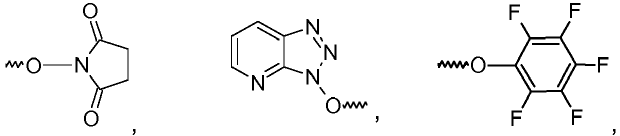 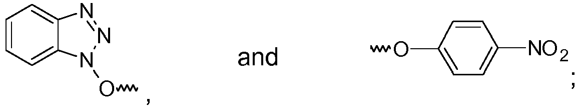
and
b4) coupling the saccharide of formula **28** to a carrier protein.

## Patentansprüche

1. Syntheseverfahren umfassend die folgenden Schritte:
a) Bereitstellen eines Disaccharids der Formel 2 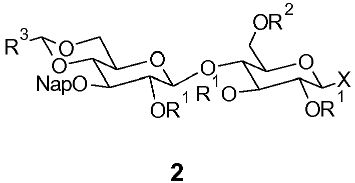 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
R³ eine Aryl- oder Alkyl-Gruppe ist und
X -OAc oder -SR⁵ mit R⁵ ausgewählt aus -Me, -Et, -Pr, -Bu, -Ph und -Tol darstellt;
und ein Saccharid der Formel 3 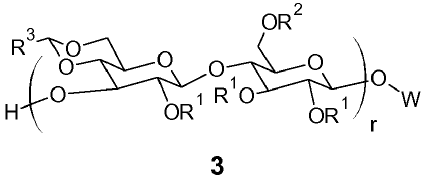 wobei
R¹ eine orthogonale oxidationsstabile und säurestabile Schutzgruppe darstellt,
R² eine orthogonale oxidationsstabile und säurestabile Schutzgruppe darstellt,
R³ eine Aryl- oder Alkyl-Gruppe ist,
W -L-P oder -Bn darstellt,
P -NR'R" oder -N₃ ist,
R'und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist,
r 1, 2 oder 3 ist;
b) Kuppeln des Disaccharids der Formel **2** mit dem Saccharid der Formel **3** in Gegenwart eines Katalysators zur Bildung eines Saccharids der Formel **1** 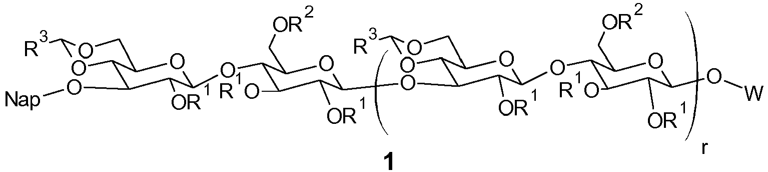 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
R³ eine Aryl- oder Alkyl-Gruppe ist,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist.

2. Verfahren gemäß Anspruch 1, weiter umfassend Durchführen einer selektiven Entfernung der Acetalgruppen durch Behandlung des Saccharids der Formel **1** gemäß Anspruch 1 mit einem Nucleophil in Gegenwart einer katalytischen Menge einer Säure zum Erhalt eines Saccharids der Formel **4,** 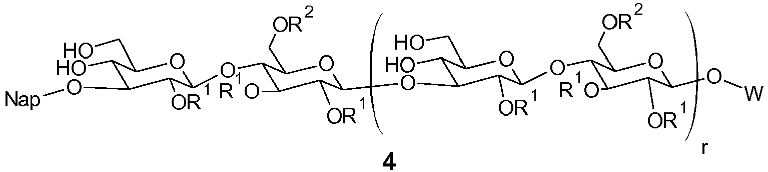 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist
oder
Durchführen einer reduktiven Entfernung der Acetalgruppen durch Behandlung des Saccharids der Formel **1** gemäß Anspruch 1 mit einem Boran in Gegenwart einer katalytischen Menge einer Lewis-Säure zum Erhalt eines Saccharids der Formel **11**, 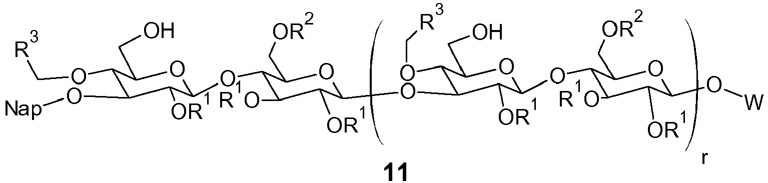 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
R³ eine Aryl-Gruppe ist,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist.

3. Das Verfahren gemäß Anspruch 2, weiter umfassend Durchführen einer Oxidation des Saccharids der Formel **4** gemäß Anspruch 2 zum Erhalt eine Saccharids der Formel **5**, 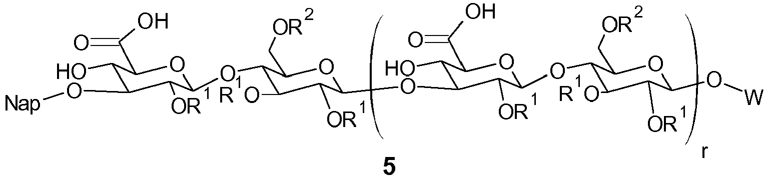 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist
oder
Durchführen einer Oxidation des Saccharids der Formel **11** gemäß Anspruch 2 zum Erhalt eines Saccharids der Formel **12**, 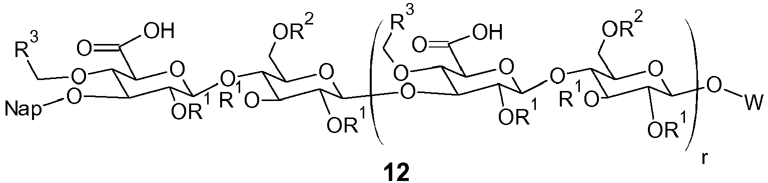 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
R³ eine Aryl-Gruppe ist,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist.

4. Verfahren gemäß Anspruch 3, weiter umfassend Durchführen einer Entfernung der Schutzgruppen R¹ des Saccharids der Formel **5** gemäß Anspruch 3 in Gegenwart einer Base zum Erhalt eine Saccharids der Formel **7**, 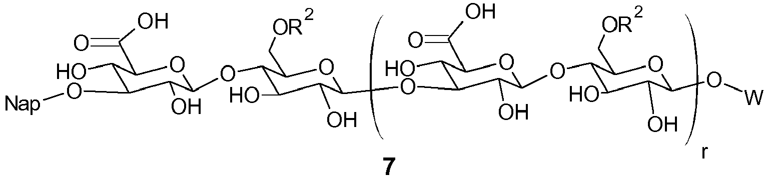 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist
oder
Durchführen einer Entfernung der Schutzgruppen R¹ des Saccharids der Formel **12** gemäß Anspruch 3 in Gegenwart einer Base zum Erhalt eines Saccharids der Formel **13**, 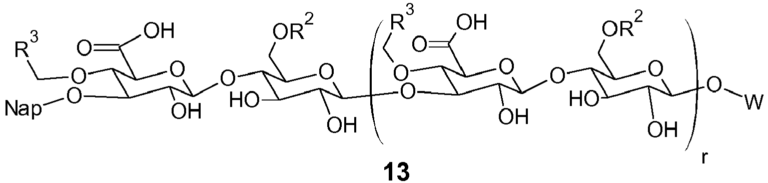 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
R³ eine Aryl-Gruppe ist,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist.

5. Verfahren gemäß Anspruch 4, weiter umfassend Hydrieren des Saccharids der Formel **7** oder der Formel **13** gemäß Anspruch 4 in Gegenwart eines Katalysators zum Erhalt eines Saccharids der Formel **8** oder eines Saccharids der Formel **8'**, 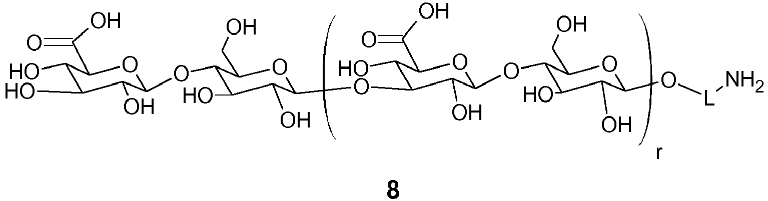 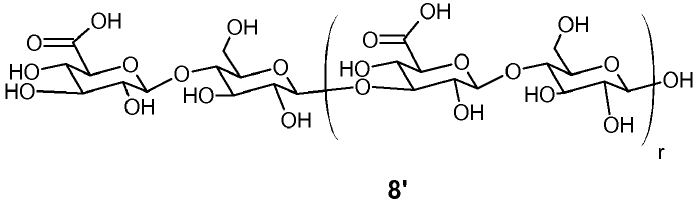
wobei L -(CH₂)ₙ- oder -C₂H₄-O-C₂H₄- darstellt,
n eine ganze Zahl ausgewählt aus 2, 3, 4, 5, 6, 7, 8, 9 und 10 ist und
r 1, 2 oder 3 ist.

6. Verfahren gemäß einem der Ansprüche 1 - 5, weiter umfassend
a6) Reagieren des Disaccharids der Formel **2** gemäß Anspruch 1 mit einem Alkohol HO-W in Gegenwart eines Katalysators zum Erhalt des Disaccharids der Formel **9,** 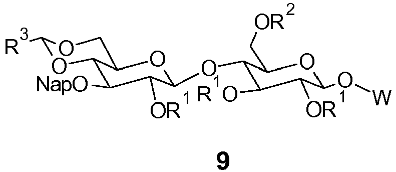 wobei
Nap eine 2-Naphthylmethyl-Gruppe darstellt,
R¹ eine orthogonale oxidationsstabile und säurestabile Schutzgruppe darstellt,
R² eine orthogonale oxidationsstabile und säurestabile Schutzgruppe darstellt,
R³ eine Aryl- oder Alkyl-Gruppe ist,
W -L-P oder -Bn darstellt,
P -NR'R" oder -N₃ ist,
R' und R" unabhängig voneinander -Bn, -Cbz, -Allyl darstellen oder
R' und R" zusammen Phthaloyl- bilden,
L -(CH₂)ₙ- oder -C₂H₄-O-C₂H₄- darstellt,
n eine ganze Zahl ausgewählt aus 2, 3, 4, 5, 6, 7, 8, 9 und 10 ist und
b6) Umwandeln des Disaccharids der Formel **9** gemäß Anspruch 6 in das Disaccharid der Formel **3-A** gemäß Anspruch 1 durch Behandlung mit einem Oxidationsmittel

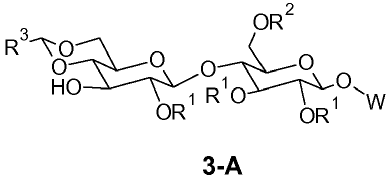

7. Verfahren gemäß einem der Ansprüche 1 bis 6, weiter umfassend b6) Umwandeln des Saccharids der Formel **1** gemäß Anspruch 1 in das Saccharid der Formel **3** durch Behandlung mit einem Oxidationsmittel.

8. Verfahren gemäß einem der Ansprüche 1 bis 7, weiter umfassend:
a2) Bereitstellen eines Monosaccharid-Bausteins der Formel **14** 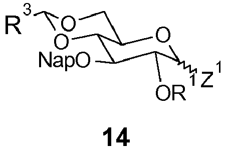 wobei
Nap eine 2-Naphthylmethyl-Gruppe darstellt,
R¹ eine orthogonale oxidationsstabile und säurestabile Schutzgruppe darstellt,
R³ eine Aryl- oder Alkyl-Gruppe ist und
Z¹ ausgewählt ist aus -O-C(=NH)-CCl₃, -O-C(=NPh)-CF₃, -OAc und -SR⁵ mit R⁵ ausgewählt aus -Me, -Et, -Pr, -Bu, -Ph und -Tol,
und eines Monosaccharid-Bausteins **15** 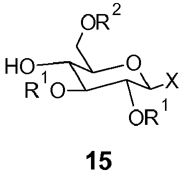 wobei
R¹ eine orthogonale oxidationsstabile und säurestabile Schutzgruppe darstellt,
R² eine orthogonale oxidationsstabile und säurestabile Schutzgruppe darstellt und
X -SR⁵ mit R⁵ ausgewählt aus -Me, -Et, -Pr, -Bu, -Ph und -Tol darstellt;
und
b2) Kuppeln des Monosaccharid-Bausteins der Formel **14** mit dem Monosaccharid-Baustein der Formel **15** in Gegenwart eines Katalysators zum Erhalt des Disaccharids der Formel **2** gemäß Anspruch 1.

9. Verfahren gemäß Anspruch 8, weiter umfassend:
a3) Bereitstellen eines Monosaccharids der Formel **18** 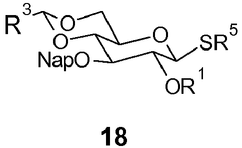 wobei
Nap eine 2-Naphthylmethyl-Gruppe darstellt,
R¹ eine orthogonale oxidationsstabile und säurestabile Schutzgruppe darstellt,
R³ eine Aryl- oder Alkyl-Gruppe ist und
R⁵ ausgewählt ist aus -Me, -Et, -Pr, -Bu, -Ph, und -Tol;
und
b3) Umwandeln des Monosaccharids der Formel **18** in ein Monosaccharid der Formel **17** 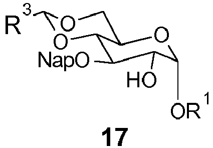 wobei
Nap eine 2-Naphthylmethyl-Gruppe darstellt,
R¹ eine orthogonale oxidationsstabile und säurestabile Schutzgruppe darstellt und
R³ eine Aryl- oder Alkyl-Gruppe ist;
und
c3) Behandeln des Monosaccharids der Formel **17** mit einer Base zum Erhalt eines Monosaccharids der Formel **16** 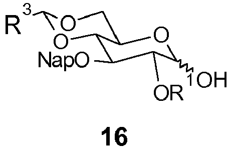 wobei
Nap eine 2-Naphthylmethyl-Gruppe darstellt,
R¹ eine orthogonale oxidationsstabile und säurestabile Schutzgruppe darstellt und
R³ eine Aryl- oder Alkyl-Gruppe ist;
und
d3) Umwandeln des Monosaccharids der Formel **16** in ein Monosaccharid der Formel **14** gemäß Anspruch 8.

10. Verfahren gemäß einem der Ansprüche 1 bis 9, wobei
R¹ ausgewählt ist aus -Bz, -ClAc, -Ac und -Piv,
R² -Bn darstellt,
R³ ausgewählt ist -Ph, -PMP und -Me,
P -NR'R" ist,
R' und R" unabhängig voneinander -Bn, -Cbz, -Allyl darstellen und
n eine ganze Zahl ausgewählt aus 2, 3, 4 und 5 ist.

11. Zwischenstufen der folgenden allgemeinen Formel: 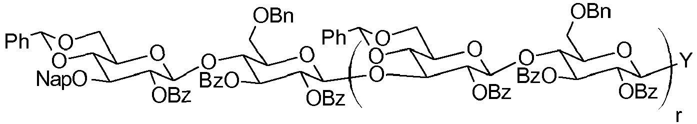
1' Y = -O-(CH₂)ₙ-N(Bn)Cbz
**1"** Y = -O-(CH₂)ₙ-N₃
**1"'** Y = -O-(CH₂)ₙ-NH₂
**1e** Y = -O-C₂H₄-O-C₂H₄-N₃
**1f** Y = -O-Bn
**1h** Y = -O-(CH₂)₂NH-CO-(CH₂)₂-S-Ac 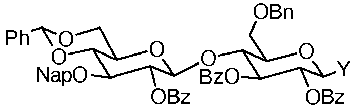
**2a** Y = -SEt
**9'** Y = -O-(CH₂)ₙ-N(Bn)Cbz
**9"** Y = -O-(CH₂)ₙ-N₃
**9e** Y = -O-C₂H₄-O-C₂H₄-N₃
**9f** Y = -O-Bn 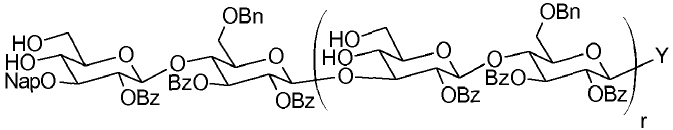
**4'** Y = -O-(CH₂)ₙ-N(Bn)Cbz
**4"** Y = -O-(CH₂)-N₃
**4e** Y = -O-C₂H₄-O-C₂H₄-N₃
**4f** Y = -O-Bn 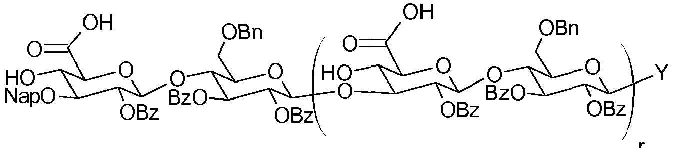
5' Y = -O-(CH₂)ₙ-N(Bn)Cbz
**5"** Y = -O-(CH₂)ₙ-N₃
**5e** Y = -O-C₂H₄-O-C₂H₄-N₃
**5f** Y = -O-Bn 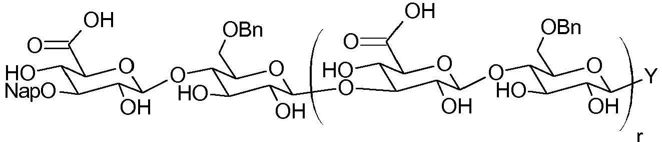
**7'** Y = -O-(CH₂)ₙ-N(Bn)Cbz
**7"** Y = -O-(CH₂)ₙ-N₃
**7e** Y = -O-C₂H₄-O-C₂H₄-N₃
**7f** Y = -O-Bn 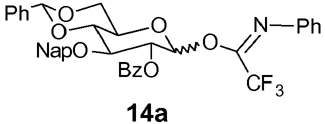
wobei Nap eine 2-Naphthylmethyl-Gruppe darstellt,
n eine ganze Zahl ausgewählt aus 2, 3, 4, 5, 6, 7, 8, 9 und 10 ist und
r 1 oder 2 oder 3 ist.

12. Zwischenstufen gemäß Anspruch 11, wobei Zwischenstufen 1', **1"**, **1'"**, **1e**, **1f**, **1h**, **2a**, **4'**, **4"**, **4e**, **4f**, **5'**, **5"**, **5e**, **5f**, **7'**, **7"**, **7e**, **7f**, **9'**, **9"**, **9e**, **9f** und **14a** kristallin sind.

13. Verfahren gemäß Anspruch 5, weiter umfassend:
a4) Reagieren des Saccharids der Formel **8** mit einem Linker J¹-L²-J² unter Zugabe einer Base zum Erhalt eines Saccharids der Formel **28**, 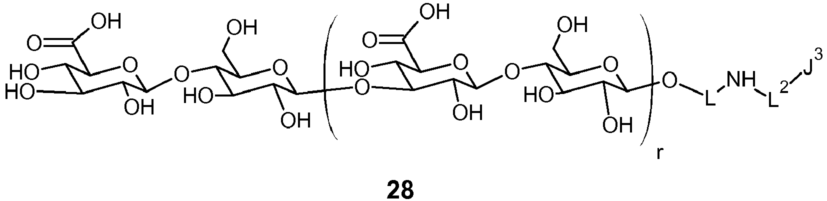
wobei L -(CH₂)ₙ- darstellt,
n eine ganze Zahl ausgewählt aus 2, 3, 4, 5, 6, 7, 8, 9 und 10 ist,
r 1, 2 oder 3 ist,
J³ J¹ oder J² ist,
L² ausgewählt ist aus:
-C(O)-, -E-, -C(O)-NH-NH-C(O)-, 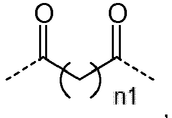 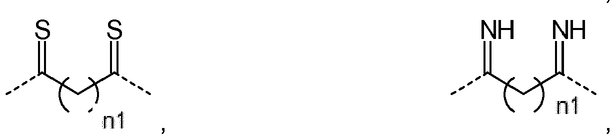 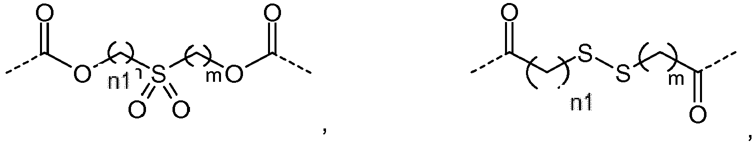 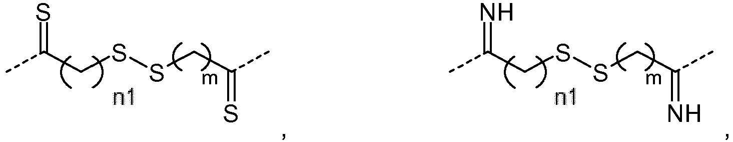 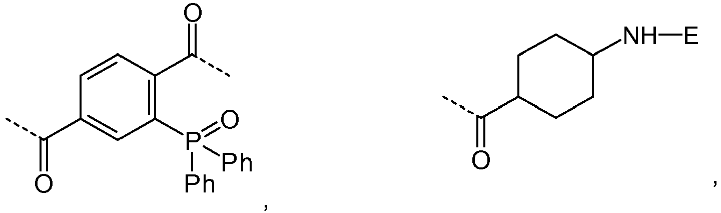 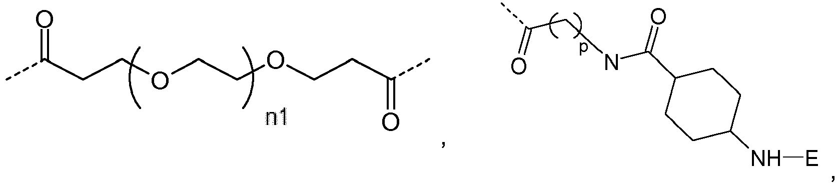 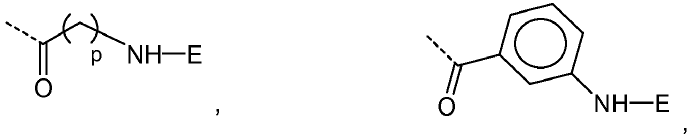 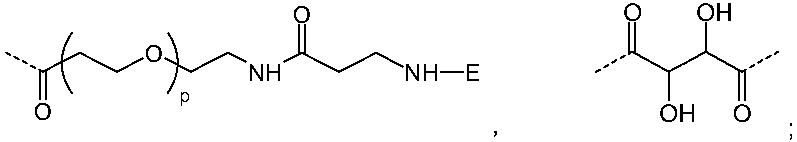
E ausgewählt ist aus 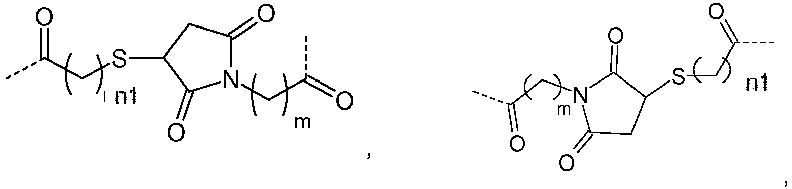 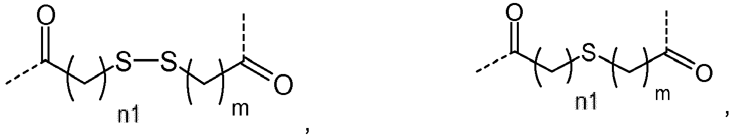 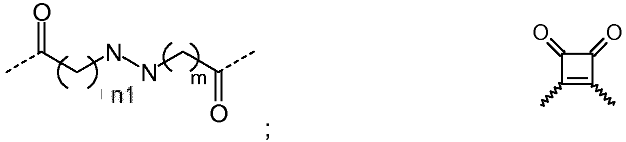
m, n1, o und p unabhängig voneinander eine ganze Zahl von 1 bis 10 darstellen
und
J¹ und J² unabhängig voneinander ausgewählt sind aus 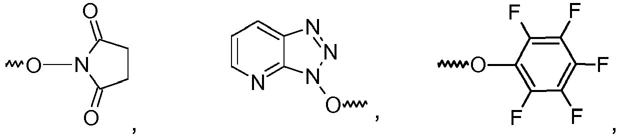 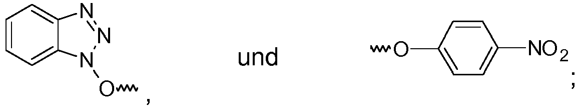
und
b4) Kuppeln des Saccharids der Formel **28** an ein Trägerprotein.

## Revendications

1. Une méthode de synthèse comprenant les étapes suivantes :
a) l'apport d'un disaccharide de formule **2** 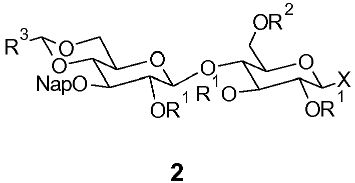 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
R³ est un groupe aryle ou alkyle, et
X représente -OAc ou -SR⁵, avec R⁵ sélectionné parmi -Me, -Et, -Pr, -Bu, -Ph et - Tol ;
et un saccharide de formule **3** 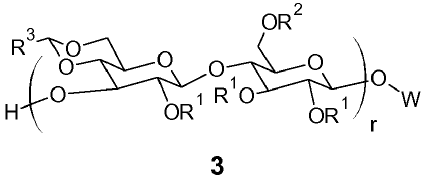 où
R¹ représente un groupe de protection orthogonal stable en milieu oxydant et en milieu acide,
R² représente un groupe de protection orthogonal stable en milieu oxydant et en milieu acide,
R³ est un groupe aryle ou alkyle,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10,
r est 1, 2 ou 3 ;
b) la liaison du disaccharide de formule **2** avec le saccharide de formule **3** en présence d'un catalyseur pour obtenir un saccharide de formule **1** 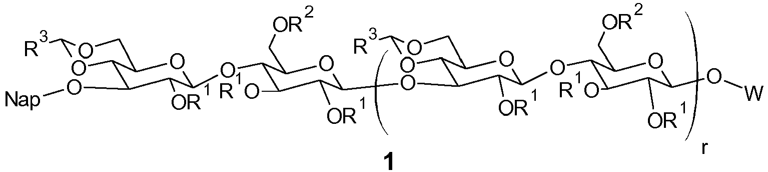 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
R³ est un groupe aryle ou alkyle,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3.

2. La méthode conforme à la revendication 1 comprenant également l'élimination sélective des groupes acétates en traitant le saccharide de formule **1** conformément à la revendication 1 avec un nucléophile en présence d'une quantité catalytique d'acide pour obtenir un saccharide de formule **4**, 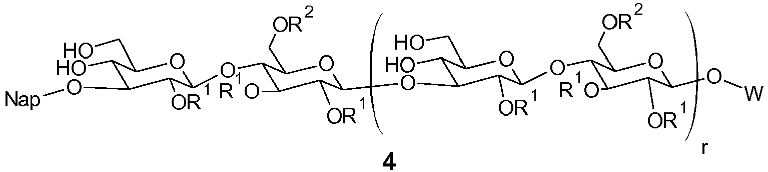 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3 ;
ou
l'élimination réductrice des groupes acétates en traitant le saccharide de formule **1** conformément à la revendication 1 avec un borane en présence d'une quantité catalytique d'acide de Lewis pour obtenir un saccharide de formule **11**, 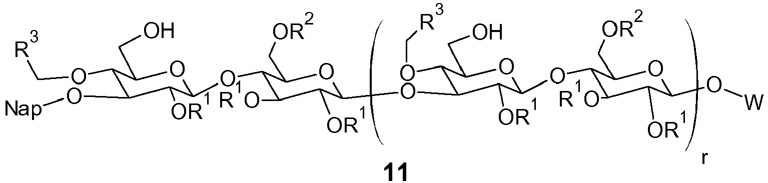 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
R³ est un groupe aryle,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3.

3. La méthode conforme à la revendication 2 comprenant également l'oxydation du saccharide de formule **4** conformément à la revendication 2 pour obtenir un saccharide de formule **5**, 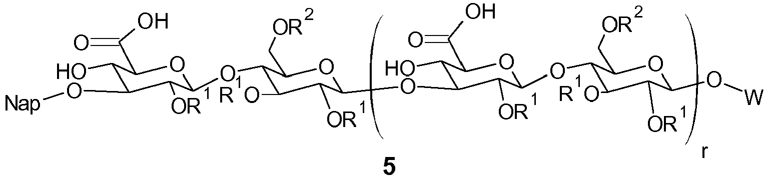 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3 ;
ou
l'oxydation du saccharide de formule **11** conformément à la revendication 2 pour obtenir un saccharide de formule **12**, 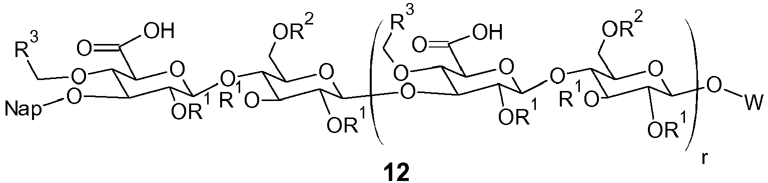 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
R³ est un groupe aryle,
W représente -L-P ou -Bn,
P est -NR'R" ou -N₃,
R' et R" représentent, indépendamment l'un de l'autre, -Bn, -Cbz, -allyle ou
R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3.

4. La méthode conforme à la revendication 3 comprenant également l'élimination des groupes de protection R¹ du saccharide de formule **5** conformément à la revendication 3 en présence d'une base pour obtenir un saccharide de formule **7**, 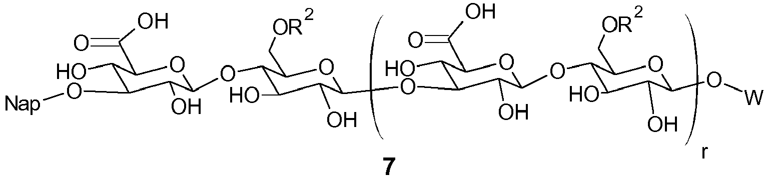 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3 ;
ou
l'élimination des groupes de protection R¹ du saccharide de formule **12** conformément à la revendication 3 en présence d'une base pour obtenir un saccharide de formule **13**, 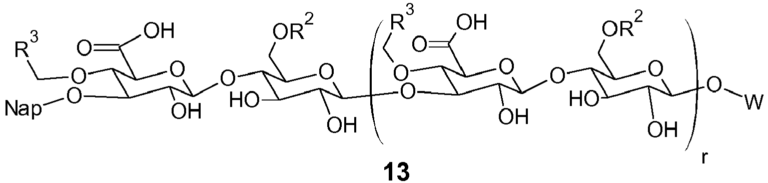 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
R³ est un groupe aryle,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3.

5. La méthode conforme à la revendication 4 comprenant également l'hydrogénation du saccharide de formule 7 ou de formule **13** conformément à la revendication 4 en présence d'un catalyseur pour obtenir un saccharide de formule **8** ou de formule **8'**, 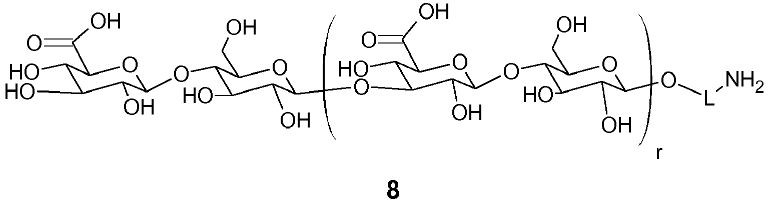 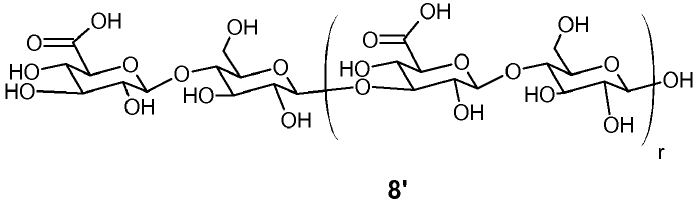
où L représente -(CH₂)ₙ- ou -C₂H₄-O-C₂H₄-,
n est un nombre entier sélectionné parmi 2, 3, 4, 5, 6, 7, 8, 9 et 10, et
r est 1, 2 ou 3.

6. La méthode conforme à l'une des revendications 1 à 5, comprenant également
a6) la réaction du disaccharide de formule **2** conformément à la revendication 1 avec un alcool HO-W en présence d'un catalyseur pour obtenir un disaccharide de formule **9** 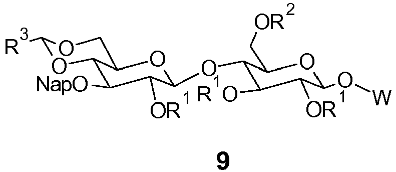 où
Nap représente le groupe 2-naphthylméthyle,
R¹ représente un groupe de protection orthogonal stable en milieu oxydant et en milieu acide,
R² représente un groupe de protection orthogonal stable en milieu oxydant et en milieu acide,
R³ est un groupe aryle ou alkyle,
W représente -L-P ou -Bn,
P est -NR'R" ou -N₃,
R' et R" représentent, indépendamment l'un de l'autre, -Bn, -Cbz, -allyle ou R' et R" forment ensemble un groupe phtaloyle,
L représente -(CH₂)ₙ- ou -C₂H₄-O-C₂H₄-,
n est un nombre entier sélectionné parmi 2, 3, 4, 5, 6, 7, 8, 9 et 10 ; et
b6) la conversion du disaccharide de formule **9** conformément à la revendication 6 en saccharide de formule **3-A** conformément à la revendication 1 par un traitement utilisant un agent oxydant.

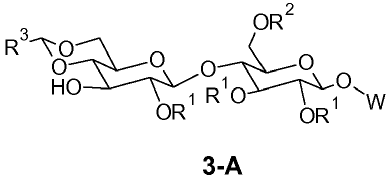

7. La méthode conforme à l'une des revendications 1 à 6, comprenant également :
b6) la conversion du saccharide de formule **1** conformément à la revendication 1 en saccharide de formule **3** par un traitement utilisant un agent oxydant.

8. La méthode conforme à l'une des revendications 1 à 7, comprenant également :
a2) l'apport d'un synthon monosaccharidique de formule **14** 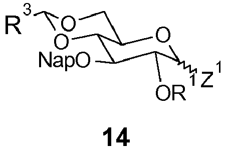 où
Nap représente le groupe 2-naphthylméthyle,
R¹ représente un groupe de protection orthogonal stable en milieu oxydant et
en milieu acide,
R³ est un groupe aryle ou alkyle,
et Z¹ est sélectionné parmi -O-C(=NH)-CCl₃, -O-C(=NPh)-CF₃, -OAc et -SR⁵, avec R⁵ sélectionné parmi -Me, -Et, -Pr, -Bu, -Ph et -Tol,
et un synthon monosaccharidique **15** 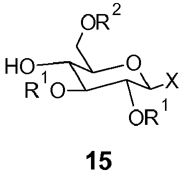 où
R¹ représente un groupe de protection orthogonal stable en milieu oxydant et en milieu acide,
R² représente un groupe de protection orthogonal stable en milieu oxydant et en milieu acide, et
X représente -SR⁵, avec R⁵ sélectionné parmi -Me, -Et, -Pr, -Bu, -Ph et -Tol ;
et
b2) la liaison du synthon monosaccharidique de formule **14** avec le synthon monosaccharidique de formule **15** en présence d'un catalyseur pour obtenir le disaccharide de formule **2** conformément à la revendication 1.

9. La méthode conforme à la revendication 8, comprenant également :
a3) l'apport d'un monosaccharide de formule **18** 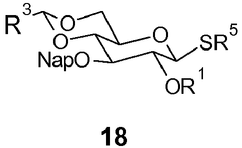 où
Nap représente le groupe 2-naphthylméthyle,
R¹ est un groupe de protection orthogonal stable en milieu oxydant et en milieu acide,
R³ est un groupe aryle ou alkyle, et
R⁵ est sélectionné parmi -Me, -Et, -Pr, -Bu, -Ph, et -Tol ;
et
b3) la conversion du monosaccharide de formule **18** en monosaccharide de formule **17** 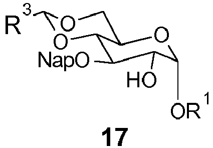 où
Nap représente le groupe 2-naphthylméthyle,
R¹ est un groupe de protection orthogonal stable en milieu oxydant et en milieu acide,
R³ est un groupe aryle ou alkyle ;
et
c3) le traitement du monosaccharide de formule **17** avec une base pour obtenir un monosaccharide de formule **16** 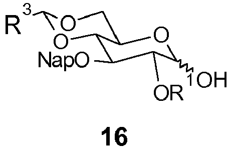 où
Nap représente le groupe 2-naphthylméthyle,
R¹ est un groupe de protection orthogonal stable en milieu oxydant et en milieu acide,
R³ est un groupe aryle ou alkyle ;
et
d3) la conversion du monosaccharide de formule **16** en monosaccharide de formule **14** conformément à la revendication 8.

10. La méthode conforme à l'une des revendications 1 à 9, où
R¹ est sélectionné parmi -Bz, -ClAc, -Ac et -Piv,
R² représente -Bn
R³ est sélectionné parmi -Ph, -PMP et -Me,
P est -NR'R",
R et R" représentent, indépendamment l'un de l'autre, -Bn, -Cbz, -allyle, et
n est un entier sélectionné parmi 2, 3, 4 et 5.

11. Intermédiaires de la formule générale suivante : 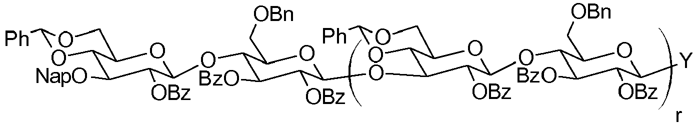
**1**' Y = -O-(CH₂)ₙ-N(Bn)Cbz
**1**" Y = -O-(CH₂)ₙ-N₃
**1**'" Y = -O-(CH₂)ₙ-NH₂
**1e** Y = -O-C₂H₄-O-C₂H₄-N₃
**1f** Y = -O-Bn
**1h** Y = -O-(CH₂)₂NH-CO-(CH₂)₂-S-Ac 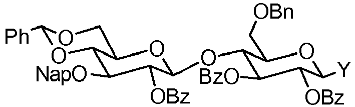
**2a** Y = -SEt
**9'** Y = -O-(CH₂)ₙ-N(Bn)Cbz
**9"** Y = -O-(CH₂)ₙ-N₃
**9e** Y = -O-C₂H₄-O-C₂H₄-N₃
**9f** Y = -O-Bn 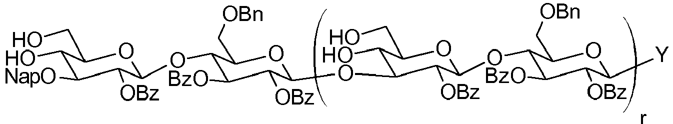
**4'** Y = -O-(CH₂)ₙ-N(Bn)Cbz
**4"** Y = -O-(CH₂)ₙ-N₃
**4e** Y = -O-C₂H₄-O-C₂H₄-N₃
**4f** Y = -O-Bn 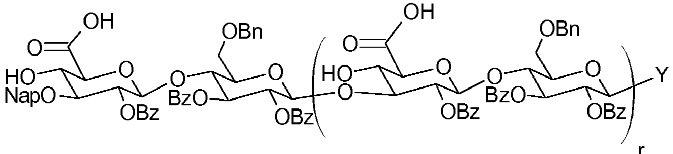
**5'** Y = -O-(CH₂)ₙ-N(Bn)Cbz
**5"** Y = -O-(CH₂)ₙ-N₃
**5e** Y = -O-C₂H₄-O-C₂H₄-N₃
**5f** Y = -O-Bn 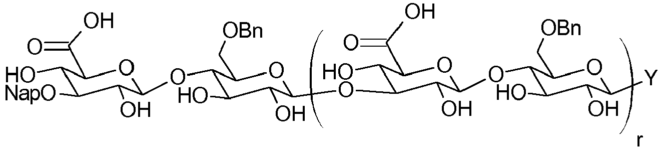
**7'** Y = -O-(CH₂)ₙ-N(Bn)Cbz
**7"** Y = -O-(CH₂)ₙ-N₃
**7e** Y = -O-C₂H₄-O-C₂H₄-N₃
**7f** Y = -O-Bn 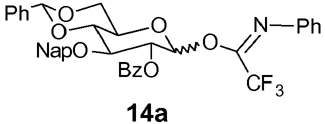
où Nap représente le groupe 2-naphthylméthyle,
n est un nombre entier sélectionné parmi 2, 3, 4, 5, 6, 7, 8, 9 et 10, et
r est 1, 2 ou 3.

12. Intermédiaires conformément à la revendication 11, où les intermédiaires **1'**, **1"**, **1'"**, **1e**, **1f**, **1h**, **2a**, **4'**, **4"**, **4e**, **4f**, **5'**, **5"**, **5e**, **5f**, **7'**, **7"**, **7e**, **7f**, **9'**, **9"**, **9e**, **9f** et **14a** sont cristallins.

13. La méthode conforme à la revendication 5, comprenant également :
a4) la réaction du saccharide de formule **8** avec un coupleur J¹-L²-J² en présence d'une base pour obtenir un saccharide de formule **28**, 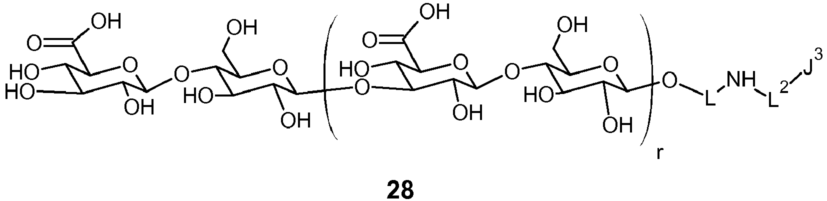
où L représente -(CH₂)ₙ-,
n est un nombre entier sélectionné parmi 2, 3, 4, 5, 6, 7, 8, 9 et 10,
r est 1, 2 ou 3.
J³ est J¹ ou J²,
L² est sélectionné parmi :
-C(O)-, -E-, -C(O)-NH-NH-C(O)-, 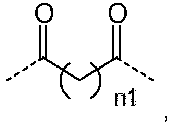 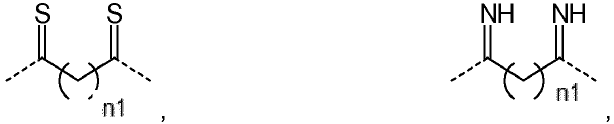 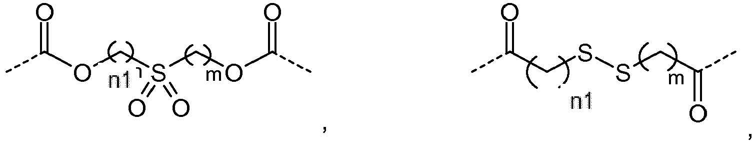 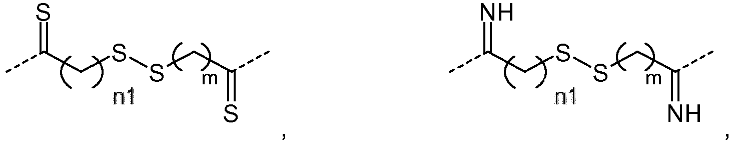 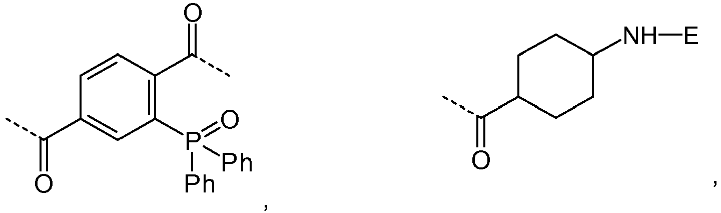 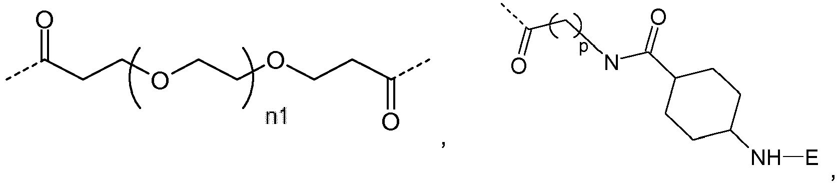 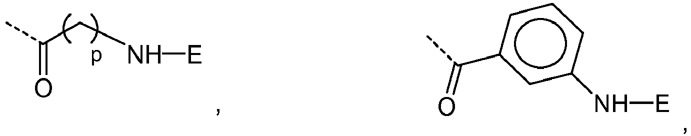 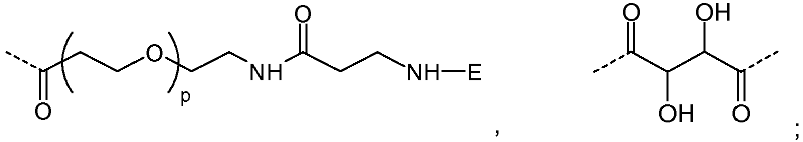
E est sélectionné parmi 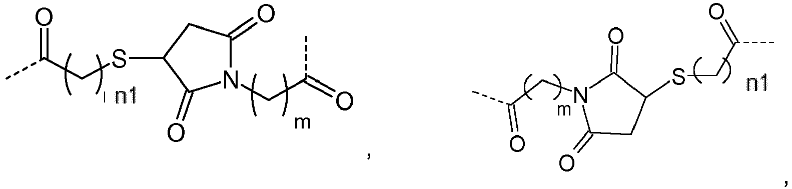 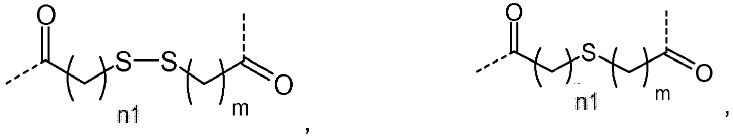 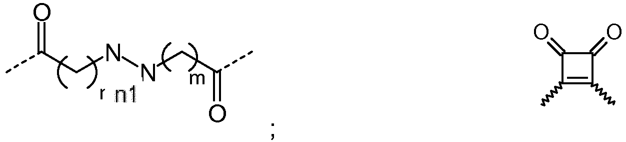
m, n1, o et p représentent chacun un entier compris entre 1 et 10, indépendamment les uns des autres
et
J¹ et J² sont sélectionnés indépendamment l'un de l'autre parmi: 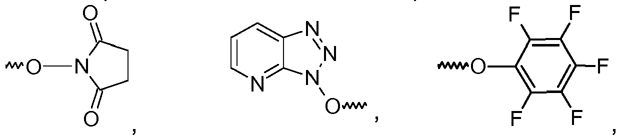 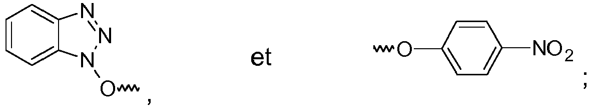
et
b4) la liaison du saccharide de formule 28 à une protéine porteuse.
